# EUROPEAN PATENT APPLICATION

(11) **EP 4 455 139 A1**
(43) Date of publication of application: **30.10.2024**
(21) Application number: 22909841.3
(22) Date of filing: 14.12.2022
(51) Int. Cl.: C07D 403/12, A61K 31/506, A61P 35/00

(54) **COMPOUND AS FAK INHIBITOR AND USE THEREOF**

(30) Priority: 21.12.2021 CN 202111574916; 11.04.2022 CN 202210377379
(71) Applicant: Wigen Biomedicine Technology (Shanghai) Co., Ltd., Shanghai 201203 (CN)
(72) Inventor: XIE, Yuli, Shanghai 201203 (CN); LIU, Wenzhong, Shanghai 201203 (CN); QIAN, Lihui, Shanghai 201203 (CN)
(74) Representative: Willett, Christopher David
(86) International application number: PCT/CN2022/138955
(87) International publication number: WO 2023/116527

(57) **Abstract**

Disclosed in the present invention are a class of compounds as FAK inhibitors and uses thereof. Specifically, the present invention relates to a compound of general formula (1), a preparation method therefor, and use of the compound of general formula (1) and an isomer, a crystalline form, a pharmaceutically acceptable salt, a hydrate or a solvate thereof as a FAK inhibitor. The compound of general formula (1) and the isomer, the crystalline form, the pharmaceutically acceptable salt, the hydrate or the solvate thereof of the present invention can be used for preparing a medicament for treating or preventing a related disease mediated by FAK.

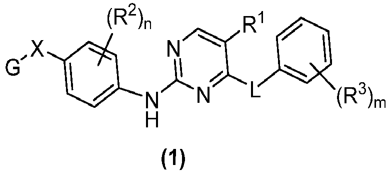

## Description

The present application claims priority to Chinese Patent Application No. 202111574916.4 filed on December 21, 2021 and Chinese Patent Application No. 202210377379.2 filed on April 11, 2022, which are incorporated herein by reference in their entirety.

### TECHNICAL FIELD

The present invention belongs to the field of pharmaceutical chemistry, and particularly relates to a class of compounds with an inhibitory effect on a focal adhesion kinase (FAK kinase), a pharmaceutically acceptable salt thereof and a pharmaceutical composition thereof, as well as use of the compound, the pharmaceutically acceptable salt thereof and the pharmaceutical composition thereof in the treatment or prevention of a related disease mediated by FAK.

### BACKGROUND

Focal adhesion kinase (FAK) is a member of the non-receptor tyrosine kinase family, located at the cell-to-cell junction. After the extracellular matrix (ECM) binds to integrin receptors on the cell membrane surface, FAK is activated, thereby up-regulating the downstream signaling pathway of the integrin receptors. Autophosphorylation of FAK Tyr397 is a biomarker of FAK kinase activity. Studies have shown that FAK plays an important role in cell survival, growth, adhesion, migration and invasion (McLean et al., 2005, Nat Rev cancer, 5: 505-515). The expression and activity of FAK are up-regulated by cancer cells, thereby promoting the proliferation and invasion of the cancer cells and increasing tumor metastasis *in vivo.*

The mRNA level of FAK is highly expressed in about 37% of ovarian cancer cells and 26% of breast cancer cells. FAK inhibitors inhibit the proliferation and invasion of tumor cells. When the function of FAK kinase is hindered, the metastasis of breast cancer is greatly reduced. In addition to promoting the function of cancer cells, FAK mediates the activation of various signaling downstream of angiogenic factors, and is involved in the proliferation, migration and differentiation of vascular endothelial cells. The specific absence of FAK in the vascular endothelial cells indicates that FAK maintains vascular stability during development. Thus, FAK inhibitors can be used for antagonizing pathological angiogenesis. Therefore, FAK inhibitors can directly and indirectly antagonize the development and progression of tumors by inhibiting the function of tumor cells and anti-angiogenesis, thereby being used for treating tumors. In addition to cancer, FAK inhibitors can also be used in non-oncological indications associated with pathological vascular proliferation such as retinal diseases.

Currently, no FAK inhibitor drug is available on the market. A series of pyrimidine compounds serving as FAK inhibitors were reported in the patent WO2010058032A2, wherein BI853520, as a highly selective FAK inhibitor, is in clinical phase I at present.

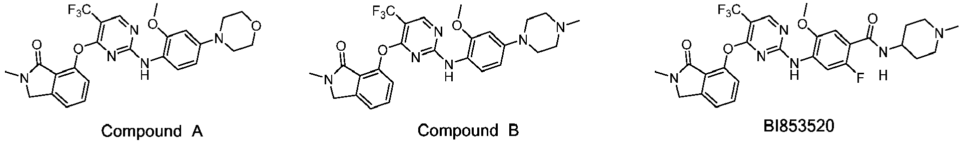

### SUMMARY

The present invention provides a compound of general formula (1) or an isomer, a crystalline form, a pharmaceutically acceptable salt, a hydrate or a solvate thereof: 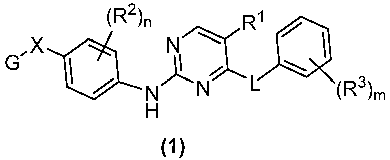 wherein in general formula (1):
L is selected from -CH₂-, -O- or -S-;
X is selected from a chemical bond, 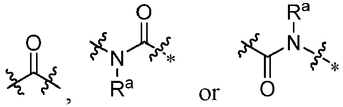 with * representing attachment to a phenyl ring;
G is selected from (9-18 membered) heterocycloalkyl, (C9-C18) cycloalkyl or (9-18 membered) heteroaryl, wherein the (9-18 membered) heterocycloalkyl, (C9-C18) cycloalkyl or (9-18 membered) heteroaryl may be each independently and optionally substituted with 1, 2, 3 or 4 R^{c}; R¹ is selected from -H, -D, halogen, hydroxy, amino, cyano, nitro, -OR^{a}, -NR^{a}R^{b}, -C(O)R^{a}, - CO₂R^{a}, -CONR^{a}R^{b}, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl or (5-14 membered) heteroaryl, wherein the (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl or (5-14 membered) heteroaryl may be each independently and optionally substituted with 1, 2, 3 or 4 R^{c};
R² is selected from -H, -D, halogen, hydroxy, amino, cyano, nitro, -OR^{a}, -NR^{a}R^{b}, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl or (5-14 membered) heteroaryl, wherein the (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl or (5-14 membered) heteroaryl may be each independently and optionally substituted with 1, 2, 3 or 4 R^{c};
R³ is selected from -H, -D, halogen, hydroxy, amino, cyano, nitro, -OR^{3a} , -NR^{3a}R^{3b}, -C(O)R^{3a}, - CO₂R^{3a}, -S(O)ₚR^{3a}, -S(O)ₚNR^{3a}R^{3b}, -CONR^{3a}R^{3b}, -C(=NR^{3a})-NR^{3b}R^{3c}, -NR^{3a}COR^{3b},-NR^{3a}CONR^{3b}R^{3c}, -NR^{3a}CO₂R^{3b}, -NR^{3a}S(O)ₚNR^{3b}R^{3c}, -NR^{3a}S(O)ₚR^{3b}, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C1-C8) alkoxy, -(C1-C8) alkylene-(C3-C14) cycloalkyl, -(C1-C8) alkylene-(3-14 membered) heterocycloalkyl, -(C1-C8) alkylene-(C6-C14) aryl and -(C1-C8) alkylene-(5-14 membered) heteroaryl; or two R³ attached to two adjacent carbon atoms, together with the two adjacent carbon atoms to which they are attached, form one partially unsaturated (C4-C7) cycloalkyl or partially unsaturated (4-7 membered) heterocycloalkyl, wherein the partially unsaturated (C4-C7) cycloalkyl or partially unsaturated (4-7 membered) heterocycloalkyl may be each independently and optionally substituted with 1, 2, 3, 4 or 5 R^{c};
R^{a} and R^{b} are each independently -H, -D, halogen, hydroxy, amino, cyano, nitro, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl or -(C1-C8) alkylene-(5-14 membered) heteroaryl, wherein the (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl or -(C1-C8) alkylene-(5-14 membered) heteroaryl may be each independently and optionally substituted with 1, 2, 3 or 4 R^{c};
R^{c} is -H, -D, halogen, hydroxy, amino, cyano, nitro, -OR^{xa}, -NR^{xa}R^{xb}, -(CH₂)ₘOR^{xa}, - (CH₂)ₘNR^{xa}R^{xb}, -C(O)R^{xa}, -CO₂R^{xa}, -S(O)ₚR^{xa}, -S(O)ₚNR^{xa}R^{xb}, -CONR^{xa}R^{xb}, -C(=NR^{xa})-NR^{xb}R^{xc}, -NR^{xa}COR^{xb}, -NR^{xa}CONR^{xb}R^{xc}, -NR^{xa}CO₂R^{xb}, -NR^{xa}S(O)_{P}NR^{xb}R^{xc}, -NR^{xa}S(O)ₚR^{xb}, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, - (C1-C8) alkylene-(C1-C8) alkoxy, -(C1-C8) alkylene-(C3-C14) cycloalkyl, -(C1-C8) alkylene-(3-14 membered) heterocycloalkyl, -(C1-C8) alkylene-(C6-C14) aryl or -(C1-C8) alkylene-(5-14 membered) heteroaryl; or when two R^{c} are attached to the same atom, two R^{c} may form one oxo;
R^{xa}, R^{xb} and R^{xc} are each independently -H, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl or -(C1-C8) alkylene-(5-14 membered) heteroaryl;
R^{3a}, R^{3b} and R^{3c} are each independently -H, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl or -(C1-C8) alkylene-(5-14 membered) heteroaryl;
p is an integer of 0, 1 or 2;
m is an integer of 0, 1, 2 or 3;
n is an integer of 0, 1, 2 or 3.

In another embodiment of the present invention, in general formula (1), L is -CH₂- or -O-; L is preferably -O-.

In another embodiment of the present invention, in general formula (1), G is (9-15 membered) heterocycloalkyl, wherein the (9-15 membered) heterocycloalkyl may be optionally substituted with 1, 2, 3 or 4 of the following groups: -H, -D, -F, -Cl, -Br, -I, -OH, -NH₂, -CN, -NO₂, -OCH₃, -NHCH₃, -N(CH₃)₂, -C(O)NH₂, -OCH₂CH₃, -OCF₃, -CH₃, -CD₃, -CH₂CH₃, -CH₂F, -CHF₂, 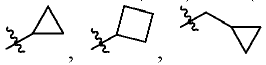 or -CF₃; or two substituents attached to the same atom may form one oxo.

In another embodiment of the present invention, in general formula (1), G is (9-12 membered) heterocycloalkyl, wherein the (9-12 membered) heterocycloalkyl may be optionally substituted with 1, 2, 3 or 4 of the following groups: -H, -D, -CH₃, -CD₃, -CH₂CH₃, -OCH₃, 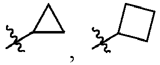 or 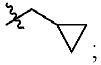 preferably -H or -CH₃; or two substituents attached to the same atom may form one oxo; preferably, G is (9-11 membered) heterocyclic spirocycloalkyl, wherein the (9-11 membered) heterocyclic spirocycloalkyl may be optionally substituted with 1, 2, 3 or 4 of the following groups: -H, -D, -CH₃, -CD₃, -CH₂CH₃,

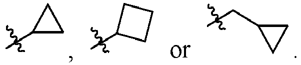

In another embodiment of the present invention, in general formula (1), G is selected from 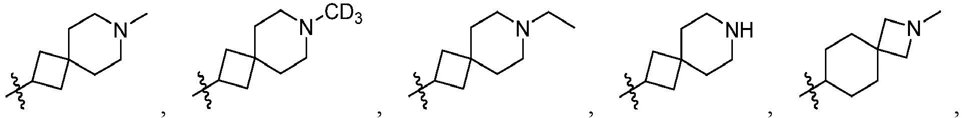 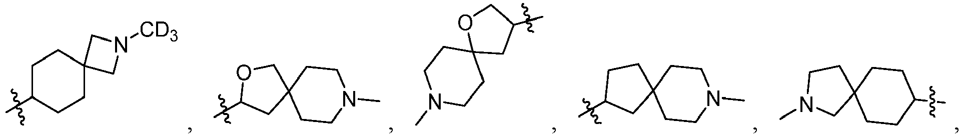 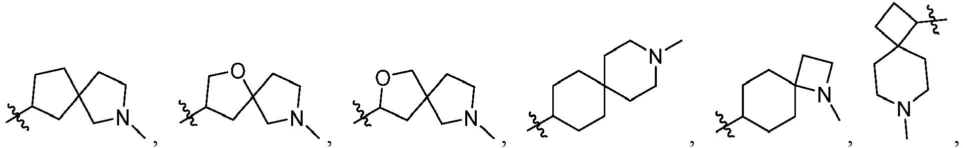 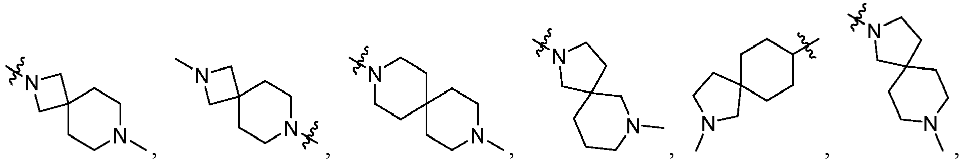 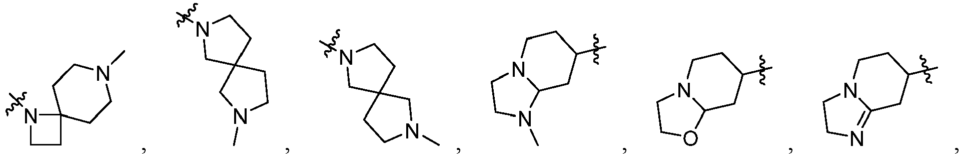 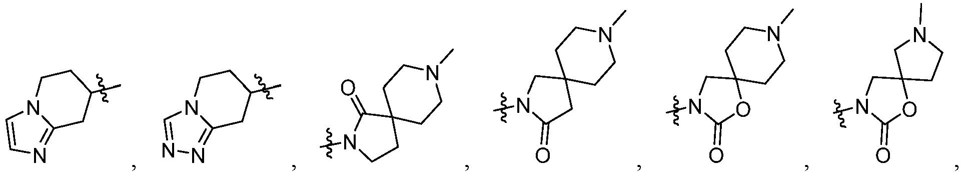 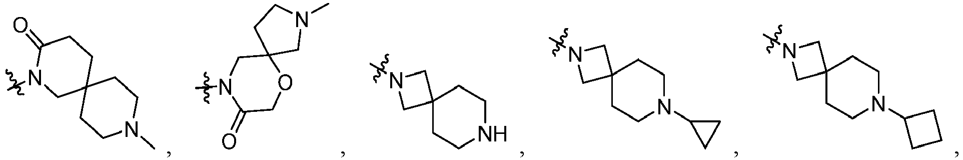 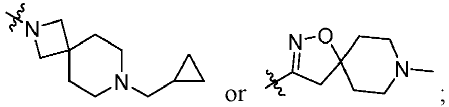 G is preferably

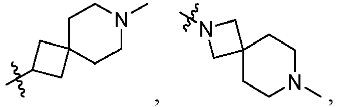

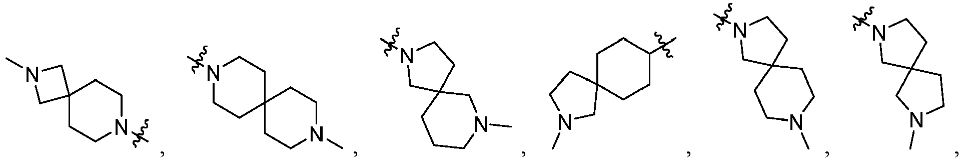

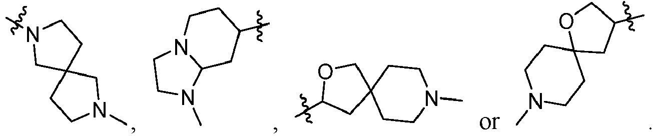

In another embodiment of the present invention, in general formula (1), X is selected from a chemical bond, 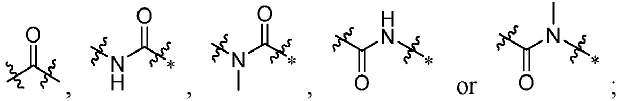 X is preferably selected from a chemical bond, 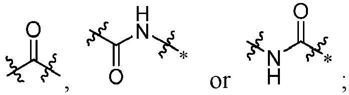 X is more preferably selected from a chemical bond, 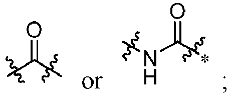 X is more preferably 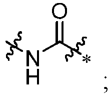 X is more preferably 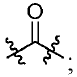 X is more preferably a chemical bond; wherein * represents attachment to a phenyl ring.

In another embodiment of the present invention, in general formula (1), R¹ is selected from -H, -D, -F, -Cl, -Br, -I, -OH, -NH₂, -CN, -NO₂, -OCH₃, -NHCH₃, -N(CH₃)₂, -C(O)NH₂, -OCH₂CH₃, -OCF₃, -CH₃, -CD₃, -CH₂CH₃, -CH₂F, -CHF₂ or -CF₃; R¹ is preferably -F, -Cl, -Br, -CN, -NO₂, - CF₃ or -C(O)NH₂; R¹ is preferably -F, -Cl, -Br, -CN, -NO₂ or -CF₃; R¹ is more preferably -CF₃; R¹ is more preferably -Cl, -Br, -CN or -NO₂.

In another embodiment of the present invention, in general formula (1), R² is -H, -D, -F, -Cl, - Br, -I, -OH, -NH₂, -CN, -NO₂, -OCF₃, -NHCH₃, -N(CH₃)₂, -NHCH₂CH₃, -N(CH₂CH₃)₂, (C1-C3) alkyl, (C1-C3) alkoxy, (C1-C3) haloalkyl, (C2-C4) alkenyl, (C2-C4) alkynyl, (C3-C6) cycloalkyl, (3-6 membered) heterocycloalkyl, (C6-C10) aryl or (5-10 membered) heteroaryl, wherein the (C1-C3) alkyl, (C1-C3) alkoxy, (C1-C3) haloalkyl, (C2-C4) alkenyl, (C2-C4) alkynyl, (C3-C6) cycloalkyl, (3-6 membered) heterocycloalkyl, (C6-C10) aryl or (5-10 membered) heteroaryl may be each independently and optionally substituted with 1, 2, 3 or 4 of the following groups: -H, -D, -F, -Cl, -Br, -I, -OH, -NH₂, -CN, -NO₂, -OCH₃, -NHCH₃, -N(CH₃)₂, -C(O)NH₂, -OCH₂CH₃, -OCF₃, -CH₃, -CD₃, -CH₂CH₃, -CH₂F, -CHF₂ or -CF₃.

In another embodiment of the present invention, in general formula (1), R² is selected from -H, -D, -F, -Cl, -Br, -I, -OH, -NH₂, -CN, -NO₂, -OCH₃, -NHCH₃, -N(CH₃)₂, -C(O)NH₂, -OCH₂CH₃, -OCF₃, -CH₃, -CD₃, -CH₂CH₃, -CH₂F, -CHF₂, -CF₃, 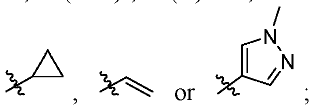 R² is preferably -D, -F, -Cl, -OCH₃, -OCF₃, -CH₃, -CD₃, -CH₂CH₃, -CF₃, 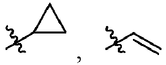 or 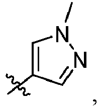 and n is 1 or 2; R² is more preferably -F, -Cl, -OCH₃, -OCF₃, -CH₂CH₃, 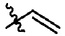 or 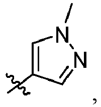 and n is 1 or 2; R² is more preferably -F, -Cl or -OCH₃, and n is 1 or 2; R² is more preferably -F or -OCH₃, and n is 1 or 2.

In another embodiment of the present invention, in general formula (1), R³ is selected from -H, -D, halogen, hydroxy, amino, cyano, nitro, -OR^{3a}, -NR^{3a}R^{3b}, -C(O)R^{3a}, -CO₂R^{3a}, -S(O)ₚR^{3a}, - S(O)ₚNR^{3a}R^{3b}, -CONR^{3a}R^{3b}, -C(=NR^{3a})-NR^{3b}R^{3c}, -NR^{3a}COR^{3b}, -NR^{3a}CONR^{3b}R^{3c}, - NR^{3a}CO₂R^{3b}, -NR^{3a}S(O)ₚR^{3b}, (C1-C6) alkyl, (C1-C6) alkoxy, (C1-C6) haloalkyl, (C2-C6) alkenyl, (C2-C6) alkynyl, (C3-C10) cycloalkyl, (3-10 membered) heterocycloalkyl, (C6-C10) aryl, (5-10 membered) heteroaryl, -(C1-C3) alkylene-(C1-C6) alkoxy, -(C1-C3) alkylene-(C3-C10) cycloalkyl, -(C1-C3) alkylene-(3-10 membered) heterocycloalkyl, -(C1-C3) alkylene-(C6-C10) aryl or -(C1-C3) alkylene-(5-10 membered) heteroaryl.

In another embodiment of the present invention, in general formula (1), R³ is selected from -H, -D, -F, -Cl, -Br, -I, -OH, -NH₂, -CN, -NO₂, -OCH₃, -NHCH₃, -N(CH₃)₂, -C(O)NH₂, -OCH₂CH₃, -OCF₃, -CH₃, -CD₃, -CH₂CH₃, -CH₂F, -CHF₂, -CF₃, 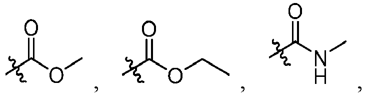 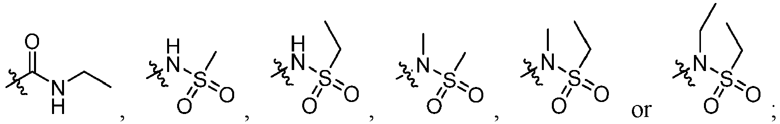 R³ is preferably selected from -H, -D, -F, -Cl, -CN, 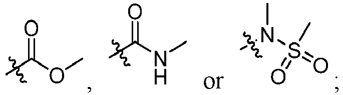 R³ is more preferably -H,

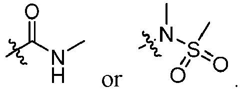

In another embodiment of the present invention, in general formula (1), structural unit 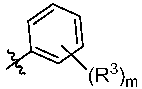 is selected from 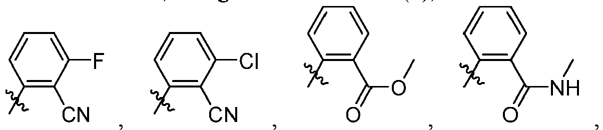 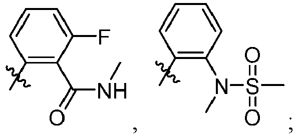 preferably

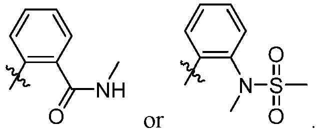

In another embodiment of the present invention, in general formula (1), two R³ attached to two adjacent carbon atoms, together with the two adjacent carbon atoms to which they are attached, form one partially unsaturated (C4-C7) cycloalkyl or partially unsaturated (4-7 membered) heterocycloalkyl, wherein the partially unsaturated (C4-C7) cycloalkyl or partially unsaturated (4-7 membered) heterocycloalkyl may be each independently and optionally substituted with 1, 2, 3, 4 or 5 substituents of -H, -D, -CH₃, -CD₃, -CH₂CH₃ or -OCH₃, preferably -H, -CH₃ or - OCH₃; or two substituents attached to the same atom may form one oxo; preferably, two R³ attached to two adjacent carbon atoms, together with the two adjacent carbon atoms to which they are attached, form one partially unsaturated (C5) cycloalkyl or partially unsaturated (5-membered) heterocycloalkyl, wherein the partially unsaturated (C5) cycloalkyl or partially unsaturated (5-membered) heterocycloalkyl may be each independently and optionally substituted with 1, 2 or 3 substituents of -D, -CH₃, -CD₃, -CH₂CH₃ or -OCH₃, and the substituent is preferably 1, 2 or 3 groups of -CH₃ or -OCH₃; more preferably, two R³ attached to two adjacent carbon atoms, together with the two adjacent carbon atoms to which they are attached, form one partially unsaturated (C5) cycloalkyl substituted with 1 oxo or partially unsaturated (5-membered) heterocycloalkyl substituted with 1 oxo, wherein the partially unsaturated (C5) cycloalkyl substituted with 1 oxo or partially unsaturated (5-membered) heterocycloalkyl substituted with 1 oxo may be each independently and optionally substituted with 1, 2 or 3 substituents of -CH₃ or -OCH₃.

In another embodiment of the present invention, in general formula (1), structural unit 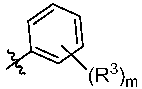 is selected from 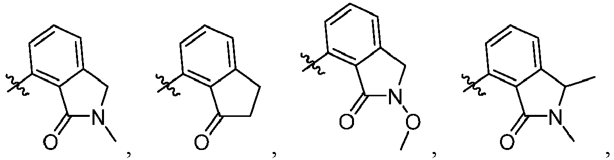 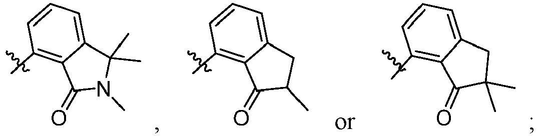 preferably 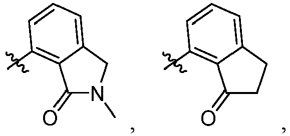 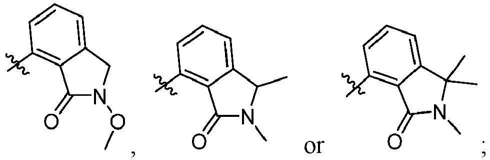 more preferably

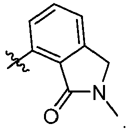

In another specific embodiment of the present invention, the compound of general formula (1) has one of the following structures:

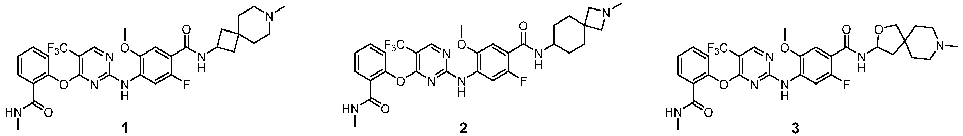

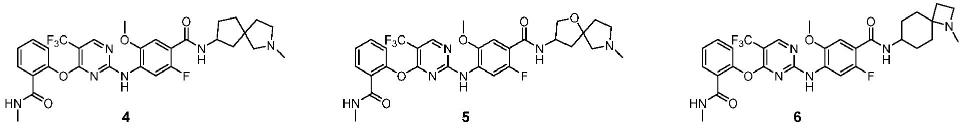

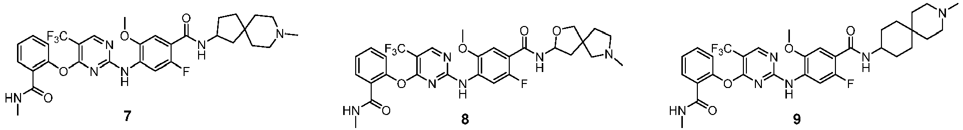

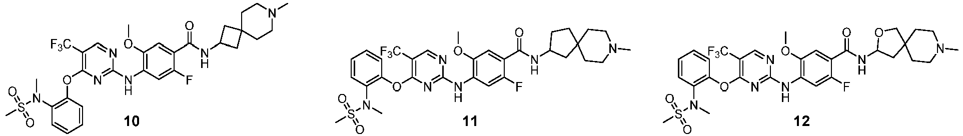

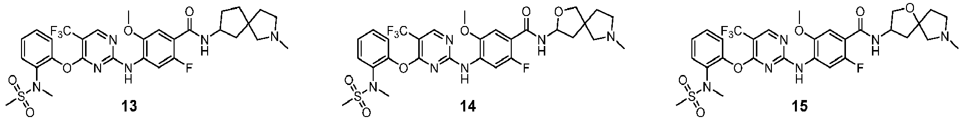

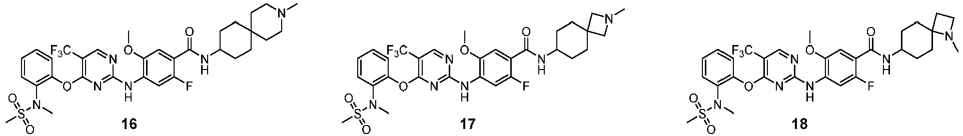

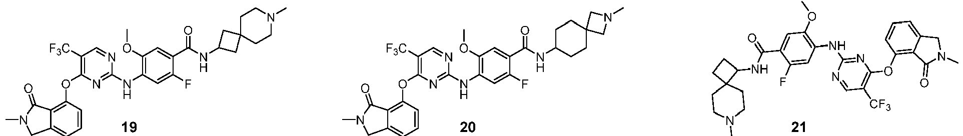

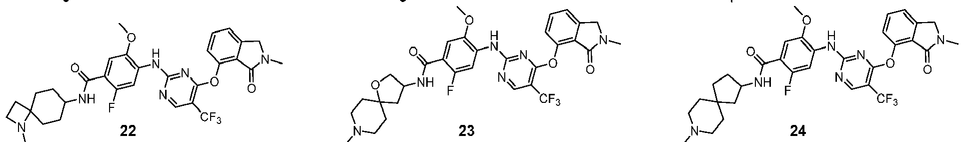

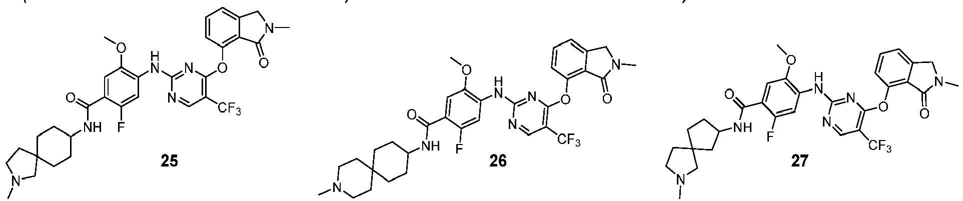

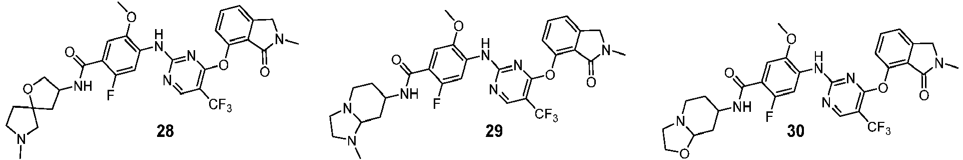

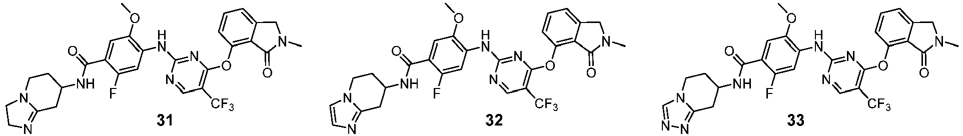

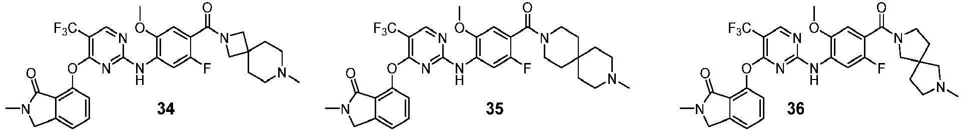

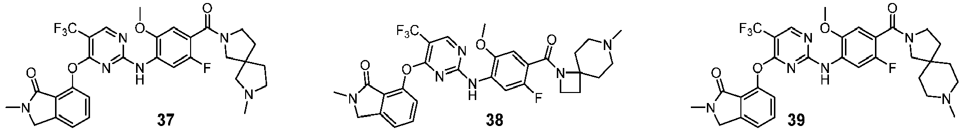

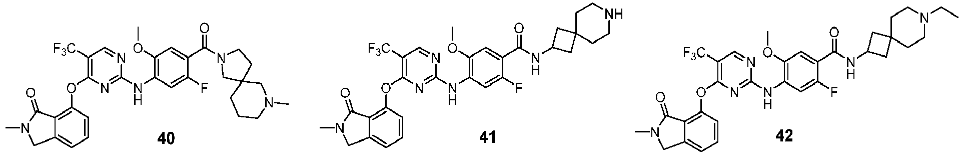

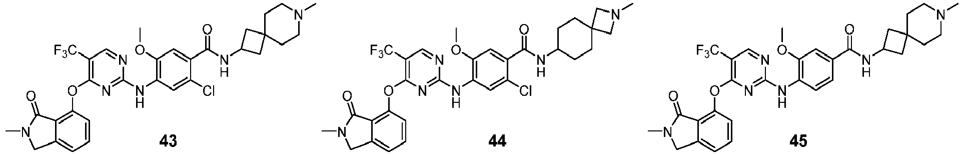

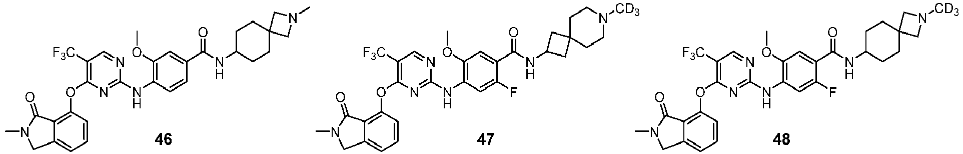

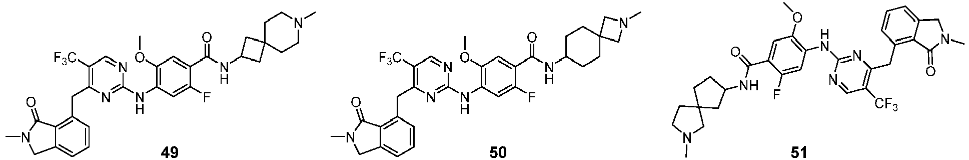

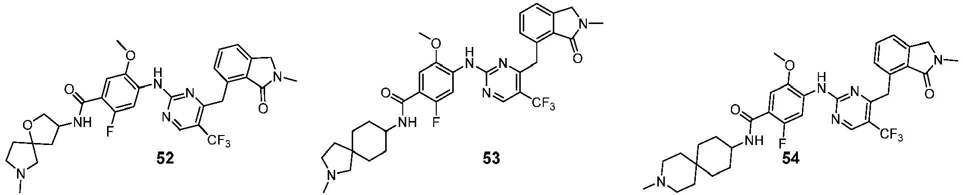

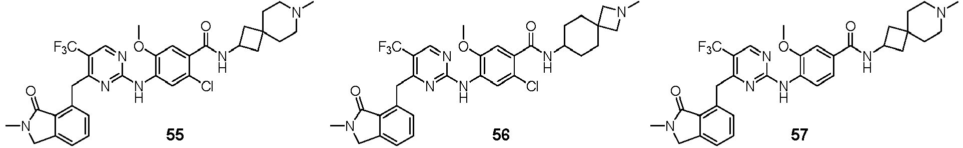

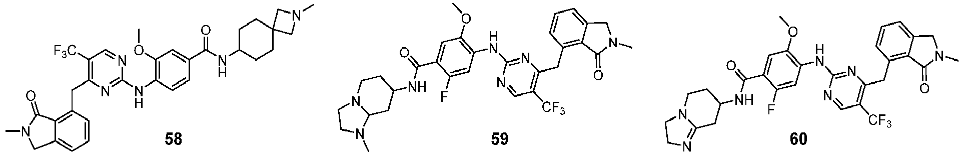

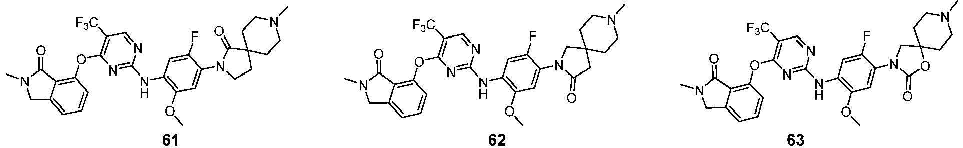

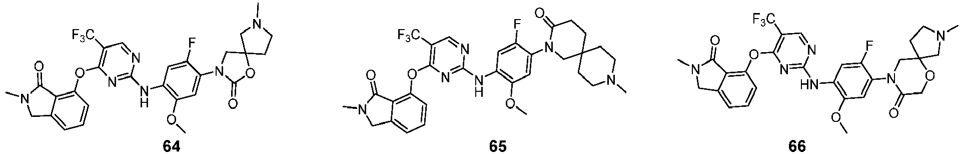

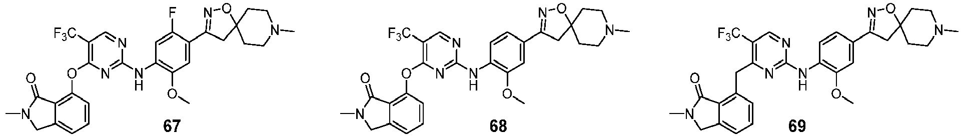

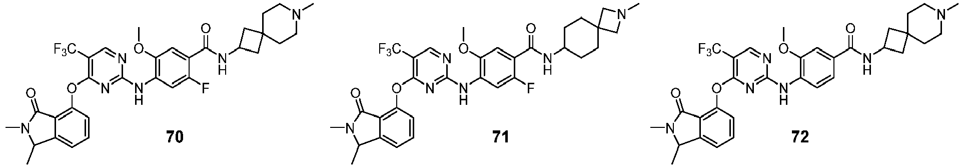

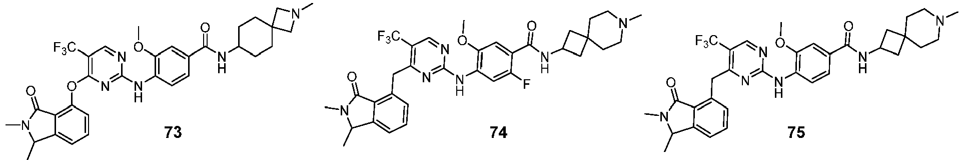

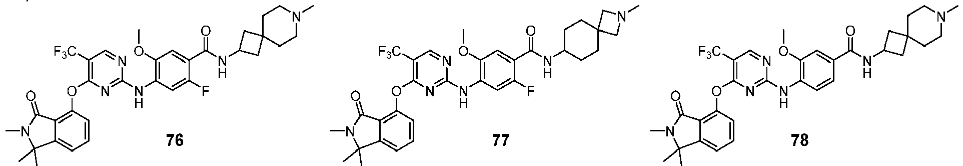

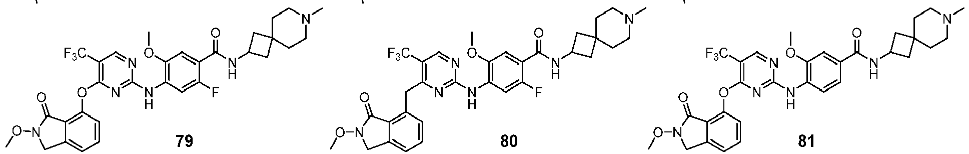

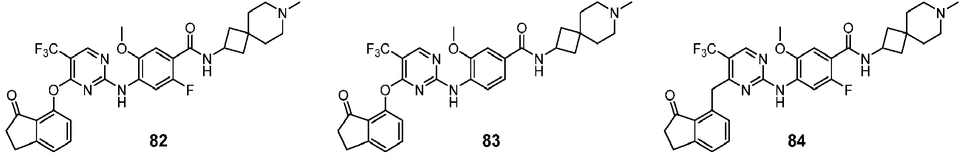

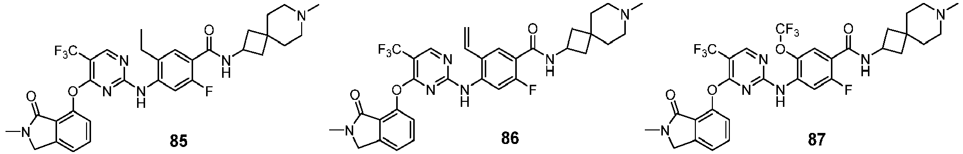

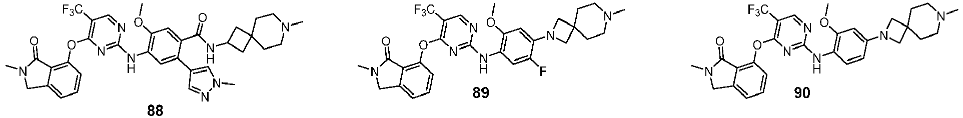

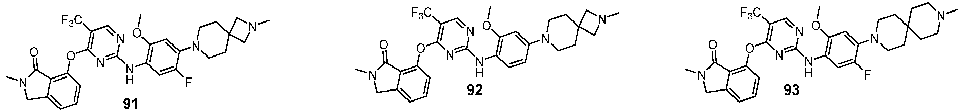

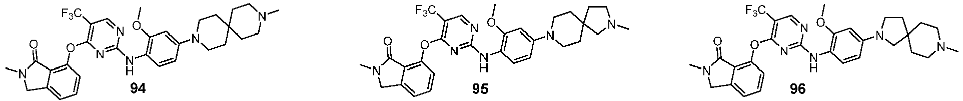

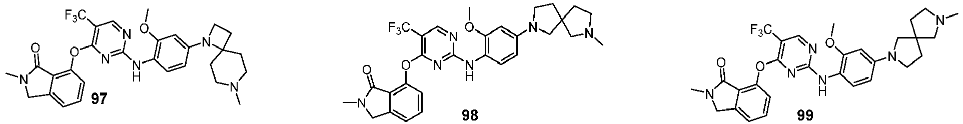

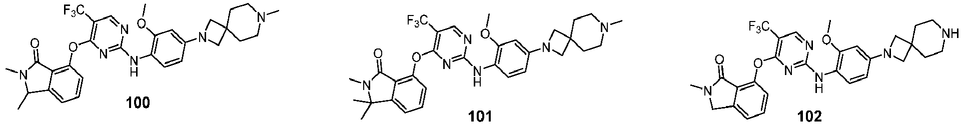

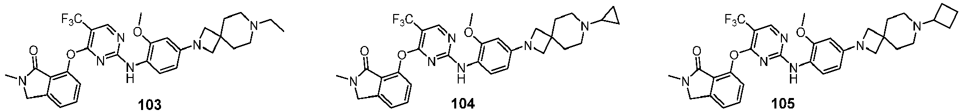

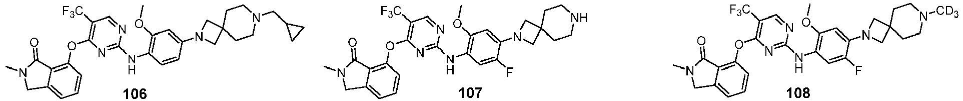

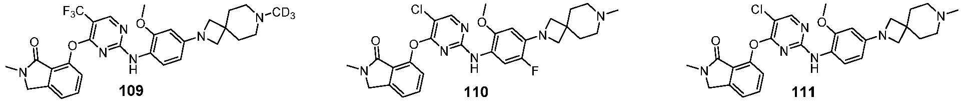

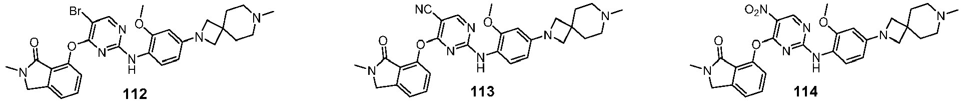

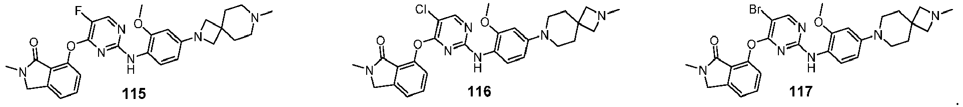

The present invention is further intended to provide a pharmaceutical composition comprising a pharmaceutically acceptable carrier, diluent and/or excipient, and the compound of general formula (1) or the isomer, the crystalline form, the pharmaceutically acceptable salt, the hydrate or the solvate thereof of the present invention as an active ingredient.

The present invention is still further intended to provide use of the compound of general formula (1) or the isomer, the crystalline form, the pharmaceutically acceptable salt, the hydrate or the solvate thereof of the present invention, or the pharmaceutical composition described above in the preparation of a medicament for treating, regulating, or preventing a disease related to FAK protein kinase, wherein the disease is preferably cancer, and the cancer is a hematologic cancer or a solid tumor.

The present invention is even further intended to provide a method for treating, regulating or preventing a related disease mediated by FAK protein kinase, the method including administering to a subject a therapeutically effective amount of the compound of general formula (1) or the isomer, the crystalline form, the pharmaceutically acceptable salt, the hydrate or the solvate thereof of the present invention, or the pharmaceutical composition described above.

It should be understood that both the above general description and the following detailed description of the present invention are exemplary and explanatory, and are intended to provide further explanation of the present invention claimed.

### Synthesis of Compounds

Methods for preparing the compounds of general formula (1) of the present invention are specifically described below, but these specific methods do not limit the present invention in any way.

The compounds of general formula (1) described above can be synthesized using standard synthetic techniques or well-known techniques in combination with the methods described herein. In addition, the solvents, temperatures and other reaction conditions mentioned herein can vary. Starting materials for the synthesis of the compounds can be obtained synthetically or commercially. The compounds described herein and other related compounds with different substituents can be synthesized using well-known techniques and starting materials, including the methods found in March, ADVANCED ORGaNIC CHEMISTRY, 4th Ed., (Wiley 1992); Carey and Sundberg, ADVANCED ORGANIC CHEMISTRY, 4th Ed., Vols. A and B (Plenum 2000, 2001); and Green and Wuts, PROTECTIVE GROUPS IN ORGANIC SYNTHESIS, 3rd Ed., (Wiley 1999). General methods for preparing the compounds can be changed by using appropriate reagents and conditions for introducing different groups into the molecular formulas provided herein.

In one aspect, the compounds described herein are prepared according to methods well known in the art. However, the conditions of the methods, such as reactants, solvents, bases, the amount of the compounds used, reaction temperature and time required for the reaction are not limited to the following explanation. The compounds of the present invention can also be conveniently prepared by optionally combining various synthetic methods described herein or known in the art, and such combinations can be easily determined by those skilled in the art to which the present invention pertains. In one aspect, the present invention further provides a method for preparing the compound of general formula (1), which is prepared using general reaction schemes 1-3 below:

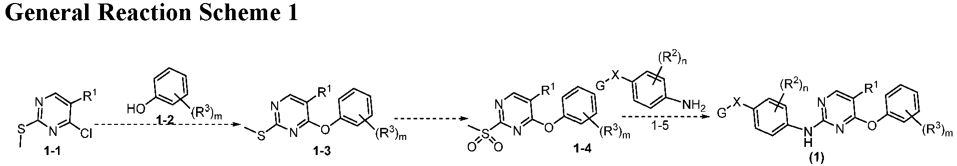

The compound of general formula (1) can be prepared according to general reaction scheme 1, wherein R¹, R², R³, X, G, m and n are as defined above. As shown in general reaction scheme 1, compound 1-1 reacts with compound 1-2 under alkaline conditions to give compound 1-3, compound 1-3 is oxidized to give compound 1-4, and compound 1-4 reacts with compound 1-5 under appropriate conditions to give the target compound (1).

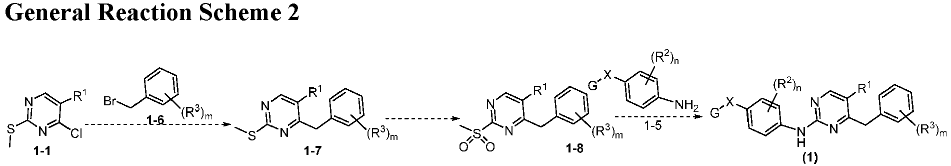

The compound of general formula (1) can be prepared according to general reaction scheme 2, wherein R¹, R², R³, X, G, m and n are as defined above. As shown in general reaction scheme 2, compound 1-1 is subjected to a coupling reaction with compound 1-6 to give compound 1-7, compound 1-7 is oxidized to give compound 1-8, and compound 1-8 reacts with compound 1-5 under appropriate conditions to give the target compound (1).

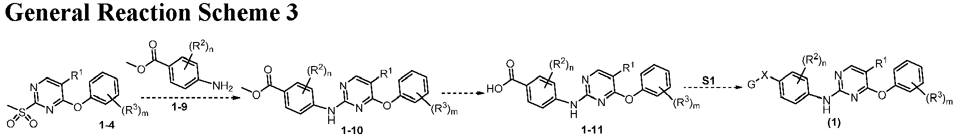

The compound of general formula (1) can be prepared according to general reaction scheme 3, wherein R¹, R², R³, X, G, m and n are as defined above. As shown in general reaction scheme 2, compound 1-4 reacts with compound 1-9 under appropriate conditions to give compound 1-10, compound 1-10 is hydrolyzed to give compound 1-11, and compound 1-11 is subjected to a condensation reaction with fragment S1 to give the target compound (1).

### Further Forms of Compounds

"Pharmaceutically acceptable" herein refers to a substance, such as a carrier or diluent, which will not lead to loss of biological activity or properties of a compound and is relatively non-toxic. For example, when an individual is given a substance, the substance will not cause undesired biological effects or interact with any component contained therein in a deleterious manner.

The term "pharmaceutically acceptable salt" refers to a form of a compound that does not cause significant irritation to the organism receiving the administration or eliminate the biological activity and properties of the compound. In certain specific aspects, the pharmaceutically acceptable salt is obtained by subjecting the compound of the general formula to a reaction with acids or bases, wherein the acids or bases include, but are not limited to, those found in Stahl and Wermuth, Handbook of Pharmaceutical Salts: Properties, Selection, and Use, 1st Ed., (Wiley, 2002).

It should be understood that references to pharmaceutically acceptable salts include solvent addition forms or crystalline forms, especially solvates or polymorphs. A solvate contains either stoichiometric or non-stoichiometric amount of solvent and is selectively formed during crystallization in a pharmaceutically acceptable solvent such as water and ethanol. Hydrates are formed when the solvent is water, or alcoholates are formed when the solvent is ethanol. The solvates of the compound of general formula (1) are conveniently prepared or formed according to the methods described herein. For example, hydrates of the compound of general formula (1) are conveniently prepared by recrystallization in a mixed solvent of water/organic solvent, wherein the organic solvent used includes, but is not limited to, tetrahydrofuran, acetone, ethanol or methanol. Furthermore, the compounds described herein may be present in either a non-solvated form or a solvated form. In general, the solvated forms are considered equivalent to the non-solvated forms for purposes of the compounds and methods provided herein.

In other specific examples, the compound of general formula (1) is prepared in different forms including, but not limited to, amorphous, pulverized and nanoparticle forms. In addition, the compound of general formula (1) includes crystalline forms, and may also be polymorphs. Polymorphs include different lattice arrangements of the same elements of a compound. Polymorphs generally have different X-ray diffraction spectra, infrared spectra, melting points, density, hardness, crystalline forms, optical and electrical properties, stability and solubility. Different factors such as a recrystallization solvent, crystallization rate, and storage temperature may lead to a single dominant crystalline form.

In another aspect, the compound of general formula (1) may have a chiral center and/or axial chirality, and thus may be present in the form of a racemate, a racemic mixture, a single enantiomer, a diastereomeric compound, a single diastereomer and a cis-trans isomer. Each chiral center or axial chirality will independently produce two optical isomers, and all possible optical isomers, diastereomeric mixtures, and pure or partially pure compounds are included within the scope of the present invention. The present invention is meant to include all such isomeric forms of these compounds.

The compound of the present invention may contain unnatural proportions of atomic isotopes at one or more of the atoms that constitute the compound. For example, the compound may be labeled with radioactive isotopes, such as tritium (³H), iodine-125 (¹²⁵I) and C-14 (¹⁴C). For another example, deuterium can be used to substitute a hydrogen atom to form a deuterated compound. The bond formed by deuterium and carbon is stronger than that formed by ordinary hydrogen and carbon. Compared with an undeuterated medicament, the deuterated medicament generally has the advantages of reduced toxic and side effects, increased pharmaceutical stability, enhanced efficacy, prolonged pharmaceutical *in vivo* half-life and the like. All isotopic variations of the compound of the present invention, whether radioactive or not, are contained within the scope of the present invention.

### Terminology

Unless otherwise stated, the terms used in the present application, including those in the specification and claims, are defined as follows. It must be noted that in the specification and the appended claims, the singular forms "a" and "an" include plural meanings unless clearly indicated otherwise. Unless otherwise stated, conventional methods for mass spectrometry, nuclear magnetic resonance spectroscopy, HPLC, protein chemistry, biochemistry, recombinant DNA techniques and pharmacology are used. As used herein, "or" or "and" refers to "and/or" unless otherwise stated.

Unless otherwise specified, "alkyl" refers to a saturated aliphatic hydrocarbon group, including linear and branched groups containing 1 to 6 carbon atoms. Lower alkyl groups containing 1 to 4 carbon atoms, such as methyl, ethyl, propyl, 2-propyl, *n*-butyl, isobutyl, or *tert*-butyl, are preferred. As used herein, "alkyl" includes unsubstituted and substituted alkyl, particularly alkyl substituted with one or more halogens. Preferred alkyl is selected from CH₃, CH₃CH₂, CF₃, CHF₂, CF₃CH₂, CF₃(CH₃)CH, ⁱPr, ⁿPr, ⁱBu, ⁿBu or ^{t}Bu.

Unless otherwise specified, "alkylene" refers to a divalent alkyl as defined above. Examples of alkylene include, but are not limited to, methylene and ethylene.

Unless otherwise specified, "alkenyl" refers to an unsaturated aliphatic hydrocarbon group containing carbon-carbon double bonds, including linear or branched groups containing 1 to 14 carbon atoms. Lower alkenyl groups containing 1 to 4 carbon atoms, such as vinyl, 1-propenyl, 1-butenyl, or 2-methylpropenyl, are preferred.

Unless otherwise specified, "alkenylene" refers to a divalent alkenyl as defined above.

Unless otherwise specified, "alkynyl" refers to an unsaturated aliphatic hydrocarbon group containing carbon-carbon triple bonds, including linear and branched groups containing 1 to 14 carbon atoms. Lower alkynyl groups containing 1 to 4 carbon atoms, such as ethynyl, 1-propynyl, or 1-butynyl, are preferred.

Unless otherwise specified, "alkynylene" refers to a divalent alkynyl as defined above.

Unless otherwise specified, "cycloalkyl" refers to a non-aromatic hydrocarbon ring system (monocyclic, bicyclic or polycyclic), and partially unsaturated cycloalkyl may be referred to as "cycloalkenyl" if the carbocyclic ring contains at least one double bond, or "cycloalkynyl" if the carbocyclic ring contains at least one triple bond. Cycloalkyl may include monocyclic or polycyclic groups (e.g., having 2, 3 or 4 fused rings) and spiro rings. In some embodiments, cycloalkyl is monocyclic. In some embodiments, cycloalkyl is monocyclic or bicyclic. The ring carbon atoms of cycloalkyl may optionally be oxidized to form an oxo or thio group. Cycloalkyl further includes cycloalkylene. In some embodiments, cycloalkyl contains 0, 1 or 2 double bonds. In some embodiments, cycloalkyl contains 1 or 2 double bonds (partially unsaturated cycloalkyl). In some embodiments, cycloalkyl may be fused to aryl, heteroaryl, cycloalkyl and heterocycloalkyl. In some embodiments, cycloalkyl may be fused to aryl, cycloalkyl and heterocycloalkyl. In some embodiments, cycloalkyl may be fused to aryl and heterocycloalkyl. In some embodiments, cycloalkyl may be fused to aryl and cycloalkyl. Examples of cycloalkyl include cyclopropyl, cyclobutyl, cyclopentyl, cyclohexyl, cycloheptyl, cyclopentenyl, cyclohexenyl, cyclohexadienyl, cycloheptatrienyl, norcamphanyl, norpinanyl, norcarnyl, bicyclo[1.1.1]pentyl, bicyclo[2.1.1]hexyl and the like.

Unless otherwise specified, "cycloalkylene" refers to a divalent cycloalkyl as defined above.

Unless otherwise specified, "alkoxy" refers to an alkyl group that bonds to the rest of the molecule through an ether oxygen atom. Representative alkoxy groups are those having 1-6 carbon atoms, such as methoxy, ethoxy, propoxy, isopropoxy, butoxy, isobutoxy, *sec*-butoxy, and *tert*-butoxy. As used herein, "alkoxy" includes unsubstituted and substituted alkoxy, particularly alkoxy substituted with one or more halogens. Preferred alkoxy is selected from OCH₃, OCF₃, CHF₂O, CF₃CH₂O, ⁱ⁻PrO, ⁿ⁻PrO, ⁱ⁻BuO, ⁿ⁻BuO or ^{t-}BuO.

Unless otherwise specified, "aryl" refers to an aromatic hydrocarbon group, which is monocyclic or polycyclic; for example, a monocyclic aryl ring is fused to one or more carbocyclic aromatic groups. Examples of aryl include, but are not limited to, phenyl, naphthyl, and phenanthryl. Unless otherwise specified, "aryloxy" refers to an aryl group that bonds to the rest of the molecule through an ether oxygen atom. Examples of aryloxy include, but are not limited to, phenoxy and naphthoxy.

Unless otherwise specified, "arylene" refers to a divalent aryl as defined above. Examples of arylene include, but are not limited to, phenylene, naphthylene, and phenanthrylene.

Unless otherwise specified, "heteroaryl" refers to an aromatic group containing one or more heteroatoms (O, S, or N), and the heteroaryl is monocyclic or polycyclic. For example, a monocyclic heteroaryl ring is fused to one or more carbocyclic aromatic groups or other monocyclic heterocycloalkyl groups. Examples of heteroaryl include, but are not limited to, pyridinyl, pyridazinyl, imidazolyl, pyrimidinyl, pyrazolyl, triazolyl, pyrazinyl, quinolinyl, isoquinolinyl, furanyl, thienyl, isoxazolyl, thiazolyl, oxazolyl, isothiazolyl, pyrrolyl, indolyl, benzimidazolyl, benzofuranyl, benzothiazolyl, benzothienyl, benzoxazolyl, benzopyridinyl, pyrrolopyrimidinyl, 1H-pyrrolo[3,2-b]pyridinyl, 1H-pyrrolo[2,3-c]pyridinyl, 1H-pyrrolo[3,2-c]pyridinyl, 1H-pyrrolo[2,3-b]pyridinyl, 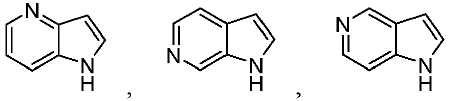 and

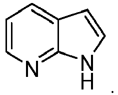

Unless otherwise specified, "heterocycloalkyl" refers to a non-aromatic ring or ring system, which may optionally contain one or more alkenylene as part of the ring structure, having at least one heteroatom ring member independently selected from boron, phosphorus, nitrogen, sulfur, oxygen, and phosphorus. Partially unsaturated heterocycloalkyl may be referred to as "heterocycloalkenyl" if heterocycloalkyl contains at least one double bond, or "heterocycloalkynyl" if the heterocycloalkyl contains at least one triple bond. Heterocycloalkyl may include monocyclic, bicyclic, spiro ring, or polycyclic systems (e.g., having two fused or bridged rings). In some embodiments, heterocycloalkyl is a monocyclic group having 1, 2, or 3 heteroatoms independently selected from nitrogen, sulfur, and oxygen. The ring carbon atoms and heteroatoms of heterocycloalkyl may optionally be oxidized to form oxo or thio groups or other oxidized bonds (e.g., C(O), S(O), C(S) or S(O)2, N-oxides, etc.), or the nitrogen atoms may be quaternized. Heterocycloalkyl may be attached via a ring carbon atom or a ring heteroatom. In some embodiments, heterocycloalkyl contains 0 to 3 double bonds. In some embodiments, heterocycloalkyl contains 0 to 2 double bonds. The definition of heterocycloalkyl further includes moieties (also referred to as partially unsaturated heterocyclic rings) having one or more aromatic rings fused to (i.e., sharing a bond with) the heterocycloalkyl ring, for example, benzo-derivatives of piperidine, morpholine, azepin, thienyl, or the like. Heterocycloalkyl containing a fused aromatic ring may be attached via any ring atom, including ring atoms of the fused aromatic ring. Examples of heterocycloalkyl include, but are not limited to, azetidinyl, azepinyl, dihydrobenzofuranyl, dihydrofuranyl, dihydropyranyl, N-morpholinyl, 3-oxa-9-azaspiro[5.5]undecyl, 1-oxa-8-azaspiro[4.5]decyl, piperidinyl, piperazinyl, oxopiperazinyl, pyranyl, pyrrolidinyl, quininyl, tetrahydrofuranyl, tetrahydropyranyl, 1,2,3,4-tetrahydroquinolinyl, tropanyl, 4,5,6,7-tetrahydrothiazolo[5,4-c]pyridinyl, 4,5,6,7-tetrahydro-1H-imidazo[4,5-c]pyridine, N-methylpiperidinyl, tetrahydroimidazolyl, pyrazolidinyl, butyrolactam, valerolactam, imidazolidinonyl, hydantoinyl, dioxolanyl, phthalimidyl, pyrimidine-2,4(1*H*,3*H*)-dione, 1,4-dioxanyl, morpholinyl, thiomorpholinyl, thiomorpholinyl-S-oxide, thiomorpholinyl-S,S-oxide, piperazinyl, pyranyl, pyridonyl, 3-pyrrolinyl, thiopyranyl, pyronyl, tetrahydrothienyl, 2-azaspiro[3.3]heptanyl, indolinyl,

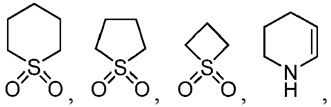

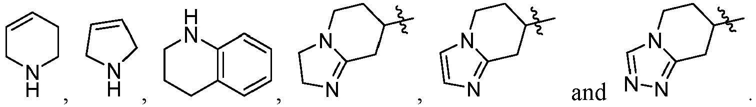

Unless otherwise specified, "heterocyclic spirocycloalkyl" refers to a polycyclic hydrocarbyl formed by sharing one carbon atom (referred to as a spiro atom) between two or more saturated or partially unsaturated monocyclic rings, wherein one or more (e.g., 1, 2 or 3) ring atoms are heteroatoms selected from nitrogen, oxygen or S(O)ₚ (where p is an integer from 0 to 2), and the remaining ring atoms are carbon atoms. When the heteroatom is a nitrogen atom, the nitrogen atom may be substituted or unsubstituted (i.e., N or NR, R being hydrogen or other substituents already defined herein). Each monocyclic ring may contain one or more double bonds, but none of the rings has a fully conjugated π-electron system. According to the number of spiro atoms shared among the rings, the spiroheterocyclyl may be monospiroheterocyclyl, bispiroheterocyclyl or polyspiroheterocyclyl. The term "(5-15 membered) heterocyclic spirocycloalkyl" refers to a heterocyclic spirocycloalkyl having 5 to 15 ring atoms, wherein the monocyclic ring that shares a spiro atom is a 3- to 8-membered monocyclic ring, and at least 1 monocyclic ring is a heterocycloalkyl ring. Preferred is (6-18 membered) heterocyclic spirocycloalkyl having 9 to 18 ring atoms, of which 1-3 ring atoms are heteroatoms. More preferred is (7-15 membered) heterocyclic spirocycloalkyl having 9 to 15 ring atoms, of which 1-3 ring atoms are heteroatoms. Most preferred is 9-membered (4-membered monocyclic (heterocyclyl) ring/6-membered monocyclic (heterocyclyl) ring, 5-membered monocyclic (heterocyclyl) ring/5-membered monocyclic (heterocyclyl) ring) monospiroheterocyclyl, 10-membered (5-membered monocyclic (heterocyclyl) ring/6-membered monocyclic (heterocyclyl) ring) monospiroheterocyclyl, or 11-membered (6-membered monocyclic (heterocyclyl) ring/6-membered monocyclic (heterocyclyl) ring) monospiroheterocyclyl. Specific examples of heterocyclic spirocycloalkyl include, but are not limited to, 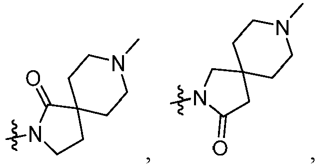 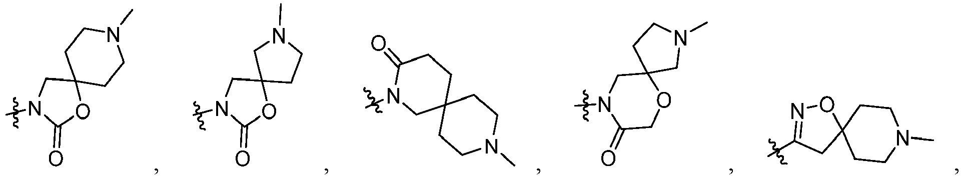 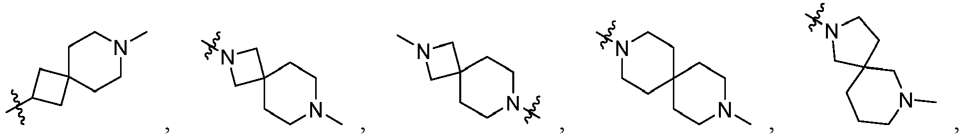 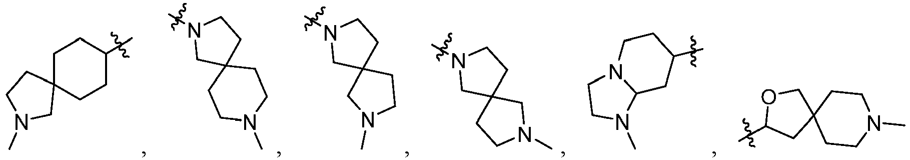 and

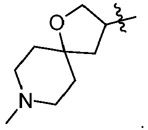

Unless otherwise specified, "oxo" refers to =O; for example, a group formed by substitution of carbon with one oxo is "carbonyl 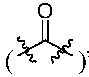 "; a group formed by substitution of sulfur with one oxo is "sulfinyl 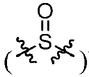 " and a group formed by substitution of sulfur with two oxos is "sulfonyl

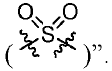

Unless otherwise specified, "halogen" (or halo) refers to fluorine, chlorine, bromine or iodine. The term "halo" (or "halogenated") before a group name indicates that the group is partially or fully halogenated, that is, substituted in any combination with F, Cl, Br or I, preferably with F or Cl.

"Optional" or "optionally" means that the subsequently described event or circumstance may, but does not necessarily, occur, and the description includes instances where the event or circumstance occurs and instances where the event or circumstance does not occur.

The substituent "-O-CH₂-O-" means that two oxygen atoms in the substituent are linked to two adjacent carbon atoms in the heterocycloalkyl, aryl or heteroaryl, for example:

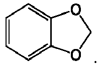

When the number of a linker group is 0, such as -(CH₂)₀-, it means that the linker group is a single bond.

When one of the variables is selected from a chemical bond, it means that the two groups linked by this variable are linked directly. For example, when L in X-L-Y represents a chemical bond, it means that the structure is actually X-Y.

The term "membered ring" includes any cyclic structure. The term "membered" is intended to refer to the number of backbone atoms that form a ring. For example, cyclohexyl, pyridinyl, pyranyl and thiopyranyl are six-membered rings, and cyclopentyl, pyrrolyl, furanyl and thienyl are five-membered rings.

The term "moiety" refers to a specific portion or functional group of a molecule. A chemical moiety is generally considered to be a chemical entity contained in or attached to a molecule.

Unless otherwise stated, the absolute configuration of a stereogenic center is represented by a wedged solid bond ( 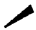 ) and a wedged dashed bond ( 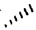 ), and the relative configuration of a stereogenic center is represented by a straight solid bond ( 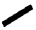 ) and a straight dashed bond ( 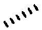 ). A wavy line ( 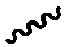 ) represents a wedged solid bond ( 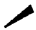 ) or a wedged dashed bond ( 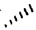 ), or a wavy line ( 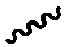 ) represents a straight solid bond ( 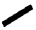 ) or a straight dashed bond ( 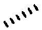 ).

Unless otherwise stated, a single bond or a double bond is represented by -̅ -̅ -̅.

### Specific Pharmaceutical and Medical Terminology

The term "acceptable", as used herein, means that a formula component or an active ingredient does not unduly and adversely affect a general therapeutic target's health.

The terms "treatment", "treatment course", and "therapy", as used herein, include alleviating, inhibiting, or ameliorating a symptom or condition of a disease; inhibiting the development of complications; ameliorating or preventing underlying metabolic syndrome; inhibiting the development of a disease or symptom, e.g., controlling the progression of a disease or condition; alleviating a disease or symptom; leading to disease or symptom regression; and alleviating a complication caused by a disease or symptom, or preventing or treating a sign caused by a disease or symptom. As used herein, a compound or pharmaceutical composition, when administered, can ameliorate a disease, symptom, or condition, which particularly refers to ameliorating the severity, delaying the onset, slowing the progression, or reducing the duration of the disease. Fixed or temporary administration, or continuous or intermittent administration, may be attributed to or associated with the administration.

"Active ingredient" refers to the compound of general formula (1), and pharmaceutically acceptable inorganic or organic salts of the compound of general formula (1). The compound of the present invention may contain one or more asymmetric centers (chiral center or axial chirality) and thus occurs in the forms of a racemate, a racemic mixture, a single enantiomer, a diastereomeric compound and a single diastereomer. Asymmetric centers that may be present depend on the nature of the various substituents on the molecule. Each of such asymmetric centers will independently produce two optical isomers, and all possible optical isomers, diastereomeric mixtures and pure or partially pure compounds are included within the scope of the present invention. The present invention is meant to include all such isomeric forms of these compounds.

The terms such as "compound", "composition", "agent", or "medicine or medicament" are used interchangeably herein and all refer to a compound or composition that, when administered to an individual (human or animal), is capable of inducing a desired pharmacological and/or physiological response by local and/or systemic action.

The term "administered, administering, or administration" refers herein to the direct administration of the compound or composition, or the administration of a prodrug, derivative, analog, or the like of the active compound.

Although the numerical ranges and parameters defining the broad scope of the present invention are approximations, the related numerical values set forth in the specific examples have been presented herein as precisely as possible. Any numerical value, however, inherently contains a standard deviation necessarily resulting from certain methods of testing. Herein, "about" generally means that the actual numerical value is within a particular numerical value or range ± 10%, 5%, 1% or 0.5%. Alternatively, the term "about" indicates that the actual numerical value falls within the acceptable standard error of a mean, as considered by those skilled in the art. All ranges, quantities, numerical values, and percentages used herein (e.g., to describe an amount of a material, a length of time, a temperature, an operating condition, a quantitative ratio, and the like) are to be understood as being modified by the word "about", except in the experimental examples or where otherwise explicitly indicated. Accordingly, unless otherwise contrarily stated, the numerical parameters set forth in the specification and the appended claims are all approximations that may vary as desired. At least, these numerical parameters should be understood as the significant digits indicated or the numerical values obtained using conventional rounding rules.

Unless otherwise defined in the specification, the scientific and technical terms used herein have the same meaning as commonly understood by those skilled in the art. Furthermore, nouns in their singular forms used in the specification encompass their plural forms, unless contradicted by context; nouns in their plural forms used also encompass their singular forms.

### Therapeutic Use

The present invention provides a method for treating a disease, including but not limited to a related condition involving FAK protein kinase (e.g., cancer), with the compound of general formula (1) or the pharmaceutical composition of the present invention.

In some embodiments, a method for treating cancer is provided, the method including administering to an individual in need thereof an effective amount of any aforementioned pharmaceutical composition comprising the compound of structural general formula (1). In some embodiments, the cancer is associated with FAK kinase. In other embodiments, the cancer is a hematologic cancer and a solid tumor, including but not limited to, leukemia, breast cancer, lung cancer, pancreatic cancer, colon cancer, bladder cancer, brain cancer, urothelial cancer, prostate cancer, liver cancer, ovarian cancer, head and neck cancer, gastric cancer, mesothelioma or all cancer metastases.

### Route of administration

The compound and the pharmaceutically acceptable salt thereof of the present invention can be made into various formulations comprising a safe and effective amount of the compound or the pharmaceutically acceptable salt thereof of the present invention, and a pharmaceutically acceptable excipient or carrier, wherein the "safe and effective amount" means that the amount of the compound is sufficient to significantly improve the condition without causing serious side effects. The safe and effective amount of the compound is determined according to the age, condition, course of treatment, and other specific conditions of a treated subject.

The "pharmaceutically acceptable excipient or carrier" refers to one or more compatible solid or liquid fillers or gel substances that are suitable for human use and must be of sufficient purity and sufficiently low toxicity. "Compatible" herein means that the components of the composition are capable of intermixing with the compound of the present invention and with each other, without significantly diminishing the pharmaceutical efficacy of the compound. Examples of pharmaceutically acceptable excipients or carriers include cellulose and derivatives thereof (e.g., sodium carboxymethylcellulose, sodium ethylcellulose, or cellulose acetate), gelatin, talc, solid lubricants (e.g., stearic acid or magnesium stearate), calcium sulfate, vegetable oil (e.g., soybean oil, sesame oil, peanut oil, or olive oil), polyols (e.g., propylene glycol, glycerol, mannitol, or sorbitol), emulsifiers (e.g., Tween^{®}), wetting agents (e.g., sodium lauryl sulfate), colorants, flavoring agents, stabilizers, antioxidants, preservatives, pyrogen-free water, etc.

When the compound of the present invention is administered, it may be administered orally, rectally, parenterally (intravenously, intramuscularly, or subcutaneously), or topically.

Solid dosage forms for oral administration include capsules, tablets, pills, pulvises, and granules. In these solid dosage forms, the active compound is mixed with at least one conventional inert excipient (or carrier), such as sodium citrate or dicalcium phosphate, or with the following ingredients: (a) fillers or extenders, such as starch, lactose, sucrose, glucose, mannitol, and silicic acid; (b) binders, such as hydroxymethyl cellulose, alginate, gelatin, polyvinylpyrrolidone, sucrose, and acacia; (c) humectants, such as glycerol; (d) disintegrants, such as agar, calcium carbonate, potato or tapioca starch, alginic acid, certain complex silicates, and sodium carbonate; (e) solution retarders, such as paraffin; (f) absorption accelerators, such as quaternary ammonium compounds; (g) wetting agents, such as cetyl alcohol and glycerol monostearate; (h) adsorbents, such as kaolin; and (i) lubricants, such as talc, calcium stearate, magnesium stearate, solid polyethylene glycol and sodium lauryl sulfate, or mixtures thereof. In the case of capsules, tablets, and pills, the dosage forms may further include buffers.

Solid dosage forms such as tablets, dragees, capsules, pills and granules can be prepared using coatings and shells such as enteric coatings and other materials well known in the art. They may include opacifying agents, and the active compound or compound in such a composition may be released in a certain part of the digestive tract in a delayed manner. Examples of embedding components that can be used are polymeric substances and wax-based substances. If necessary, the active compound can also be in microcapsule form with one or more of the excipients described above.

Liquid dosage forms for oral administration include pharmaceutically acceptable emulsions, solutions, suspensions, syrups, and elixirs. In addition to the active compound, the liquid dosage form may include inert diluents commonly used in the art, such as water or other solvents, solubilizers and emulsifiers, for example, ethanol, isopropanol, ethyl carbonate, ethyl acetate, propylene glycol, 1,3-butanediol, dimethylformamide, and oils, especially cottonseed oil, peanut oil, corn germ oil, olive oil, castor oil and sesame oil, or mixtures of these substances.

Besides such inert diluents, the composition may further include adjuvants, such as wetting agents, emulsifiers, suspending agents, sweeteners, flavoring agents, and perfuming agents.

In addition to the active compound, suspensions may include suspending agents, such as ethoxylated isostearyl alcohols, polyoxyethylene sorbitol and sorbitan esters, microcrystalline cellulose, aluminum methylate and agar, or mixtures of these substances.

Compositions for parenteral injection may include physiologically acceptable sterile aqueous or anhydrous solutions, dispersions, suspensions or emulsions, and sterile powders for redissolving into sterile injectable solutions or dispersions. Suitable aqueous and non-aqueous carriers, diluents, solvents, or excipients include water, ethanol, polyols, and suitable mixtures thereof. Dosage forms for topical administration of the compound of the present invention include ointments, pulvises, patches, sprays, and inhalants. The active ingredient is mixed under sterile conditions with a physiologically acceptable carrier and any preservatives, buffers or propellants that may be required if necessary.

The compound of the present invention may be administered alone or in combination with other pharmaceutically acceptable compounds. When the pharmaceutical composition is used, a safe and effective amount of the compound of the present invention is administered to a mammal (such as a human) to be treated, wherein the dose of administration is a pharmaceutically effective dose. For a human of 60 kg, the daily dose of administration is usually 1-2000 mg, preferably 50-1000 mg. In determining a specific dose, such factors as the route of administration, the health condition of the patient and the like will also be considered, which are well-known to skilled physicians.

The above features mentioned in the present invention or those mentioned in the examples may be combined arbitrarily. All the features disclosed in this specification may be used with any composition form and the various features disclosed in this specification may be replaced with any alternative features that provide the same, equivalent, or similar purpose. Thus, unless otherwise specified, the features disclosed herein are merely general examples of equivalent or similar features.

### DETAILED DESCRIPTION

Various specific aspects, features, and advantages of the compounds, methods, and pharmaceutical compositions described above will be set forth in detail in the following description, which will make the content of the present invention very clear. It should be understood that the detailed description and examples below describe specific examples for reference only. After reading the description of the present invention, those skilled in the art can make various changes or modifications to the present invention, and such equivalents also fall within the scope of the present application defined herein.

In all the examples, ¹H-NMR spectra were recorded with a Varian Mercury 400 nuclear magnetic resonance spectrometer, and chemical shifts are represented by δ (ppm); silica gel for separation was 200-300 mesh silica gel if not specified, and the ratio of the eluents was a volume ratio. The following abbreviations are used in the present invention: ACN for acetonitrile; AcOH for glacial acetic acid; (Boc)₂O for di-*tert*-butyl dicarbonate; BPO for benzoyl peroxide; CCl₄ for carbon tetrachloride; CDCl₃ for deuterated chloroform; CD₃I for deuterated iodomethane; CuBr₂ for cupric bromide; Cs₂CO₃ for anhydrous cesium carbonate; DCM for dichloromethane; DIPEA for diisopropylethylamine; Dioxane for 1,4-dioxane; DMF for N,N-dimethylformamide; DMAP for 4-(dimethylamino)pyridine; DMSO for dimethyl sulfoxide; EA for ethyl acetate; EDCI for 1-(3-dimethylaminopropyl)-3-ethylcarbodiimide hydrochloride; Flash for purification by flash preparative medium-pressure liquid chromatography; H₂ for hydrogen; HCHO for formaldehyde; Hexane for *n*-hexane; HOBt for 1-hydroxybenzotriazole; HPLC for high-performance liquid chromatography; h for hour; HCl/Diox for a solution of hydrogen chloride in 1,4-dioxane; IPA for isopropanol; IV for intravenous administration; K₂CO₃ for potassium carbonate; KOAc for potassium acetate; K₃PO₄ for potassium phosphate; LiOH for lithium hydroxide; min for minute; MeOH for methanol; mL for milliliter; MS for mass spectrometry; MsOH for methanesulfonic acid; m-CPCA for *m*-chloroperoxybenzoic acid; NaBH(OAc)₃ for sodium triacetoxyborohydride; NaNO₂ for sodium nitrite; NBS for N-bromosuccinimide; *n-*BuLi for *n*-butyllithium; NaBH₄ for sodium borohydride; n-BuOH for *n*-butanol; NH₃/THF for a solution of ammonia in tetrahydrofuran; NMP for N-methylpyrrolidone; NMR for nuclear magnetic resonance; Oxone for potassium peroxymonosulfonate; Pd/C for palladium on carbon; Pd(PPh₃)₄ for tetrakis(triphenylphosphine)palladium; Pd₂(dba)₃ for tris(dibenzylideneacetone)dipalladium(0); PE for petroleum ether; PPTS for pyridinium *p-*toluenesulfonate; PO for oral administration; QD for administration once a day; QW for administration once a week; SOCl₂ for thionyl chloride; TFA for trifluoroacetic acid; THF for tetrahydrofuran; TLC for thin-layer chromatography; Toluene for methylbenzene; T₃P for 1-propanephosphonic anhydride; XantPhos for 4,5-bis(diphenylphosphino)-9,9-dimethylxanthene; XPhos for 2-dicyclohexylphosphonium-2',4',6'-triisopropylbiphenyl; Zn for zinc powder.

**Example 1. Synthesis of 2-fluoro-5-methoxy-N-(7-methyl-7-azaspiro[3.5]nonan-2-yl)-4-((4-(2-(methylcarbamoyl)phenoxy)-5-(trifluoromethyl)pyrimidin-2-yl)amino)benzamide**

### (Compound 1)

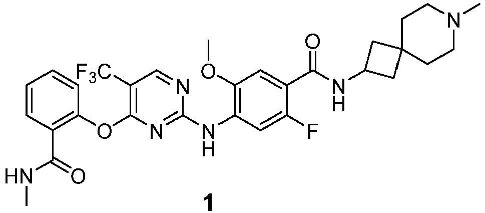

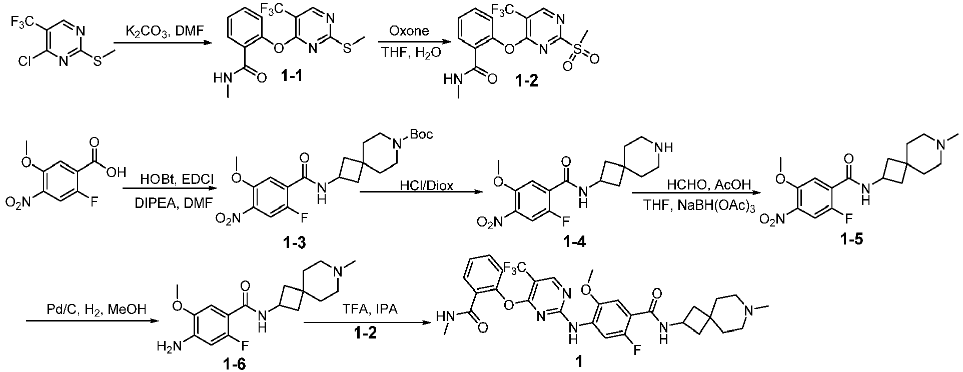

### Synthesis of 1-1:

**2-Hydroxy-N-methylbenzamide** (250 mg, 1.65 mmol) and anhydrous potassium carbonate (456 mg, 3.3 mmol) were added to DMF (11 mL), and the mixed solution was purged with argon. A solution of **4-chloro-2-(methylthio)-5-(trifluoromethyl)pyrimidine** (378 mg, 1.65 mmol) in DMF (5 mL) was added dropwise at room temperature. After the dropwise addition was completed, the mixed solution was stirred at room temperature for 3 h. After the completion of the reaction as detected by LC-MS, the system was quenched with water (30 mL). The mixed solution was stirred at room temperature for 30 min and then filtered to give an impure product.

This impure product was purified by Flash to give a white solid product (299 mg, yield: 53%).

ESI-MS m/z: 344.0 [M+H]⁺.

### Synthesis of 1-2:

Compound **1-1** (200 mg, 0.583 mmol) was added to a mixed solution of THF (10 mL)/water (2 mL), and Oxone (1.8 g, 2.93 mmol) was added. The mixture was stirred at room temperature for 20 h. After the completion of the reaction as detected by LC-MS, water (20 mL) was added to the system, followed by extraction with EA (20 mL). The organic phase was washed twice with saturated sodium chloride solution (20 mL), dried over anhydrous sodium sulfate and filtered, and the filtrate was concentrated to dryness to give a crude product as a light brown solid (248 mg, yield: > 100%).

ESI-MS m/z: 376.0 [M+H]⁺.

### Synthesis of 1-3:

**2-Fluoro-5-methoxy-4-nitrobenzoic acid** (610 mg, 2.08 mmol), ***tert*-butyl 2-amino-7-azaspiro[3.5]nonane-7-carboxylate** (500 mg, 2.08 mol), HOBt (420 mg, 3.1 mmol), EDCI (598 mg, 3.1 mg) and DIPEA (1.1 mL) were added to DMF (10 mL). The mixture was purged with argon and then stirred at room temperature for 20 h. After the completion of the reaction as detected by LC-MS, water (20 mL) was added to the mixed solution. The resulting mixture was stirred at room temperature for 30 min and then filtered. The filter cake was washed with water and dried under vacuum to give a product (930 mg, yield: > 100%).

ESI-MS m/z: 438.2 [M+H]⁺.

### Synthesis of 1-4:

**1-3** described above (930 mg, crude, 2.08 mmol) was added to EA (10 mL). After complete dissolution, 4 M HCl/Diox (10 mL, 40 mmol) was added. The mixture was stirred at room temperature for 3 h. After the completion of the reaction as detected by LC-MS, the mixture was concentrated to dryness. EA (20 mL) and saturated sodium bicarbonate solution (20 mL) were added to the residue. The resulting mixture was stirred, and liquid separation was performed. The aqueous phase was extracted twice with EA (20 mL × 2). The organic phases were combined, washed with saturated sodium chloride solution, dried over anhydrous sodium sulfate and filtered. The filtrate was concentrated to dryness to give a brownish-yellow oil (508 mg, yield: 72.4%).

ESI-MS m/z: 338.1 [M+H]⁺.

### Synthesis of 1-5:

Compound **1-4** described above (508 mg, 1.51 mmol) was added to THF (10 mL), and glacial acetic acid (136 mg, 2.265 mmol) and an aqueous formaldehyde solution (147 mg, 37%, 1.8 mmol) were added at room temperature. The mixed solution was stirred at room temperature for 30 min. NaBH(OAc)₃ (479 mg, 2.26 mmol) was then added, and the mixed solution was stirred at room temperature for another 20 h. After the completion of the reaction as detected by LC-MS, EA (20 mL) and saturated sodium bicarbonate solution (20 mL) were added to the mixed solution. The resulting mixture was stirred for 20 min, and liquid separation was then performed.

The aqueous phase was extracted twice with EA (20 mL × 2). The organic phases were combined, washed with saturated sodium chloride solution, dried over anhydrous sodium sulfate and filtered. The filtrate was concentrated to dryness to give a crude product as a brown oil (630 mg, yield: > 100%).

ESI-MS m/z: 352.1 [M+H]⁺.

### Synthesis of 1-6:

**1-5** described above (630 mg, crude, 1.51 mmol) and 10% wet Pd/C (100 mg, containing 50%-55% water) were added to MeOH (20 mL). The mixed solution was purged with hydrogen three times and then vigorously stirred at room temperature under normal pressure for 20 h. After the completion of the reaction as detected by LC-MS, the mixed solution was filtered through celite. The filter cake was rinsed with MeOH, and the filtrate was concentrated to dryness to give a white solid product (433 mg, yield: 89%).

ESI-MS m/z: 322.1 [M+H]⁺.

### Synthesis of compound 1:

Compounds **1-6** (50 mg, 0.156 mmol) and **1-2** (70 mg, 0.187 mmol) described above were added to isopropanol (5 mL), and TFA (35.6 mg, 0.312 mmol) was added at room temperature. The mixed solution was purged with argon, heated to 80 °C and stirred for 3 h. After the basic completion of the reaction as detected by LC-MS, the mixture was concentrated, and the residue was purified by Flash and lyophilized to give an off-white solid product (18 mg, yield: 18.7%). ESI-MS m/z: 617.2 [M+H]⁺.

### Examples 2-18. Synthesis of compounds 2-18

The target compounds 2-18 in Table 1 were obtained with reference to a similar synthetic method to that in Example 1 using different starting materials.

**Table 1**

| **Compound** | **Structural formula** | **MS: [M+H]⁺** | **Compound** | **Structural formula** | **MS: [M+H]⁺** |
|---|---|---|---|---|---|
| **2** | 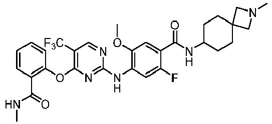 | **617.2** | **3** | 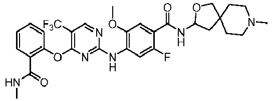 | **633.2** |
| **4** | 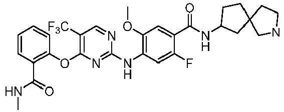 | **617.2** | **5** | 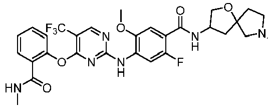 | **619.2** |
| **6** | 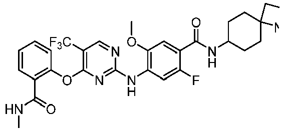 | **617.2** | **7** | 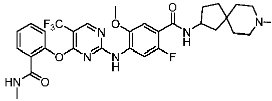 | **631.2** |
| **8** | 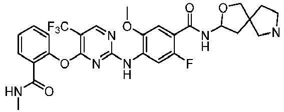 | **619.2** | **9** | 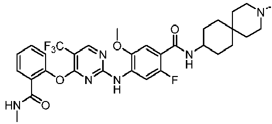 | **645.2** |
| **10** | 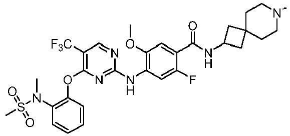 | **667.2** | **11** | 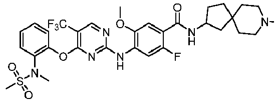 | **681.2** |
| **12** | 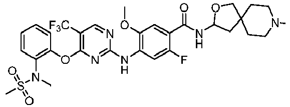 | **683.2** | **13** | 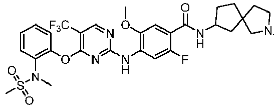 | **667.2** |
| **14** | 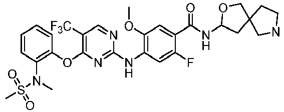 | **669.2** | **15** | 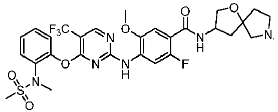 | **669.2** |
| **16** | 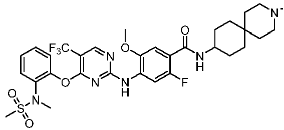 | **695.2** | **17** | 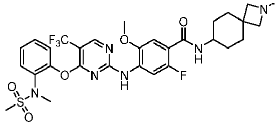 | **667.2** |
| **18** | 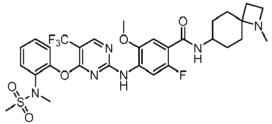 | **667.2** | | | |

### Example 19. Synthesis of 2-fluoro-5-methoxy-4-((4-((2-methyl-3-oxoisoindolin-4-yl)oxy)-5-(trifluoromethyl)pyrimidin-2-yl)amino)-N-(7-methyl-7-azaspiro[3.5]nonan-2-yl)benzamide (Compound 19)

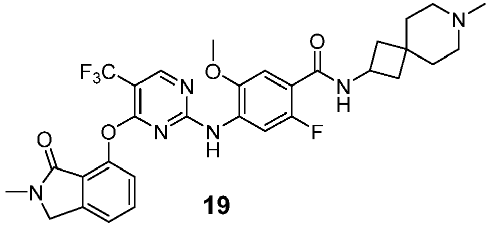

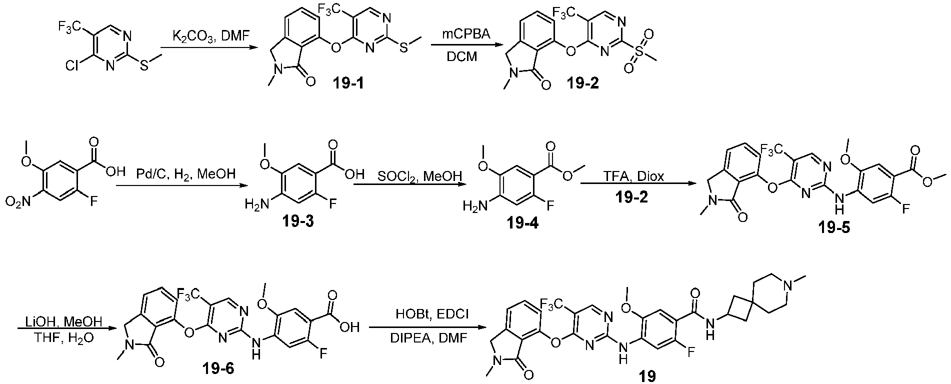

### Synthesis of 19-1:

**7-Hydroxy-2-methylisoindolin-1-one** (1.7 g, 10.44 mmol) and anhydrous potassium carbonate (2.42 g, 17.5 mmol) were added to DMF (60 mL), and the mixed solution was purged with argon.

A solution of **4-chloro-2-(methylthio)-5-(trifluoromethyl)pyrimidine** (2 g, 8.7 mmol) in DMF (10 mL) was added dropwise at room temperature. After the dropwise addition was completed, the mixed solution was stirred at room temperature for 20 h. After the completion of the reaction as detected by LC-MS, the system was quenched with water (30 mL). The mixed solution was stirred at room temperature for 30 min and then filtered. The filter cake was rinsed with water and dried under vacuum to give an off-white solid product (3.048 g, yield: 98%).

ESI-MS m/z: 356.0 [M+H]⁺.

### Synthesis of 19-2:

Compound **19-1** (3.048 g, 8.6 mmol) was added to DCM (50 mL), and mCPBA (6.1 g, 30.1 mmol) was added in batches in an ice bath. The mixed solution was stirred at room temperature for 20 h. After the completion of the reaction as detected by LC-MS, the mixed solution was quenched with saturated sodium thiosulfate (50 mL) and saturated sodium bicarbonate solution (50 mL). The resulting mixture was stirred at room temperature for 15 min, and liquid separation was then performed. The aqueous phase was extracted with DCM (50 mL). The organic phases were combined, washed twice with saturated sodium chloride solution (50 mL × 2), dried over anhydrous sodium sulfate and filtered, and the filtrate was concentrated to dryness to give a crude product as a white solid (3.38 g, yield: > 100%).

ESI-MS m/z: 388.0 [M+H]⁺.

### Synthesis of 19-3:

2-Fluoro-5-methoxy-4-nitrobenzoic acid (32 g, 0.149 mol) and 10% wet Pd/C (3 g, containing 50%-55% water) were added to MeOH (300 mL). The mixed solution was purged with hydrogen three times and then vigorously stirred at room temperature under normal pressure for 20 h. After the completion of the reaction as detected by LC-MS, the mixed solution was filtered through celite. The filter cake was rinsed with MeOH, and the filtrate was concentrated to dryness to give a pale gray product (29 g, yield: > 100%).

ESI-MS m/z: 186.0 [M+H]⁺.

### Synthesis of 19-4:

Compound **19-3** described above (29 g, 0.149 mol) was added to MeOH (300 mL), and SOCl₂ (35.7 g, 0.3 mol) was slowly added dropwise in an ice bath. After the dropwise addition was completed, the mixed solution was stirred at a constant temperature of 40 °C for 20 h. After the completion of the reaction as detected by LC-MS, the mixed solution was concentrated, and EA (300 mL) was added to the residue. Saturated sodium bicarbonate solution (300 mL) was then carefully added to adjust the pH to 7-8. The mixed solution was stirred at room temperature for 10 min, and liquid separation was then performed. The aqueous phase was extracted with EA (100 mL). The organic phases were combined, washed with saturated sodium chloride solution (200 mL × 2), dried over anhydrous sodium sulfate and filtered, and the filtrate was concentrated to dryness to give a brown solid product (27 g, yield: 91%).

ESI-MS m/z: 200.0 [M+H]⁺.

### Synthesis of 19-5:

Compounds **19-4** (1.028 g, 5.2 mmol) and **19-2** (2 g, crude, 5.2 mmol) described above were added to Diox (20 mL), and TFA (593 mg, 5.2 mmol) was added at room temperature. The mixed solution was purged with argon, heated to 95 °C and stirred for 3 h. After the basic completion of the reaction as detected by LC-MS, the mixture was concentrated, and the residue was purified by Flash and lyophilized to give an off-white solid product (1.432 g, yield: 43%).

ESI-MS m/z: 507.1 [M+H]⁺.

### Synthesis of 19-6:

Compound **19-5** described above (1.4 g, 2.76 mmol) was added to THF (20 mL)/MeOH (10 mL)/water (10 mL), and lithium hydroxide monohydrate (464 mg, 11.06 mmol) was added at room temperature. The mixed solution was stirred at room temperature for 6 h. After the completion of the reaction as detected by LC-MS, the mixed solution was concentrated to a small amount, and water (10 mL) was then added. The pH was adjusted to 2-3 with 2 N HCl aq, and the resulting mixture was extracted three times with EA (30 mL × 3). The organic phases were combined, washed with saturated sodium chloride solution (20 mL × 2), dried over anhydrous sodium sulfate and filtered, and the filtrate was concentrated to dryness to give a light brown solid product (1.029 g, yield: 71%).

ESI-MS m/z: 493.0 [M+H]⁺.

### Synthesis of compound 19:

Compound **19-6** described above (100 mg, 0.203 mmol), **7-methyl-7-azaspiro[3.5]nonan-2-amine** (32 mg, 0.208 mol), HOBt (42 mg, 0.31 mmol), EDCI (60 mg, 0.31 mg) and DIPEA (0.11 mL) were added to DMF (5 mL). The mixture was purged with argon and then stirred at room temperature for 20 h. After the completion of the reaction as detected by LC-MS, the mixed solution was purified by Flash and lyophilized to give a white solid product (56 mg, yield: 44%). ESI-MS m/z: 629.2 [M+H]⁺.

### Examples 20-48. Synthesis of compounds 20-48

The target compounds 20-48 in Table 2 were obtained with reference to a similar synthetic method to that in Example 2 using different starting materials.

**Table 2**

| **Compound** | **Structural formula** | **MS: [M+H]⁺** | **Compound** | **Structural formula** | **MS: [M+H]⁺** |
|---|---|---|---|---|---|
| **20** | 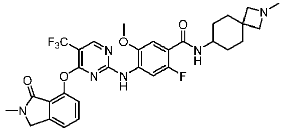 | **629.2** | **21** | 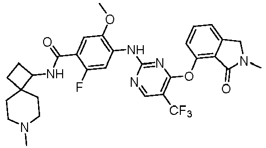 | **629.2** |
| **22** | 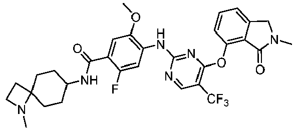 | **629.2** | **23** | 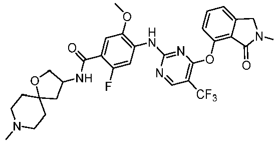 | **645.2** |
| **24** | 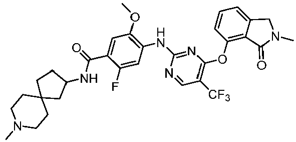 | **643.2** | **25** | 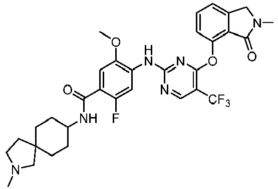 | **643.2** |
| **26** | 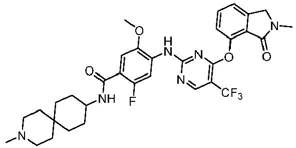 | **657.2** | **27** | 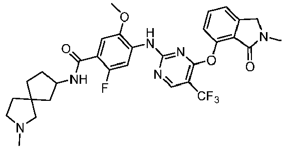 | **629.2** |
| **28** | 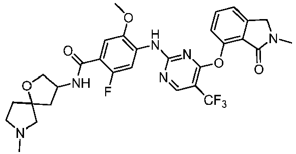 | **631.2** | **29** | 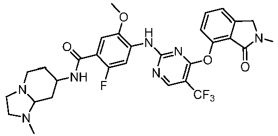 | **630.2** |
| **30** | 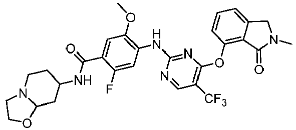 | **617.2** | **31** | 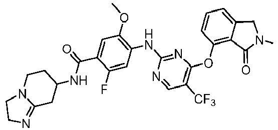 | **614.2** |
| **32** | 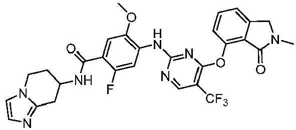 | **612.1** | **33** | 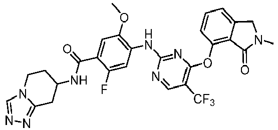 | **613.1** |
| **34** | 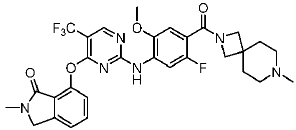 | **615.2** | **35** | 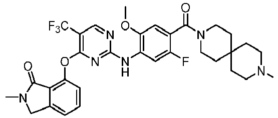 | **643.2** |
| **36** | 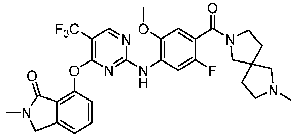 | **615.2** | **37** | 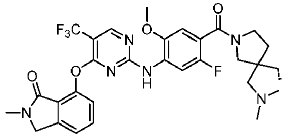 | **615.2** |
| **38** | 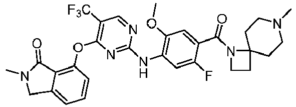 | **615.2** | **39** | 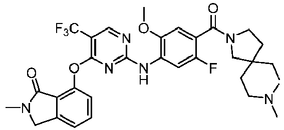 | **629.2** |
| **40** | 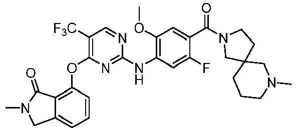 | **629.2** | **41** | 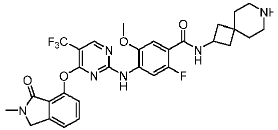 | **614.2** |
| **42** | 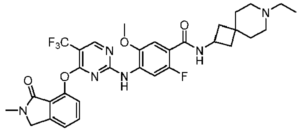 | **643.2** | **43** | 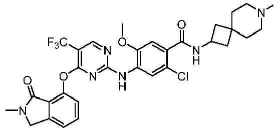 | **645.1** |
| **44** | 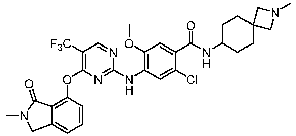 | **645.1** | **45** | 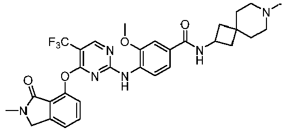 | **611.2** |
| **46** | 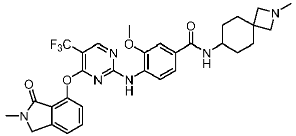 | **611.2** | **47** | 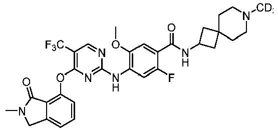 | **632.2** |
| **48** | 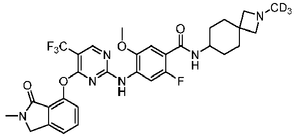 | **632.2** | | | |

### Example 49. Synthesis of 2-fluoro-5-methoxy-4-((4-((2-methyl-3-oxoisoindolin-4-yl)methyl)-5-(trifluoromethyl)pyrimidin-2-yl)amino)-N-(7-methyl-7-azaspiro[3.5]nonan-2-yl)benzamide (Compound 49)

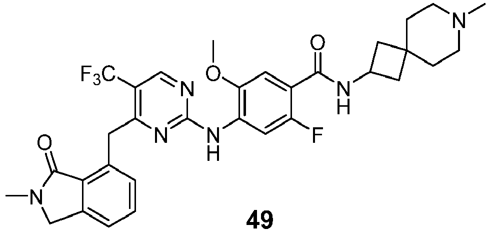

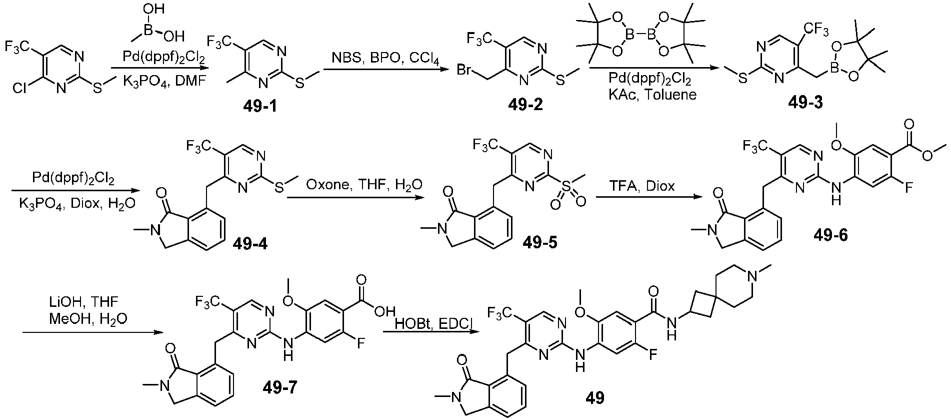

### Synthesis of 49-1:

**4-Chloro-2-(methylthio)-5-(trifluoromethyl)pyrimidine** (5 g, 21.93 mmol), anhydrous potassium phosphate (13.9 g, 65.8 mmol) and methylboronic acid (4 g, 65.8 mmol) were added to DMF (100 mL). After the mixture was purged with argon, Pd(dppf)₂Cl₂ (1.61 g, 2.2 mmol) was added. The mixed solution was heated to 100 °C and stirred for 20 h under argon atmosphere.

After the completion of the reaction as detected by LC-MS, the mixed solution was cooled to room temperature and filtered through celite, and the filter cake was washed with EA (50 mL).

The filtrate was concentrated, and the residue was purified by column chromatography to give a product (3.28 g, yield: 72%).

ESI-MS m/z: 209.0 [M+H]⁺.

### Synthesis of 49-2:

Compound **49-1** described above (3.2 g, 15.38 mmol) and BPO (372 mg, 1.538 mmol) were added to carbon tetrachloride (100 mL). After the mixture was purged with argon, NBS (3.29 g, 18.456 mmol) was added in batches. The mixed solution was heated to 80 °C and stirred for 20 h under argon atmosphere. After the completion of the reaction as detected by LC-MS, the mixed solution was concentrated to dryness, and the residue was purified by Flash to give a product (1.62 g, yield: 36.8%).

ESI-MS m/z: 287.0/289.0 [M+H]⁺.

### Synthesis of 49-3:

Compound **49-2** described above (1.62 g, 5.644 mmol), bis(pinacolato)diboron (2.15 g, 8.467 mmol) and potassium acetate (1.66 g, 16.932 mmol) were added to toluene (50 mL). After the mixture was purged with argon, Pd(dppf)₂Cl₂ (402 mg, 0.55 mmol) was added. The mixed solution was heated to 50 °C and stirred for 20 h under argon atmosphere. After the completion of the reaction as detected by LC-MS, the mixed solution was purified by column chromatography to give a product (1.31 g, yield: 69.5%).

ESI-MS m/z: 335.1 [M+H]⁺.

### Synthesis of 49-4:

Compound **49-3** described above (1.22 g, 3.653 mmol), 7-bromo-2-methylisoindolin-1-one (0.991 g, 4.383 mmol) and potassium phosphate (2.323 g, 10.96 mmol) were added to Diox (50 mL)/water (10 mL). After the mixture was purged with argon, Pd(dppf)₂Cl₂ (270 mg, 0.37 mmol) was added. The mixed solution was heated to 100 °C and stirred for 20 h under argon atmosphere.

After the completion of the reaction as detected by LC-MS, the mixed solution was concentrated, and the residue was purified by Flash to give a product (735 mg, yield: 57%).

ESI-MS m/z: 354.1 [M+H]⁺.

### Synthesis of 49-5:

Compound **49-4** described above (700 mg, 1.983 mmol) was added to a mixed solution of THF (10 mL)/water (2 mL), and Oxone (4.9 g, 7.93 mmol) was added. The mixture was stirred at room temperature for 6 h. After the completion of the reaction as detected by LC-MS, water (20 mL) was added to the system, followed by extraction with EA (20 mL). The organic phase was washed twice with saturated sodium chloride solution (20 mL), dried over anhydrous sodium sulfate and filtered, and the filtrate was concentrated to dryness to give a crude product as a light brown solid (760 mg, yield: > 100%).

ESI-MS m/z: 386.0 [M+H]⁺.

### Synthesis of 49-6:

Compounds **49-5** (500 mg, 1.3 mmol) and **19-4** (259 mg, 1.3 mmol) described above were added to Diox (10 mL), and TFA (296 mg, 2.6 mmol) was added at room temperature. The mixed solution was purged with argon, heated to 95 °C and stirred for 5 h. After the completion of the reaction as detected by LC-MS, the mixture was concentrated, and the residue was purified by Flash and lyophilized to give an off-white solid product (472 mg, yield: 72%).

ESI-MS m/z: 505.1 [M+H]⁺.

### Synthesis of 49-7:

Compound **49-6** described above (450 mg, 0.893 mmol) was added to THF (10 mL)/MeOH (5 mL)/water (5 mL), and lithium hydroxide monohydrate (188 mg, 4.465 mmol) was added at room temperature. The mixed solution was stirred at room temperature for 6 h. After the completion of the reaction as detected by LC-MS, the mixed solution was concentrated to a small amount, and water (10 mL) was then added. The pH was adjusted to 2-3 with 2 N HCl aq, and the resulting mixture was extracted three times with EA (20 mL × 3). The organic phases were combined, washed with saturated sodium chloride solution (20 mL × 2), dried over anhydrous sodium sulfate and filtered, and the filtrate was concentrated to dryness to give a light brown solid product (271 mg, yield: 62%).

ESI-MS m/z: 491.0 [M+H]⁺.

### Synthesis of compound 49:

Compound **49-7** described above (50 mg, 0.102 mmol), 7-methyl-7-azaspiro[3.5]nonan-2-amine (16 mg, 0.104 mol), HOBt (21 mg, 0.155 mmol), EDCI (30 mg, 0.155 mg) and DIPEA (39 mg, 0.302 mmol) were added to DMF (5 mL). The mixture was purged with argon and then stirred at room temperature for 20 h. After the completion of the reaction as detected by LC-MS, the mixed solution was purified by pre-TLC (DCM:MeOH = 20: 1) to give a white solid product (12 mg, yield: 18.8%).

ESI-MS m/z: 627.2 [M+H]⁺.

### Examples 50-60. Synthesis of compounds 50-60

The target compounds 50-60 in Table 3 were obtained with reference to a similar synthetic method to that in Example 49 using different starting materials.

**Table 3**

| **Compound** | **Structural formula** | **MS: [M+H]⁺** | **Compound** | **Structural formula** | **MS: [M+H]⁺** |
|---|---|---|---|---|---|
| **50** | 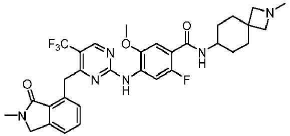 | **627.2** | **51** | 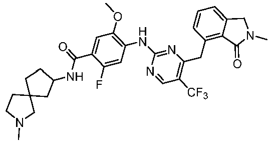 | **627.2** |
| **52** | 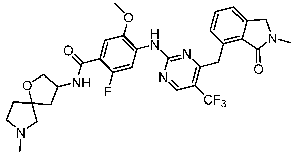 | **629.2** | **53** | 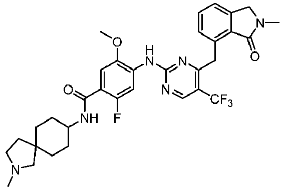 | **641.2** |
| **54** | 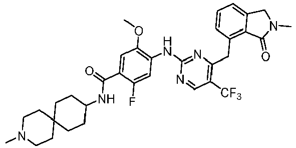 | **655.2** | **55** | 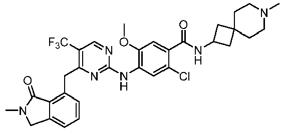 | **643.2** |
| **56** | 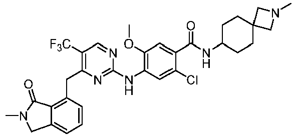 | **643.2** | **57** | 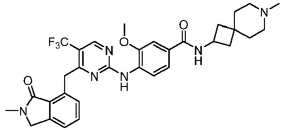 | **609.2** |
| **58** | 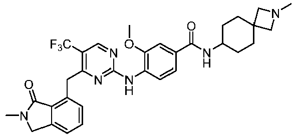 | **609.2** | **59** | 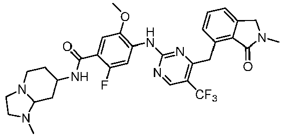 | **628.2** |
| **60** | 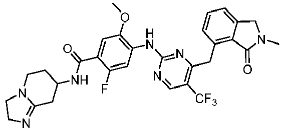 | **612.2** | | | |

### Example 61. Synthesis of 2-(2-fluoro-5-methoxy-4-((4-((2-methyl-3-oxoisoindol-4-yl)oxy)-5-(trifluoromethyl)pyrimidin-2-yl)amino)phenyl)-8-methyl-2,8-diazaspiro[4.5]decan-1-one (Compound 61)

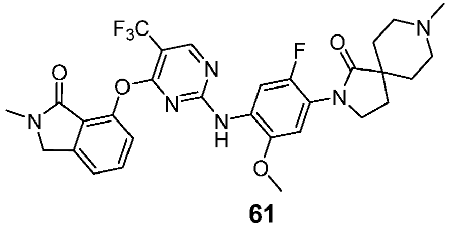

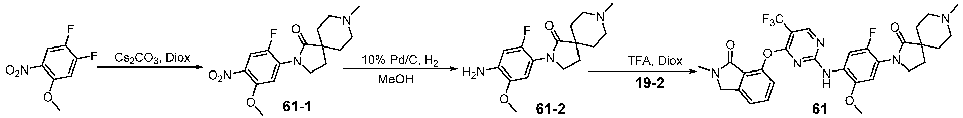

### Synthesis of 61-1:

**1,2-Difluoro-4-methoxy-5-nitrobenzene** (250 mg, 1.323 mmol) and anhydrous cesium carbonate (864 mg, 2.65 mmol) were added to Diox (10 mL). The mixed solution was purged with argon, heated to 50 °C and stirred for 4 h. After the completion of the reaction as detected by LC-MS, the system was quenched with water (20 mL). The mixed solution was stirred at room temperature for 30 min and then filtered. The filter cake was washed with water and dried under vacuum to give a brown solid product (321 mg, yield: 72%).

ESI-MS m/z: 338.1 [M+H]⁺.

### Synthesis of 61-2:

Compound **61-1** described above (321 mg, 0.953 mmol) and 10% wet Pd/C (50 mg, containing 50%-55% water) were added to MeOH (10 mL). The mixed solution was purged with hydrogen three times and then vigorously stirred at room temperature under normal pressure for 20 h. After the completion of the reaction as detected by LC-MS, the mixed solution was filtered through celite. The filter cake was rinsed with MeOH, and the filtrate was concentrated to dryness to give a brown-black solid product (320 mg, yield: > 100%).

ESI-MS m/z: 308.1 [M+H]⁺.

### Synthesis of compound 61:

Compounds **61-2** (50 mg, 0.163 mmol) and **19-2** (50 mg, 0.129 mmol) described above were added to Diox (5 mL), and TFA (18.6 mg, 0.163 mmol) was added at room temperature. The mixed solution was purged with argon, heated to 80 °C and stirred for 3 h. After the basic completion of the reaction as detected by LC-MS, the mixture was concentrated, and the residue was purified by Pre-TLC (DCM:MeOH = 20:1) to give an off-white solid product (9 mg, yield: 11.4%).

ESI-MS m/z: 615.2 [M+H]⁺.

### Examples 62-66. Synthesis of compounds 62-66

The target compounds 62-66 in Table 4 were obtained with reference to a similar synthetic method to that in Example 61 using different starting materials.

**Table 4**

| **Compound** | **Structural formula** | **MS: [M+H]⁺** | **Compound** | **Structural formula** | **MS: [M+H]⁺** |
|---|---|---|---|---|---|
| **62** | 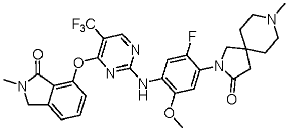 | **614.2** | **63** | 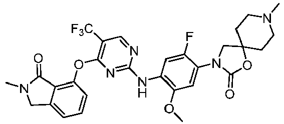 | **617.2** |
| **64** | 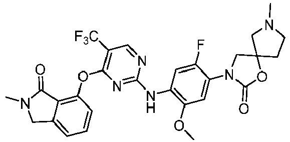 | **603.1** | **65** | 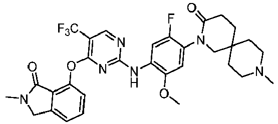 | **629.2** |
| **66** | 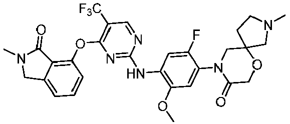 | **617.2** | | | |

### Example 67. Synthesis of 2-(2-fluoro-5-methoxy-4-((4-((2-methyl-3-oxoisoindol-4-yl)oxy)-5-(trifluoromethyl)pyrimidin-2-yl)amino)phenyl)-8-methyl-2,8-diazaspiro[4.5]decan-1-one (Compound 67)

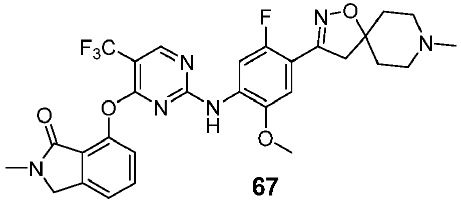

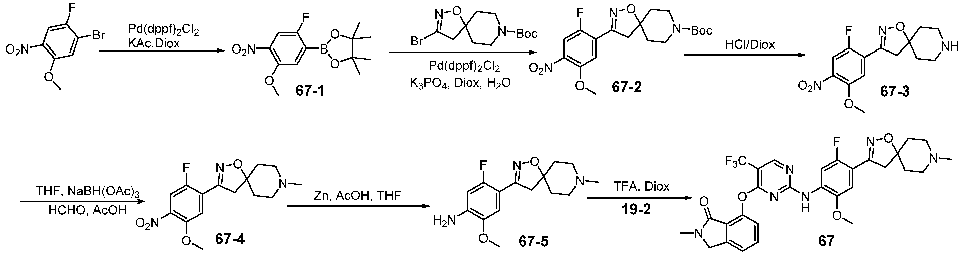

### Synthesis of 67-1:

The compound **1-bromo-2-fluoro-5-methoxy-4-nitrobenzene** described above (1.0 g, 4.016 mmol), bis(pinacolato)diboron (2.04 g, 8.032 mmol) and potassium acetate (1.41 g, 16 mmol), were added to Diox (20 mL). After the mixture was purged with argon, Pd(dppf)₂Cl₂ (293 mg, 0.4 mmol) was added. The mixed solution was heated to 100 °C and stirred for 20 h under argon atmosphere. After the completion of the reaction as detected by LC-MS, the mixed solution was purified by column chromatography to give a product (620 mg, yield: 52%).

ESI-MS m/z: 298.1 [M+H]⁺.

### Synthesis of 67-2:

Compound **67-1** described above (280 mg, 0.943 mmol), ***tert*-butyl 3-bromo-1-oxa-2,8-diazaspiro[4.5]dec-2-ene-8-carboxylate** (200 mg, 0.629 mmol) and potassium phosphate (400 mg, 1.887 mmol) were added to Diox (10 mL)/water (2 mL). After the mixture was purged with argon, Pd(dppf)₂Cl₂ (73 mg, 0.1 mmol) was added. The mixed solution was heated to 100 °C and stirred for 5 h under argon atmosphere. After the completion of the reaction as detected by LC-MS, the mixed solution was concentrated, and the residue was purified by Flash to give a product (122 mg, yield: 47.4%).

ESI-MS m/z: 410.1 [M+H]⁺.

### Synthesis of 67-3:

**67-2** described above (122 mg, 0.298 mmol) was added to EA (5 mL). After complete dissolution, 4 M HCl/Diox (2 mL, 8 mmol) was added. The mixture was stirred at room temperature for 2 h. After the completion of the reaction as detected by LC-MS, the mixture was concentrated to dryness. EA (20 mL) and saturated sodium bicarbonate solution (10 mL) were added to the residue. The resulting mixture was stirred, and liquid separation was performed. The aqueous phase was extracted twice with EA (20 mL × 2). The organic phases were combined, washed with saturated sodium chloride solution, dried over anhydrous sodium sulfate and filtered. The filtrate was concentrated to dryness to give a brownish-yellow oil (95 mg, yield: > 100%).

ESI-MS m/z: 310.1 [M+H]⁺.

### Synthesis of 67-4:

Compound **67-3** described above (95 mg, 0.298 mmol) was added to THF (10 mL), and glacial acetic acid (30 mg, 0.5 mmol) and an aqueous formaldehyde solution (122 mg, 37%, 1.5 mmol) were added at room temperature. The mixed solution was stirred at room temperature for 30 min. NaBH(OAc)₃ (318 mg, 1.5 mmol) was then added, and the mixed solution was stirred at room temperature for another 2 h. After the completion of the reaction as detected by LC-MS, EA (20 mL) and saturated sodium bicarbonate solution (10 mL) were added to the mixed solution. The resulting mixture was stirred for 20 min, and liquid separation was then performed. The aqueous phase was extracted twice with EA (20 mL × 2). The organic phases were combined, washed with saturated sodium chloride solution, dried over anhydrous sodium sulfate and filtered. The filtrate was concentrated to dryness to give a crude product as a brown oil (110 mg, yield: > 100%).

ESI-MS m/z: 324.1 [M+H]⁺.

### Synthesis of 67-5:

Compound **67-4** described above (110 mg, 0.298 mmol) and zinc powder (192 mg, 3.0 mmol) were added to THF (10 mL), and a solution of glacial acetic acid (180 mg, 3 mmol) in THF (3 mL) was added dropwise at room temperature. After the dropwise addition was completed, the mixture was stirred at room temperature for 5 h. After the completion of the reaction as detected by LC-MS, the mixed solution was filtered, and the filtrate was concentrated. The residue was purified by Flash and lyophilized to give a pale brown solid product (47 mg, yield: 53.8%).

ESI-MS m/z: 294.1 [M+H]⁺.

### Synthesis of compound 67:

Compounds **67-5** (47 mg, 0.160 mmol) and **19-2** (50 mg, 0.129 mmol) described above were added to Diox (5 mL), and TFA (18.6 mg, 0.163 mmol) was added at room temperature. The mixed solution was purged with argon, heated to 80 °C and stirred for 3 h. After the basic completion of the reaction as detected by LC-MS, the mixture was concentrated, and the residue was purified by Pre-TLC (DCM:MeOH = 20: 1) to give an off-white solid product (13 mg, yield: 16.8%).

ESI-MS m/z: 601.2 [M+H]⁺.

### Examples 68-69. Synthesis of compounds 68-69

The target compounds 68-69 in Table 5 were obtained with reference to a similar synthetic method to that in Example 67 using different starting materials.

**Table 5**

| **Compound** | **Structural formula** | **MS: [M+H]⁺** | **Compound** | **Structural formula** | **MS: [M+H]⁺** |
|---|---|---|---|---|---|
| **68** | 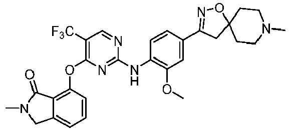 | **583.2** | **69** | 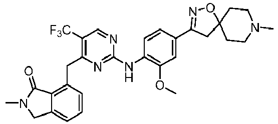 | **581.2** |

### Example 70. Synthesis of 4-((4-((1,2-dimethyl-3-oxoisoindol-4-yl)oxy)-5-(trifluoromethyl)pyrimidin-2-yl)amino)-2-fluoro-5-methoxy-N-(7-methyl-7-azaspiro[3.5]nonan-2-yl)benzamide (Compound 70)

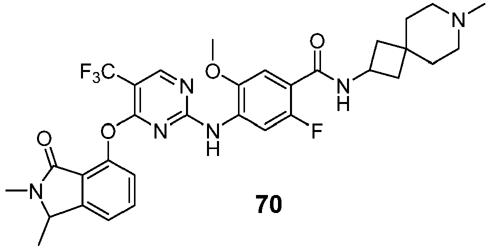

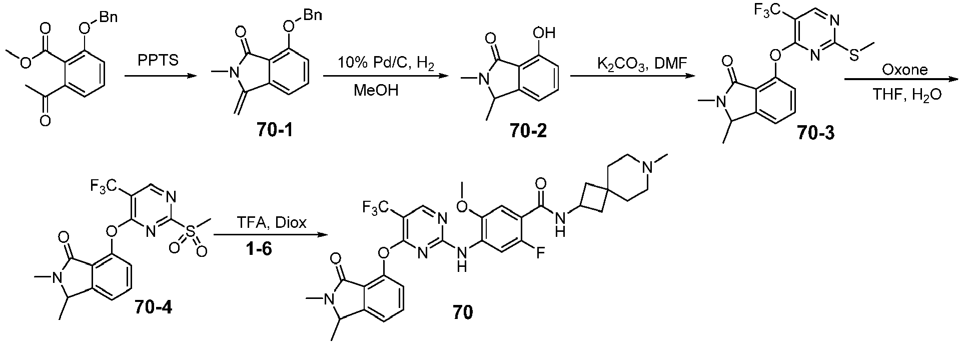

### Synthesis of 70-1:

**Methyl 2-acetyl-6-(benzyloxy)benzoate** (1.0 g, 3.52 mmol), a methylamine/ethanol solution (2 mL) and PPTS (250 mg, 1.0 mmol) were added to toluene (50 mL). The mixed solution was heated to 100 °C and stirred for 20 h under argon atmosphere. After the completion of the reaction as detected by LC-MS, saturated sodium bicarbonate solution (20 mL) was added to the mixed solution. The resulting mixture was stirred, and liquid separation was performed. The aqueous phase was extracted with EA (20 mL). The organic phases were combined, washed with saturated sodium chloride solution, dried over anhydrous sodium sulfate and filtered. The filtrate was concentrated to dryness to give a crude product (1.1 g, yield: > 100%).

ESI-MS m/z: 266.1 [M+H]⁺.

### Synthesis of 70-2:

**70-1** described above (1.1 g, crude, 3.52 mmol) and 10% wet Pd/C (100 mg, containing 50%-55% water) were added to MeOH (20 mL). The mixed solution was purged with hydrogen three times and then vigorously stirred at room temperature under normal pressure for 20 h. After the completion of the reaction as detected by LC-MS, the mixed solution was filtered through celite. The filter cake was rinsed with MeOH, and the filtrate was concentrated. The residue was purified by column chromatography to give a white solid product (442 mg, yield: 71%). ESI-MS m/z: 178.0 [M+H]⁺.

### Synthesis of 70-3:

**Compound 70-2 described above** (400 mg, 2.26 mmol) and anhydrous potassium carbonate (624 mg, 4.52 mmol) were added to DMF (10 mL), and the mixed solution was purged with argon. A solution of **4-chloro-2-(methylthio)-5-(trifluoromethyl)pyrimidine** (518 mg, 2.26 mmol) in DMF (5 mL) was added dropwise at room temperature. After the dropwise addition was completed, the mixed solution was stirred at room temperature for 3 h. After the completion of the reaction as detected by LC-MS, the system was quenched with water (30 mL). The mixed solution was stirred at room temperature for 30 min and then filtered to give an impure product.

This impure product was purified by Flash to give a white solid product (509 mg, yield: 61%).

ESI-MS m/z: 370.1 [M+H]⁺.

### Synthesis of 70-4:

Compound **70-3** described above (455 mg, 1.232 mmol) was added to a mixed solution of THF (10 mL)/water (2 mL), and Oxone (3.78 g, 6.16 mmol) was added. The mixture was stirred at room temperature for 20 h. After the completion of the reaction as detected by LC-MS, water (20 mL) was added to the system, followed by extraction with EA (20 mL). The organic phase was washed twice with saturated sodium chloride solution (20 mL), dried over anhydrous sodium sulfate and filtered, and the filtrate was concentrated to dryness to give a crude product as a light brown solid (480 mg, yield: > 100%).

ESI-MS m/z: 376.0 [M+H]⁺.

### Synthesis of compound 70:

Compounds **70-4** (100 mg, crude) and **1-6** (50 mg, 0.156 mmol) described above were added to Diox (5 mL), and TFA (35.6 mg, 0.312 mmol) was added at room temperature. The mixed solution was purged with argon, heated to 80 °C and stirred for 3 h. After the basic completion of the reaction as detected by LC-MS, the mixture was concentrated, and the residue was purified by Flash and lyophilized to give an off-white solid product (12 mg, yield: 12%).

ESI-MS m/z: 643.2 [M+H]⁺.

### Examples 71-73. Synthesis of compounds 71-73

The target compounds 71-73 in Table 6 were obtained with reference to a similar synthetic method to that in Example 70 using different starting materials.

**Table 6**

| **Compound** | **Structural formula** | **MS: [M+H]⁺** | **Compound** | **Structural formula** | **MS: [M+H]⁺** |
|---|---|---|---|---|---|
| **71** | 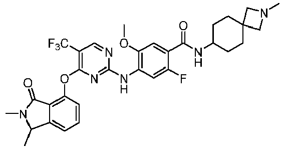 | **643.2** | **72** | 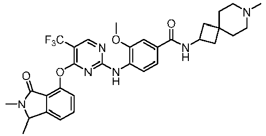 | **625.2** |
| **73** | 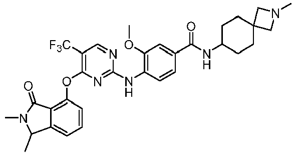 | **625.2** | | | |

### Example 74. Synthesis of 4-((4-((1,2-dimethyl-3-oxoisoindol-4-yl)methyl)-5-(trifluoromethyl)pyrimidin-2-yl)amino)-2-fluoro-5-methoxy-N-(7-methyl-7-azaspiro[3.5]nonan-2-yl)benzamide (Compound 74)

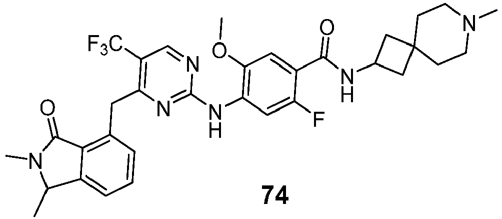

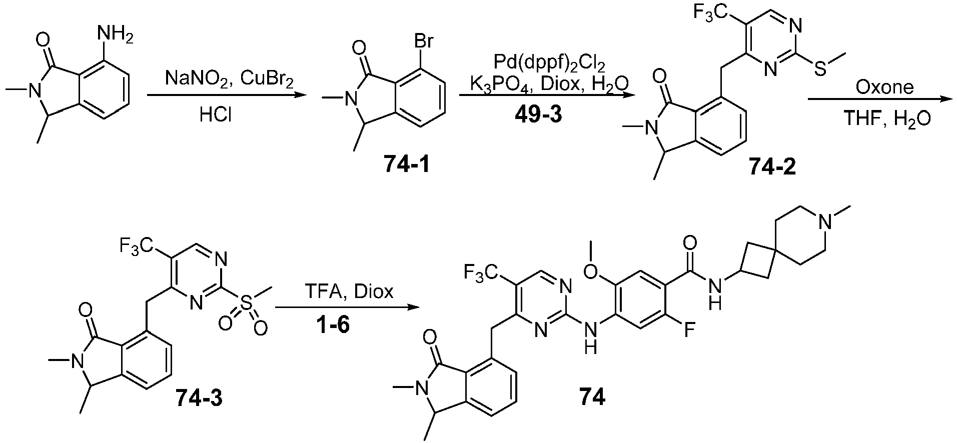

### Synthesis of 74-1:

**7-Amino-2,3-dimethylisoindolin-1-one** (250 mg, 1.42 mmol) was added to THF (10 mL) and 1 N HCl (3 mL), and then an aqueous solution of NaNO₂ (196 mg, 2.84 mmol) was added dropwise at room temperature. After the dropwise addition was completed, the mixture was stirred at room temperature for 10 min, followed by the addition of CuBr₂ (636 mg, 2.84 mmol).

The mixture was heated to reflux and reacted for 8 h. After the completion of the reaction as detected by LC-MS, the mixed solution was concentrated, and the residue was purified by Flash to give a product (162 mg, yield: 47.5%).

ESI-MS m/z: 240.0/242.0 [M+H]⁺.

### Synthesis of 74-2:

Compounds **74-1** (162 mg, 0.675 mmol) and **49-3** (225 mg, 0.675 mmol) described above and potassium phosphate (430 mg, 2.025 mmol) were added to Diox (10 mL)/water (2 mL). After the mixture was purged with argon, Pd(dppf)₂Cl₂ (73 mg, 0.1 mmol) was added. The mixed solution was heated to 100 °C and stirred for 20 h under argon atmosphere. After the completion of the reaction as detected by LC-MS, the mixed solution was concentrated, and the residue was purified by Flash to give a product (86 mg, yield: 34.7%).

ESI-MS m/z: 368.1 [M+H]⁺.

### Synthesis of 74-3:

Compound **74-2** described above (86 mg, 0.234 mmol) was added to a mixed solution of THF (10 mL)/water (2 mL), and Oxone (723 mg, 1.17 mmol) was added. The mixture was stirred at room temperature for 6 h. After the completion of the reaction as detected by LC-MS, water (10 mL) was added to the system, followed by extraction with EA (10 mL). The organic phase was washed twice with saturated sodium chloride solution (10 mL), dried over anhydrous sodium sulfate and filtered, and the filtrate was concentrated to dryness to give a crude product as a light brown solid (110 mg, yield: > 100%).

ESI-MS m/z: 400.1 [M+H]⁺.

### Synthesis of compound 74:

Compounds **74-3** (50 mg, crude) and **1-6** (40 mg, 0.125 mmol) described above were added to Diox (5 mL), and TFA (28 mg, 0.25 mmol) was added at room temperature. The mixed solution was purged with argon, heated to 80 °C and stirred for 3 h. After the basic completion of the reaction as detected by LC-MS, the mixture was concentrated, and the residue was purified by pre-TLC to give an off-white solid product (8 mg, yield: 10%).

ESI-MS m/z: 641.2 [M+H]⁺.

### Examples 75-84: Synthesis of Compounds 75-84

The target compounds 75-84 in Table 7 were obtained with reference to similar synthetic methods to those in Examples 70-74 using different starting materials.

**Table 7**

| **Compound** | **Structural formula** | **MS: [M+H]⁺** | **Compound** | **Structural formula** | **MS: [M+H]⁺** |
|---|---|---|---|---|---|
| **75** | 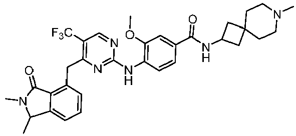 | **623.2** | **76** | 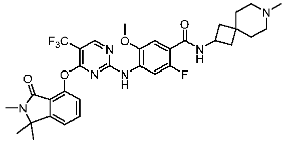 | **657.2** |
| **77** | 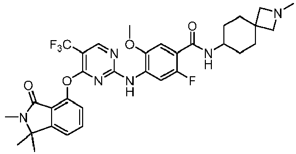 | **657.2** | **78** | 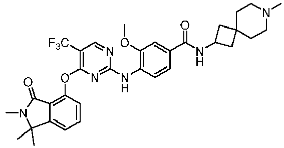 | **639.2** |
| **79** | 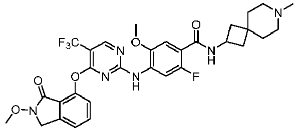 | **645.2** | **80** | 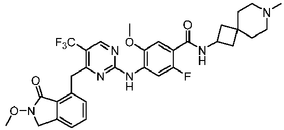 | **643.2** |
| **81** | 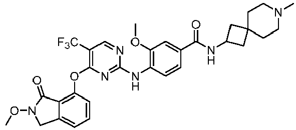 | **627.2** | **82** | 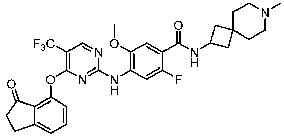 | **614.2** |
| **83** | 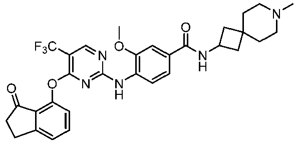 | **596.2** | **84** | 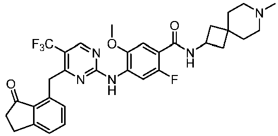 | **612.2** |

### Example 85. Synthesis of 2-fluoro-4-((4-((2-methyl-3-oxoisoindol-4-yl)oxy)-5-(trifluoromethyl)pyrimidin-2-yl)amino)-N-(7-methyl-7-azaspiro[3.5]nonan-2-yl)-5-ethylbenzamide (Compound 85)

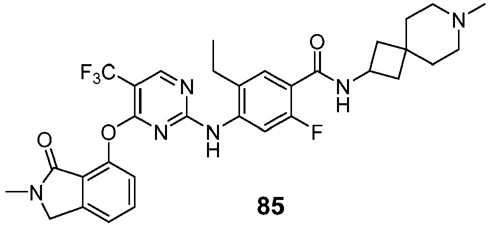

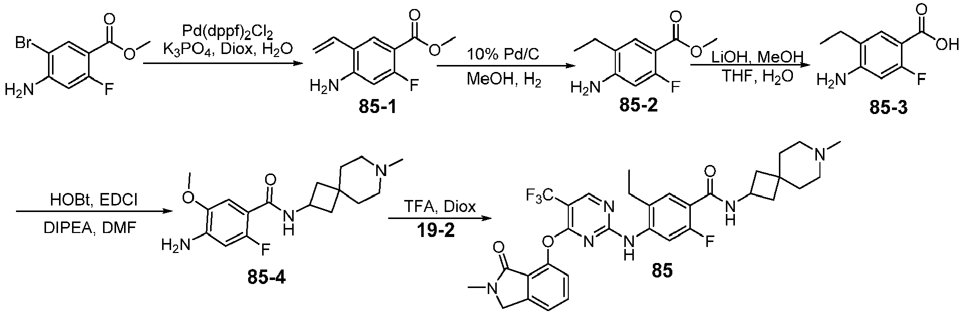

### Synthesis of 85-1:

Methyl 4-amino-5-bromo-2-fluorobenzoate (1.0 g, 4.032 mmol), potassium vinyltrifluoroborate (1.08 g, 8.06 mmol) and potassium phosphate (2.54 g, 12 mmol) were added to Diox (50 mL)/water (10 mL). After the mixture was purged with argon, Pd(dppf)₂Cl₂ (293 mg, 0.4 mmol) was added. The mixed solution was heated to 100 °C and stirred for 20 h under argon atmosphere.

After the completion of the reaction as detected by LC-MS, the mixed solution was concentrated, and the residue was purified by Flash to give a product (512 mg, yield: 65%).

ESI-MS m/z: 196.0 [M+H]⁺.

### Synthesis of 85-2:

**85-1** described above (200 mg, 1.026 mmol) and 10% wet Pd/C (30 mg, containing 50%-55% water) were added to MeOH (10 mL). The mixed solution was purged with hydrogen three times and then vigorously stirred at room temperature under normal pressure for 20 h. After the completion of the reaction as detected by LC-MS, the mixed solution was filtered through celite. The filter cake was rinsed with MeOH, and the filtrate was concentrated to give a white solid product (212 mg, yield: > 100%).

ESI-MS m/z: 198.0 [M+H]⁺.

### Synthesis of 85-3:

Compound **85-2** described above (212 mg, 1.026 mmol) was added to THF (10 mL)/MeOH (5 mL)/water (5 mL), and lithium hydroxide monohydrate (188 mg, 4.465 mmol) was added at room temperature. The mixed solution was stirred at room temperature for 6 h. After the completion of the reaction as detected by LC-MS, the mixed solution was concentrated to a small amount, and water (10 mL) was then added. The pH was adjusted to 2-3 with 2 N HCl aq, and the resulting mixture was extracted three times with EA (20 mL × 3). The organic phases were combined, washed with saturated sodium chloride solution (20 mL × 2), dried over anhydrous sodium sulfate and filtered, and the filtrate was concentrated to dryness to give an off-white solid product (135 mg, yield: 72%).

ESI-MS m/z: 184.0 [M+H]⁺.

### Synthesis of 85-4:

Compound **85-3** described above (65 mg, 0.355 mmol), **7-methyl-7-azaspiro[3.5]nonan-2-amine** (55 mg, 0.355 mol), HOBt (68 mg, 0.5 mmol), EDCI (97 mg, 0.5 mg) and DIPEA (129 mg, 1.0 mmol) were added to DMF (10 mL). The mixture was purged with argon and then stirred at room temperature for 20 h. After the completion of the reaction as detected by LC-MS, the mixed solution was purified by Flash to give a white solid product (55 mg, yield: 48.2%).

ESI-MS m/z: 322.1 [M+H]⁺.

### Synthesis of compound 85:

Compounds **85-4** (55 mg, 0.171 mmol) and **19-2** (50 mg, 0.129 mmol) described above were added to Diox (5 mL), and TFA (28 mg, 0.25 mmol) was added at room temperature. The mixed solution was purged with argon, heated to 80 °C and stirred for 3 h. After the basic completion of the reaction as detected by LC-MS, the mixture was concentrated, and the residue was purified by pre-TLC to give an off-white solid product (16 mg, yield: 19.8%).

ESI-MS m/z: 627.2 [M+H]⁺.

### Examples 86-88. Synthesis of compounds 86-88

The target compounds 86-88 in Table 8 were obtained with reference to a similar synthetic method to that in Example 85 using different starting materials.

**Table 8**

| **Compound** | **Structural formula** | **MS: [M+H]⁺** | **Compound** | **Structural formula** | **MS: [M+H]⁺** |
|---|---|---|---|---|---|
| **86** | 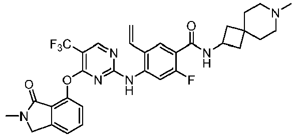 | **625.2** | **87** | 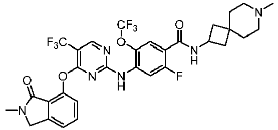 | **683.2** |
| **88** | 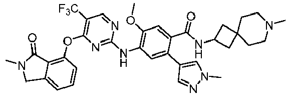 | **691.2** | | | |

### Example 89. Synthesis of 7-((2-((5-fluoro-2-methoxy-4-(7-methyl-2,7-diazaspiro[3.5]non-2-yl)phenyl)amino)-5-(trifluoromethyl)pyrimidin-4-yl)oxy)-2-methylisoindolin-1-one (Compound 89)

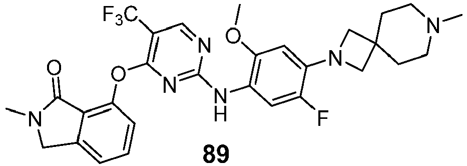

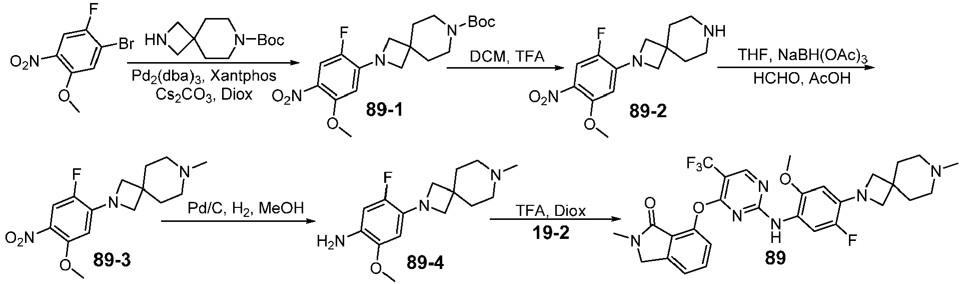

### Synthesis of 89-1:

The compound **1-bromo-2-fluoro-5-methoxy-4-nitrobenzene** described above (770 mg, 3.079 mmol), tert-butyl 2,7-diazaspiro[3.5]nonane-7-carboxylate (697 mg, 3.079 mmol), Xantphos (178 mg, 0.308 mmol) and cesium carbonate (2.0 g, 6.158 mmol) were added to Diox (10 mL).

After the mixture was purged with argon, Pd₂(dba)₃ (141 mg, 0.154 mmol) was added. The mixed solution was heated to 100 °C and stirred for 20 h under argon atmosphere. After the completion of the reaction as detected by LC-MS, the mixed solution was filtered. The filter cake was washed with EA (40 mL), and water (20 mL) was added to the filtrate. The resulting mixture was stirred, and liquid separation was performed. The aqueous phase was extracted with EA (20 mL). The organic phases were combined, washed with saturated sodium chloride solution and concentrated. The residue was purified by column chromatography to give a product (890 mg, yield: 73.1%).

ESI-MS m/z: 396.2 [M+H]⁺.

### Synthesis of 89-2:

Compound **89-1** described above (450 mg, 1.138 mmol) and TFA (1 mL) were added to DCM (2 mL). The mixed solution was purged with argon and stirred at room temperature for 2 h. After the completion of the reaction as detected by LC-MS, the mixed solution was concentrated to give a crude product (737 mg, yield: > 100%).

ESI-MS m/z: 296.1 [M+H]⁺.

### Synthesis of 89-3:

Compound **89-2** described above (737 mg, crude, 1.138 mmol) and DIPEA (455 mg, 3.527 mmol) were added to THF (10 mL)/MeOH (2 mL). The mixed solution was stirred at room temperature for 10 min. Glacial acetic acid (137 mg, 2.276 mmol) and an aqueous formaldehyde solution (111 mg, 37% in water, 1.366 mmol) were then added, and the mixed solution was stirred at room temperature for 20 min. NaBH(OAc)₃ (482 mg, 2.276 mmol) was then added, and the mixed solution was stirred at room temperature for another 1 h. After the completion of the reaction as detected by LC-MS, the mixed solution was concentrated, and the residue was purified by Flash to give a yellow solid product (335 mg, yield: 95%).

ESI-MS m/z: 310.1 [M+H]⁺.

### Synthesis of 89-4:

Compound **89-3** described above (335 mg, 1.083 mmol) and 10% Pd/C (34 mg) were added to MeOH (10 mL). The mixed solution was purged with H₂ 3 times and then stirred at room temperature under normal pressure for 2 h. After the completion of the reaction as detected by LC-MS, the mixed solution was filtered, and the filtrate was concentrated to dryness to give a dark purple oily product (303 mg, yield: 100%).

ESI-MS m/z: 280.1 [M+H]⁺.

### Synthesis of compound 89:

Compounds **89-4** (270 mg, 0.966 mmol) and **19-2** (374 mg, 0.966 mmol) described above were added to Diox (10 mL), and TFA (110 mg, 0.966 mmol) was added at room temperature. The mixed solution was purged with argon, heated to 90 °C and stirred for 3 h. After the basic completion of the reaction as detected by LC-MS, the mixture was concentrated, and the residue was purified by Flash and lyophilized to give an off-white solid product (195 mg, yield: 34.4%).

ESI-MS m/z: 587.2 [M+H]⁺.

### Example 90. Synthesis of 7-((2-((2-methoxy-4-(7-methyl-2,7-diazaspiro[3.5]non-2-yl)phenyl)amino)-5-(trifluoromethyl)pyrimidin-4-yl)oxy)-2-methylisoindolin-1-one (Compound 90)

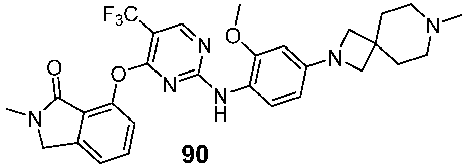

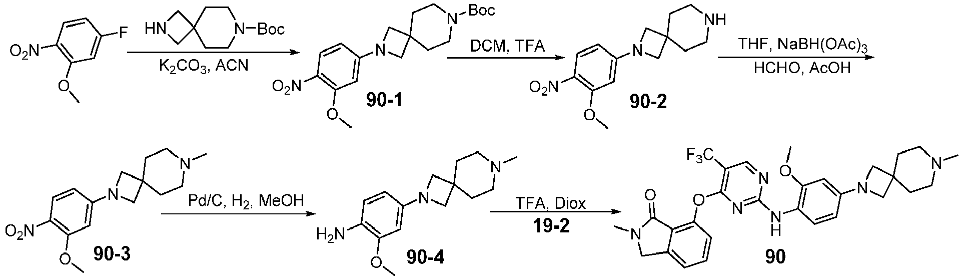

### Synthesis of 90-1:

The compound **4-fluoro-2-methoxy-1-nitrobenzene** described above (794 mg, 4.64 mmol), *tert*-butyl 2,7-diazaspiro[3.5]nonane-7-carboxylate (1 g, 4.42 mmol) and anhydrous potassium carbonate (1.3 g, 9.28 mmol) were added to acetonitrile (10 mL). The mixed solution was purged with argon, heated to 85 °C and stirred for 40 h. After the basic completion of the reaction as detected by LC-MS, the mixed solution was concentrated. EA (20 mL) and water (20 mL) were added to the residue. The resulting mixture was stirred, and liquid separation was performed.

The aqueous phase was extracted with EA (20 mL). The organic phases were combined, washed with saturated sodium chloride solution and concentrated. The residue was purified by column chromatography to give a product (1.5 g, yield: 90%).

ESI-MS m/z: 378.2 [M+H]⁺.

### Synthesis of 90-2:

Compound **90-1** described above (680 mg, 1.8 mmol) and TFA (2 mL) were added to DCM (4 mL). The mixed solution was purged with argon and stirred at room temperature for 2 h. After the completion of the reaction as detected by LC-MS, the mixed solution was concentrated. EA (5 mL) and PE (5 mL) were added to the residue. The resulting mixture was stirred at room temperature for 30 min and then filtered. The filter cake was dried under vacuum to give a yellow solid product (730 mg, yield: > 100%).

ESI-MS m/z: 278.1 [M+H]⁺.

### Synthesis of 90-3:

Compound **90-2** described above (730 mg, 1.8 mmol) and DIPEA (233 mg, 1.8 mmol) were added to THF (20 mL)/MeOH (4 mL). The mixed solution was stirred at room temperature for 10 min. Glacial acetic acid (216 mg, 3.6 mmol) and an aqueous formaldehyde solution (175 mg, 37% in water, 2.16 mmol) were then added, and the mixed solution was stirred at room temperature for 20 min. NaBH(OAc)₃ (763 mg, 3.6 mmol) was then added, and the mixed solution was stirred at room temperature for another 1 h. After the completion of the reaction as detected by LC-MS, the mixed solution was concentrated, and the residue was purified by Flash to give a yellow foamy solid product (516 mg, yield: 98%).

ESI-MS m/z: 292.1 [M+H]⁺.

### Synthesis of 90-4:

Compound **90-3** described above (512 mg, 1.757 mmol) and 10% Pd/C (50 mg) were added to MeOH (10 mL). The mixed solution was purged with H₂ 3 times and then stirred at room temperature under normal pressure for 20 h. After the completion of the reaction as detected by LC-MS, the mixed solution was filtered, and the filtrate was concentrated to dryness to give a dark purple oily product (460 mg, yield: 100%).

ESI-MS m/z: 262.1 [M+H]⁺.

### Synthesis of compound 90:

Compounds **90-4** (220 mg, 0.843 mmol) and **19-2** (327 mg, 0.843 mmol) described above were added to Diox (10 mL), and TFA (96 mg, 0.843 mmol) was added at room temperature. The mixed solution was purged with argon, heated to 90 °C and stirred for 3 h. After the basic completion of the reaction as detected by LC-MS, the mixture was concentrated, and the residue was purified by Flash and lyophilized to give an off-white impure product (210 mg). The impure product was then purified by pre-TLC (DCM:MeOH = 10:1) to give an off-white solid product (127 mg, yield: 26.5%).

ESI-MS m/z: 569.2 [M+H]⁺.

### Examples 91-106. Synthesis of compounds 91-106

The target compounds **91-106** in Table 9 were obtained with reference to similar synthetic methods to those in Example 89 and Example 90 using different starting materials.

**Table 9**

| **Compound** | **Structural formula** | **MS: [M+H]⁺** | **Compound** | **Structural formula** | **MS: [M+H]⁺** |
|---|---|---|---|---|---|
| **91** | 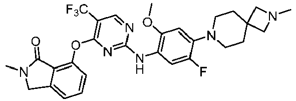 | **587.2** | **92** | 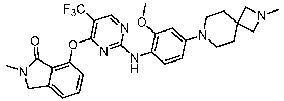 | **569.2** |
| **93** | 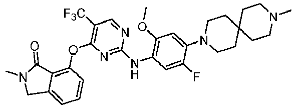 | **615.2** | **94** | 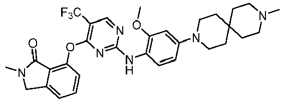 | **597.2** |
| **95** | 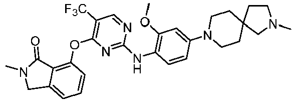 | **583.2** | **96** | 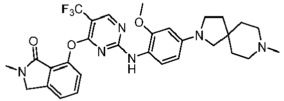 | **583.2** |
| **97** | 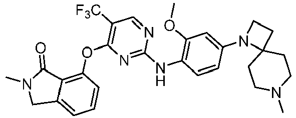 | **569.2** | **98** | 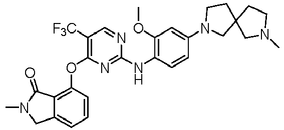 | **569.2** |
| **99** | 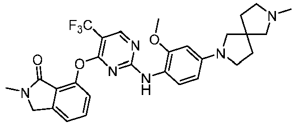 | **569.2** | **100** | 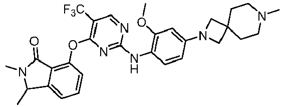 | **583.2** |
| **101** | 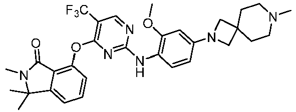 | **597.2** | **102** | 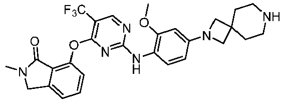 | **555.2** |
| **103** | 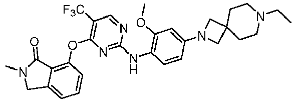 | **583.2** | **104** | 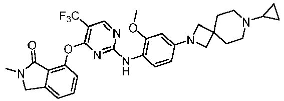 | **595.2** |
| **105** | 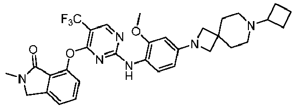 | **609.2** | **106** | 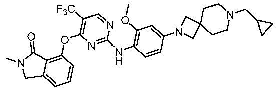 | **609.2** |

### Example 107. Synthesis of 7-((2-((5-fluoro-2-methoxy-4-(2,7-diazaspiro[3.5]non-2-yl)phenyl)amino)-5-(trifluoromethyl)pyrimidin-4-yl)oxy)-2-methylisoindolin-1-one

### (Compound 107)

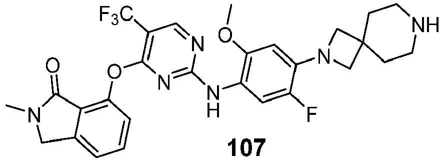

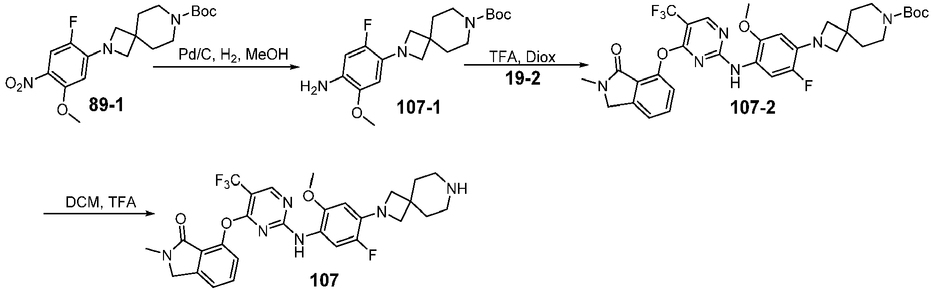

### Synthesis of 107-1:

Compound **89-1** described above (150 mg, 0.38 mmol) and 10% Pd/C (30 mg) were added to MeOH (10 mL). The mixed solution was purged with H₂ 3 times and then stirred at room temperature under normal pressure for 2 h. After the completion of the reaction as detected by LC-MS, the mixed solution was filtered, and the filtrate was concentrated to dryness to give a purple oily product (142 mg, yield: 100%).

### Synthesis of 107-2:

Compounds **107-1** (142 mg, 0.38 mmol) and **19-2** (147 mg, 0.38 mmol) described above were added to Diox (5 mL), and TFA (43 mg, 0.38 mmol) was added at room temperature. The mixed solution was purged with argon, heated to 90 °C and stirred for 3 h. After the basic completion of the reaction as detected by LC-MS, the mixture was concentrated to give a brown-black solid product, and the crude product was used in the next step.

ESI-MS m/z: 673.2 [M+H]⁺.

### Synthesis of compound 107:

Compound **107-2** described above (450 mg, 0.38 mmol) and TFA (2 mL) were added to DCM (5 mL). The mixed solution was purged with argon and stirred at room temperature for 2 h. After the completion of the reaction as detected by LC-MS, the mixed solution was concentrated, and the residue was purified by Flash and lyophilized to give an off-white solid product (78 mg, yield: 35.8%).

ESI-MS m/z: 573.2 [M+H]⁺.

### Example 108. Synthesis of 7-((5-chloro-2-((5-fluoro-2-methoxy-4-(7-(methyl-d3)-2,7-diazaspiro [3.5] non-2-yl)phenyl)amino)pyrimidin-4-yl)oxy)-2-methylisoindolin-1-one

### (Compound 108)

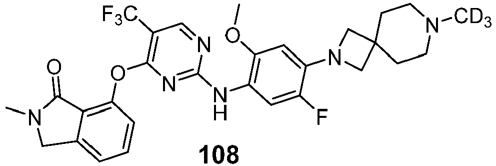

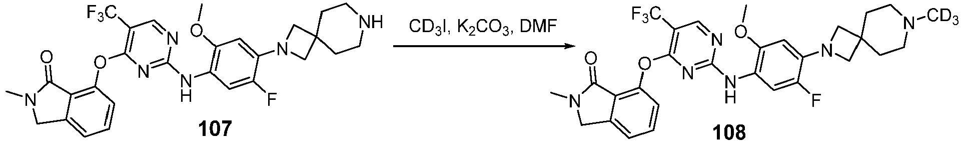

### Synthesis of compound 108:

Compound **107** described above (65 mg, 0.114 mmol) and **K₂CO₃** (47 mg, 0.34 mmol) were added to DMF (3 mL), and CD₃I (18 mg, 0.125 mmol) was added at room temperature. The mixed solution was stirred at room temperature for 3 h. After the basic completion of the reaction as detected by LC-MS, the mixture was directly loaded, purified by Flash and lyophilized to give an off-white solid product (23 mg, yield: 34%).

ESI-MS m/z: 590.2 [M+H]⁺.

### Example 109. Synthesis of 7-((2-((2-methoxy-4-(7-(methyl-d3)-2,7-diazaspiro[3.5]non-2-yl)phenyl)amino)-5-(trifluoromethyl)pyrimidin-4-yl)oxy)-2-methylisoindolin-1-one

### (Compound 109)

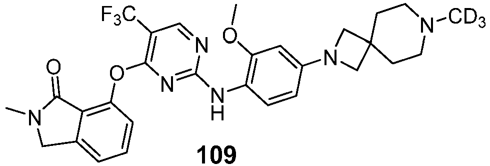

The target compound **109** was obtained with reference to similar synthetic methods to those in Examples **107** and **108** using different starting materials.

ESI-MS m/z: 572.3 [M+H]⁺.

### Example 110. Synthesis of 7-((5-chloro-2-((5-fluoro-2-methoxy-4-(7-methyl-2,7-diazaspiro [3.5] non-2-yl)phenyl)amino)pyrimidin-4-yl)oxy)-2-methylisoindolin-1-one

### (Compound 110)

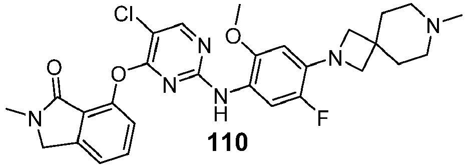

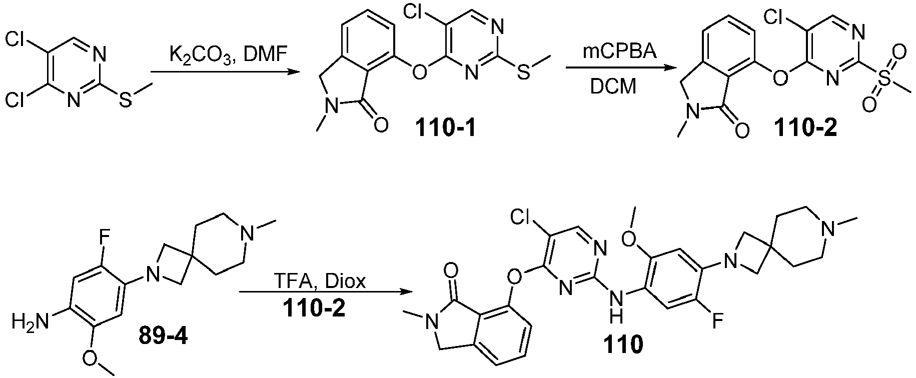

### Synthesis of 110-1:

**7-Hydroxy-2-methylisoindolin-1-one** (0.85 g, 5.22 mmol) and anhydrous potassium carbonate (1.44 g, 10.44 mmol) were added to DMF (10 mL), and the mixed solution was purged with argon. A solution of **4,5-dichloro-2-(methylthio)pyrimidine** (1.02 g, 5.22 mmol) in DMF (5 mL) was added dropwise at room temperature. After the dropwise addition was completed, the mixed solution was stirred at room temperature for 20 h. After the completion of the reaction as detected by LC-MS, the system was quenched with water (30 mL). The mixed solution was stirred at room temperature for 30 min and then filtered. The filter cake was rinsed with water and dried under vacuum to give an off-white solid product (1.42 g, yield: 84.5%).

ESI-MS m/z: 322.0 [M+H]⁺.

### Synthesis of 110-2:

Compound **110-1** (1.40 g, 4.35 mmol) was added to DCM (30 mL), and mCPBA (3.5 g, 17.4 mmol) was added in batches in an ice bath. The mixed solution was stirred at room temperature for 20 h. After the completion of the reaction as detected by LC-MS, the mixed solution was quenched with saturated sodium thiosulfate (30 mL) and saturated sodium bicarbonate solution (30 mL). The resulting mixture was stirred at room temperature for 15 min, and liquid separation was then performed. The aqueous phase was extracted with DCM (50 mL). The organic phases were combined, washed twice with saturated sodium chloride solution (50 mL × 2), dried over anhydrous sodium sulfate and filtered, and the filtrate was concentrated to dryness to give a crude product as a white solid (1.56 g, yield: > 100%).

ESI-MS m/z: 354.0 [M+H]⁺.

### Synthesis of compound 110:

Compounds **110-2** (100 mg, 0.283 mmol) and **89-4** (79 mg, 0.283 mmol) described above were added to Diox (5 mL), and TFA (97 mg, 0.85 mmol) was added at room temperature. The mixed solution was purged with argon, heated to 90 °C and stirred for 20 h. A small amount of starting material remained as detected by LC-MS. The mixture was concentrated, and the residue was purified by Flash and lyophilized to give an off-white solid product (32 mg, yield: 20.4%).

ESI-MS m/z: 553.2 [M+H]⁺.

### Examples 111-117. Synthesis of compounds 111-117

The target compounds **111-117** in Table 10 were obtained with reference to a similar synthetic method to that in Example 110 using different starting materials.

**Table 10**

| **Compound** | **Structural formula** | **MS: [M+H]⁺** | **Compound** | **Structural formula** | **MS: [M+H]⁺** |
|---|---|---|---|---|---|
| **111** | 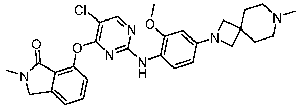 | **535.2** | **112** | 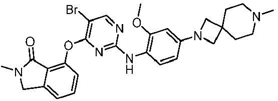 | **579.1** |
| **113** | 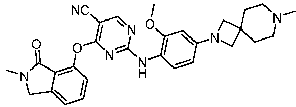 | **526.2** | **114** | 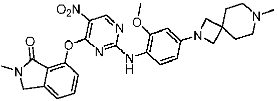 | **546.2** |
| **115** | 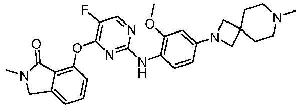 | **519.2** | **116** | 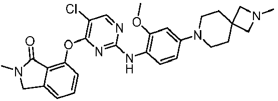 | **535.2** |
| **117** | 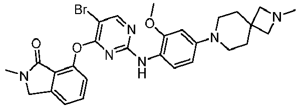 | **579.1** | | | |

**The nuclear magnetic hydrogen spectra of some of the compounds of the present invention are shown in Table 11 below:**

**Table 11**

| **Compound** | **NMR (H spectrum)** |
|---|---|
| **1** | ¹H NMR (400 MHz, CDCl₃) δ 8.60 (s, 1H), 8.31 (s, 1H), 8.02 (s, 1H), 7.68 (t, *J* = 7.8 Hz, 1H), 7.44 (dd, *J* = 15.8, 7.3 Hz, 2H), 7.26 (m, 2H), 6.72 (dd, *J* = 14.2, 7.0 Hz, 1H), 4.47 (q, *J* = 7.9 Hz, 1H), 3.85 (s, 3H), 2.73 (s, 3H), 2.41 - 2.29 (m, 4H), 2.25 (s, 3H), 1.78 - 1.60 (m, 8H) |
| **19** | ¹H NMR (400 MHz, CDCl₃) δ 8.60 (s, 1H), 8.01 (s, 1H), 7.68 (t, *J* = 7.8 Hz, 1H), 7.47 (dd, *J* = 15.8, 7.3 Hz, 2H), 7.26 (d, *J* = 5.9 Hz, 1H), 6.70 (dd, *J* = 14.2, 7.0 Hz, 1H), 4.50 (q, *J* = 7.9 Hz, 1H), 4.43 (s, 2H), 3.87 (s, 3H), 3.06 (s, 3H), 2.43 - 2.28 (m, 4H), 2.26 (s, 3H), 1.78 - 1.57 (m, 8H) |
| **20** | ¹H NMR (400 MHz, CDCl₃) δ 8.59 (s, 1H), 8.00 (s, 1H), 7.67 (t, *J* = 7.8 Hz, 1H), 7.46 (dd, *J* = 11.9, 7.3 Hz, 2H), 7.24 (m, 2H), 6.43 (dd, *J* = 14.1, 7.5 Hz, 1H), 4.42 (s, 2H), 3.87 (s, 4H), 3.22 - 3.07 (m, 4H), 3.05 (s, 3H), 2.42 (s, 3H), 1.96 (d, *J* = 11.2 Hz, 4H), 1.60 (t, *J* = 11.8 Hz, 2H), 1.27 (d, *J* = 13.0 Hz, 2H) |
| **49** | ¹H NMR (400 MHz, CDCl₃) δ 8.58 (s, 1H), 8.21 (s, 1H), 7.67 (t, *J* = 7.8 Hz, 1H), 7.45 (dd, *J* = 15.8, 7.3 Hz, 2H), 7.26 (m, 2H), 6.73 (dd, *J* = 14.2, 7.0 Hz, 1H), 4.50 (q, *J* = 7.9 Hz, 1H), 4.43 (s, 2H), 4.03 (s, 2H), 3.87 (s, 3H), 3.05 (s, 3H), 2.41 - 2.30 (m, 4H), 2.24 (s, 3H), 1.78 - 1.61 (m, 8H) |
| **70** | ¹H NMR (400 MHz, CDCl₃) δ 8.62 (s, 1H), 8.01 (s, 1H), 7.66 (t, *J* = 7.8 Hz, 1H), 7.46 (dd, *J* = 15.8, 7.3 Hz, 2H), 7.26 (d, *J* = 5.9 Hz, 1H), 6.71 (dd, *J* = 14.2, 7.0 Hz, 1H), 4.86 (m, 1H) , 4.50 (q, *J* = 7.9 Hz, 1H), 3.87 (s, 3H), 3.11 (s, 3H), 2.43 - 2.28 (m, 4H), 2.25 (s, 3H), 1.78 - 1.61 (m, 8H), 1.51 (d, *J* = 4.9 Hz, 3H) |
| 89 | ¹H NMR (400 MHz, Chloroform-*d*) δ 8.47 (s, 1H), 7.61 (t, *J* = 7.9 Hz, 2H), 7.41 (d, *J* = 7.6 Hz, 1H), 7.20 (d, *J* = 8.0 Hz, 1H), 6.92 (s, 1H), 5.90 (d, *J* = 8.2 Hz, 1H), 4.40 (s, 2H), 3.76 (s, 3H), 3.61 (d, *J* = 2.1 Hz, 4H), 3.05 (s, 3H), 2.35 (m, 4H), 2.27 (s, 3H), 1.84 (m, 4H). |
| **90** | ¹H NMR (400 MHz, DMSO-*d*₆) δ 8.57 (s, 1H), 8.47 (s, 1H), 7.60 (d, *J* = 8.8 Hz, 1H), 7.47 (d, *J* = 7.6 Hz, 1H), 7.19 (s, 1H), 6.70 (s, 1H), 5.91 (s, 1H), 4.43 (s, 2H), 3.61 (s, 3H), 3.51 (s, 4H), 2.95 (s, 3H), 2.63 (m, 4H), 2.39 (s, 3H), 1.81 (m, 4H) |
| **92** | ¹H NMR (400 MHz, CDCl₃) δ 8.49 (s, 1H), 7.64 (t, *J* = 7.8 Hz, 1H), 7.45 (d, *J* = 7.5 Hz, 1H), 7.26 - 7.20 (m, 3H), 6.39 (d, *J* = 2.6 Hz, 1H), 5.97 (s, 1H), 4.39 (s, 2H), 3.84 (m, 2H), 3.79 (s, 3H), 3.48 (m, 2H), 3.05 (s, 3H), 2.97 (m, 4H), 2.86 (s, 3H), 2.09 (m, 4H) |
| **108** | ¹H NMR (400 MHz, Chloroform-*d*) δ 8.47 (s, 1H), 7.61 (t, *J* = 7.9 Hz, 2H), 7.41 (d, *J* = 7.6 Hz, 1H), 7.20 (d, *J* = 8.0 Hz, 1H), 6.92 (s, 1H), 5.90 (d, *J* = 8.2 Hz, 1H), 4.40 (s, 2H), 3.76 (s, 3H), 3.61 (d, *J* = 2.1 Hz, 4H), 3.05 (s, 3H), 2.35 (m, 4H), 1.84 (m, 4H). |
| **109** | ¹H NMR (400 MHz, DMSO-*d*₆) δ 8.57 (s, 1H), 8.47 (s, 1H), 7.60 (d, *J* = 8.8 Hz, 1H), 7.47 (d, *J* = 7.6 Hz, 1H), 7.19 (s, 1H), 6.70 (s, 1H), 5.91 (s, 1H), 4.43 (s, 2H), 3.61 (s, 3H), 3.51 (s, 4H), 2.95 (s, 3H), 2.63 (m, 4H), 1.81 (m, 4H) |
| **111** | 1H NMR (400 MHz, Chloroform-d) δ 8.23 (s, 1H), 7.61 (t, J = 7.8 Hz, 1H), 7.39 (d, J = 7.5 Hz, 1H), 7.32 (s, 1H), 7.23 (d, J = 6.9 Hz, 2H), 5.92 (d, J = 2.4 Hz, 1H), 5.60 (d, J = 8.7 Hz, 1H), 4.37 (s, 2H), 3.76 (s, 3H), 3.52 (s, 4H), 3.05 (s, 3H), 2.37 (s, 4H), 2.28 (s, 3H), 1.84 (t, J = 5.5 Hz, 4H). |
| **113** | ¹H NMR (400 MHz, Chloroform-*d*) δ 8.49 (s, 1H), 7.79 (s, 1H), 7.62 (t, *J* = 7.9 Hz, 1H), 7.41 (d, *J* = 7.5 Hz, 1H), 7.22 (d, *J* = 8.1 Hz, 1H), 7.04 (s, 1H), 5.90 (s, 1H), 4.38 (s, 2H), 3.77 (s, 3H), 3.53 (s, 4H), 3.05 (s, 3H), 2.41 (s, 4H), 2.31 (s, 3H), 1.86 (t, *J* = 5.6 Hz, 4H). |

### Example 118. Assay for Inhibitory Activity of Compounds of the Present Invention Against FAK Enzyme

The compound serially diluted with DMSO and a FAK recombinant protein were mixed, and the mixture was left to stand at room temperature for 10 min. A biotin-labeled TK substrate (TK) and ATP were then added. After the reaction was performed at room temperature for 40 min, Sa-XL 665 and a Crytate-labeled TK antibody were added. After the mixture was incubated at room temperature for 1 h, the fluorescence intensity at 615 nm and 665 nm was measured. The ratio of the fluorescence intensity at 665 nm and 615 nm was calculated. The inhibition percentages and IC₅₀ of the compounds were calculated compared to the DMSO control group. The results are shown in Table 12 below.

**Table 12. Inhibitory activity of the compounds of the present invention against FAK enzyme (IC₅₀, nM)**

| **Compound** | **IC₅₀** | **Compound** | **IC₅₀** | **Compound** | **IC₅₀** | **Compound** | **IC₅₀** | **Compound** | **IC₅₀** |
|---|---|---|---|---|---|---|---|---|---|
| **1** | A | **25** | A | **49** | A | **73** | A | **97** | A |
| **2** | A | **26** | A | **50** | A | **74** | A | **98** | A |
| **3** | A | **27** | A | **51** | B | **75** | A | **99** | A |
| **4** | A | **28** | A | **52** | A | **76** | A | **100** | A |
| **5** | A | **29** | A | **53** | A | **77** | A | **101** | A |
| **6** | A | **30** | A | **54** | B | **78** | A | **102** | A |
| **7** | A | **31** | A | **55** | A | **79** | A | **103** | A |
| **8** | A | **32** | A | **56** | A | **80** | A | **104** | A |
| **9** | B | **33** | A | **57** | A | **81** | A | **105** | A |
| **10** | A | **34** | A | **58** | A | **82** | A | **106** | A |
| **11** | A | **35** | C | **59** | A | **83** | A | **107** | A |
| **12** | A | **36** | A | **60** | C | **84** | A | **108** | A |
| **13** | A | **37** | A | **61** | A | **85** | B | **109** | A |
| **14** | A | **38** | B | **62** | A | **86** | A | **110** | A |
| **15** | A | **39** | A | **63** | B | **87** | A | **111** | A |
| **16** | B | **40** | A | **64** | A | **88** | B | **112** | A |
| **17** | A | **41** | B | **65** | C | **89** | A | **113** | A |
| **18** | A | **42** | A | **66** | A | **90** | A | **114** | A |
| **19** | A | **43** | A | **67** | B | **91** | A | **115** | A |
| **20** | A | **44** | A | **68** | B | **92** | A | **116** | A |
| **21** | B | **45** | A | **69** | B | **93** | A | **117** | A |
| **22** | A | **46** | A | **70** | A | **94** | A | **BI853520** | A |
| **23** | A | **47** | A | **71** | A | **95** | A | **Compound A** | A |
| **24** | A | **48** | A | **72** | A | **96** | A | **Compound B** | A |

| | | | | | | | | | |
|---|---|---|---|---|---|---|---|---|---|
| A represents IC₅₀ < 10 nM B represents 10 nM ≤ IC₅₀ ≤ 50 nM C represents IC₅₀ > 50 nM | | | | | | | | | |

### Example 119. Assay for Inhibitory Activity of Compounds of the Present Invention Against PC3 Cell Proliferation

2D activity detection: 1200 PC3 cells were seeded in a 96-well plate. After overnight adherence culture, the compound serially diluted was added. 72 h after addition, Cell Titer-Lumi (Beyotime C0068XL) was added to measure the ATP content in the cells. The growth of the cells was evaluated, and IC₅₀ of the compounds against cell growth was calculated.

3D activity detection: PC3 cells were seeded in a 96-well plate at 1200 cells/well, and 3D culture was performed. 14 days after the compound serially diluted was added, the growth of the cells was determined. The inhibition percentage and IC₅₀ of the compound were calculated compared to the DMSO control group.

The results of the 2D/3D inhibitory activity of the compounds of the present invention against PC3 cells are shown in Table 13 below.

**Table 13. 2D/3D Inhibitory activity of the compounds of the present invention against PC3 cells (IC₅₀, nM**

| **Compound** | **2D IC₅₀** | **3D IC₅₀** | **Compound** | **2D IC₅₀** | **3D IC₅₀** | **Compound** | **2D IC₅₀** | **3D IC₅₀** |
|---|---|---|---|---|---|---|---|---|
| **1** | + | B | **41** | + | B | **81** | + | A |
| **2** | + | B | **42** | + | A | **82** | + | B |
| **3** | + | B | **43** | + | A | **83** | + | B |
| **4** | + | B | **44** | + | B | **84** | + | B |
| **5** | + | B | **45** | + | A | **85** | + | B |
| **6** | + | B | **46** | + | B | **86** | + | B |
| **7** | + | B | **47** | + | A | **87** | + | |
| **8** | + | B | **48** | + | B | **88** | + | B |
| **9** | + | B | **49** | + | A | **89** | >10000 | 0.84 |
| **10** | + | A | **50** | + | B | **90** | >10000 | 0.80(4)* |
| **11** | + | B | **51** | + | B | **91** | + | A |
| **12** | + | A | **52** | + | A | **92** | >10000 | 0.83 |
| **13** | + | B | **53** | + | B | **93** | + | A |
| **14** | + | A | **54** | + | B | **94** | + | A |
| **15** | + | A | **55** | + | A | **95** | + | A |
| **16** | + | B | **56** | + | B | **96** | + | A |
| **17** | + | B | **57** | + | A | **97** | + | A |
| **18** | + | B | **58** | + | B | **98** | + | A |
| **19** | >10000 | 1.88 (3)* | **59** | + | B | **99** | + | A |
| **20** | >10000 | 4.23 | **60** | + | C | **100** | + | 2.22 |
| **21** | >10000 | 1.32 (2)* | **61** | + | B | **101** | + | 4.86 |
| **22** | + | A | **62** | + | A | **102** | + | A |
| **23** | + | 3.71 | **63** | + | B | **103** | + | A |
| **24** | + | B | **64** | + | B | **104** | + | A |
| **25** | + | A | **65** | + | C | **105** | + | A |
| **26** | + | B | **66** | + | C | **106** | + | A |
| **27** | + | B | **67** | + | B | **107** | + | A |
| **28** | + | 3.34(2)* | **68** | + | B | **108** | + | A |
| **29** | + | B | **69** | + | B | **109** | + | A |
| **30** | + | B | **70** | + | A | **110** | + | A |
| **31** | + | B | **71** | + | B | **111** | + | 1.78(2)* |
| **32** | + | B | **72** | + | A | **112** | + | 0.66 |
| **33** | + | B | **73** | + | B | **113** | + | 1.58 |
| **34** | + | 41.3 | **74** | + | B | **114** | + | A |
| **35** | + | C | **75** | + | B | **115** | + | 67 |
| **36** | + | B | **76** | + | B | **116** | + | A |
| **37** | + | B | **77** | + | B | **117** | + | A |
| **38** | + | B | **78** | + | B | **BI853520** | >10000 | 6.49(5)* |
| **39** | + | B | **79** | + | A | **Compound A** | >10000 | 10.45 |
| **40** | + | B | **80** | + | B | **Compound B** | >10000 | 6.85 |

| | | | | | | | | |
|---|---|---|---|---|---|---|---|---|
| A represents IC₅₀ < 5 nM B represents 5 nM ≤ IC₅₀ ≤ 50 nM C represents IC₅₀ > 50 nM + represents IC₅₀ > 2000 nM | | | | | | | | |

In the values marked by *, the bracket represents the number of detections, and the IC₅₀ values are mean values.

As can be seen from the data in the table above, the compounds of the present invention had substantially no inhibitory activity against PC3 2D cells, but had a strong inhibitory activity against PC3 3D cells. Moreover, some of the compounds had a significantly stronger inhibitory activity than that of the control compound BI853520.

### Example 120. Assay for Inhibition of PC3 Cell Adherence by Compounds of the Present Invention

PC3 cells were added at 8000 cells/well. 4 h after the cells were treated with the compound, the number of adherent cells was counted. The inhibition percentages and IC₅₀ of the compounds were calculated compared to the DMSO group. The results are shown in Table 14 below.

**Table 14. Inhibitory activity of the compounds of the present invention against PC-3 cell adherence (IC₅₀, nM)**

| **Compound** | **IC₅₀** | **Compound** | **IC₅₀** | **Compound** | **IC₅₀** | **Compound** | **IC₅₀** | **Compound** | **IC₅₀** |
|---|---|---|---|---|---|---|---|---|---|
| **BI853520** | 10.78 | **21** | 8.50 | **66** | 7.01 | **94** | 2.58 | **111** | 1.62 |
| **Compound A** | 16.84 | **34** | 86 | **89** | 0.73 | **100** | 1.33 | **112** | 0.93 |
| **Compound B** | 12.50 | **62** | 2.88 | **90** | 1.86 | **101** | 2.26 | **113** | 3.56 |
| **19** | 4.23 | **64** | 3.33 | **92** | 3.05 | **102** | 3.01 | **114** | 2.63 |

As can be seen from the data in the table above, the inhibitory activity of some of the compounds of the present invention against PC3 cell adherence was significantly better than that of the control compound BI853520.

### Example 121. Assay for Inhibition of FAK Y397 Phosphorylation by Compounds of the Present Invention

PC3 cells were added at 8000 cells/well. 3 h after the cells were treated with the compound, the phosphorylation level at the FAK Y397 site in the cells was detected by enzyme-linked immunosorbent assay (ELISA) using an antibody specifically recognizing the phosphorylation at the FAK Y397 site. The inhibition percentages and IC₅₀ of the compounds were calculated compared to the DMSO group.

**Example 122. *In Vivo* Pharmacokinetic Experiment of Compounds of the Present Invention** CD-1 female mice aged 7 to 10 weeks were intravenously administered and orally administered at a dose of 2 mg/kg and 10 mg/kg, respectively. The mice were fasted for at least 12 h before the administration and given food 4 h after the administration, and they were given *ad libitum* access to water during the experiment.

On the day of the experiment, animals in the intravenous group were administered the corresponding compound by single injection via the tail vein at an administration volume of 10 mL/kg, and animals in the oral group were administered the corresponding compound by single intragastric injection at an administration volume of 10 mL/kg. The animals were weighed before administration, and the administration volume was calculated according to the body weight. The sample collection time was 0.083 h, 0.167 h, 0.5 h, 1 h, 2 h, 4 h, 8 h and 24 h. About 200 µL of whole blood was collected through the orbital venous plexus at each time point and used to prepare plasma for concentration determination by high-performance liquid chromatographytandem mass spectrometry (LC-MS/MS). The plasma concentrations were processed using a non-compartmental model of Winnolin pharmacokinetic software, and the pharmacokinetic parameters were calculated using a linear-log trapezoidal method. The evaluation results of PK properties in mice are shown in Table 15.

**Table 15. Results of in vivo pharmacokinetic evaluation of some of the compounds of the present invention**

| **Route of administration** | **Pharmacokinetic parameters** | **Compound 19** | **Compound 20** | **Compound 89*** | **Compound 90*** | **BI853520** |
|---|---|---|---|---|---|---|
| Injection (2 mg/kg) | Vdss (L/Kg) | 3.95 | 4.99 | 5.58 | 5.34 | 5.03 |
| | T_{1/2} (h) | 2.50 | 3.46 | 4.41 | 3.08 | 3.62 |
| | Cl (mL/min/Kg) | 23.79 | 26.79 | 16.95 | 20.53 | 22.53 |
| | AUC₀₋ₗₐₛₜ (h*ng/mL) | 1400.88 | 622.19 | 983.39 | 811.61 | 1479.26 |
| Oral administration (10 mg/kg) | Cₘₐₓ (ng/mL) | 648 | 189.67 | 754.33 | 634.67 | 635.67 |
| | Tₘₐₓ (h) | 4 | 2 | 2 | 4 | 4 |
| | T_{1/2} (h) | 2.98 | 1.97 | 5.81 | 2.82 | 3.10 |
| | AUC₀₋ₗₐₛₜ (h*ng/mL) | 6689.72 | 933.77 | 9400.85 | 7459.78 | 7449.81 |
| | F% | 95.51 | 30.02 | 95.6 | 91.95 | 100.72 |

| | | | | | | |
|---|---|---|---|---|---|---|
| *Note: The injection groups of compounds 89 and 90 were administered at a dose of 1 mg/kg | | | | | | |

As can be seen from the data in the table above, compound 19, compound 89 and compound 90 had good oral bioavailability and good oral absorption properties, which are of great significance in improving the efficacy of drugs, reducing the dose of administration and reducing the cost of administration.

### Example 123. In Vivo Efficacy Test of Compounds of the Present Invention in MiaPaCa-2 Model

Female BALB/c nude mice (aged 6 weeks, 18-22 g) were provided by Vital River Laboratory Animal Technology Co., Ltd. (China) and used one week after quarantine and acclimation. All animals were kept in a room at 23±2 °C with a relative humidity of 50±5%, artificial lighting was provided from 08:00 to 20:00 every day, and the air was exchanged 13-18 times per hour. They had free access to standard laboratory diets and water.

Human pancreatic cancer Mia PaCa-2 cells were cultured conventionally in 1640 medium containing 10% fetal bovine serum in a 37 °C/5% CO₂ incubator. After being subcultured, the cells were collected when they reached the desired amount. 1 × 10⁷ Mia PaCa-2 cells were injected into the left dorsal side of each nude mouse, and the animals were randomly grouped for administration after tumors grew to 100-200 mm³. The mice in the solvent control group were intragastrically administered 0.5% MC-Tween-80 twice a day; the mice in the compound groups were intragastrically administered 0.5% MC-Tween-80 suspension once a day. On Tuesday and Thursday each week, tumor volumes and body weight of the mice were measured, and the nude mice were sacrificed on day 21 after the administration.

The ability of the compounds to inhibit tumor growth was evaluated according to the tumor growth inhibition rate (TGI) = 1 - (tumor volume on day 28 in treatment group - tumor volume on day 1 in treatment group)/(administration volume on day 28 in control group - tumor volume on day 1 in control group). The toxicity of the compounds was evaluated according to the body weight of the mice.

The groups were as follows:
1) a solvent control group; 2) a control compound BI853520 group; 3) a control compound B group; 4) a compound 19 group; 5) a compound 89 group; 6) a compound 90 group; 7) a compound 111 group.

The results are shown in Table 16 below.

**Table 16. In vivo efficacy of some of the compounds of the present invention in MiaPaCa-2 model**

| **Group** | **Compound** | **Dose** | **Administration period** | **Route of administration** | **TGI(%)** | **Change in body weight** |
|---|---|---|---|---|---|---|
| 1 | Solvent control group | - | QD (21 days) | PO | -- | +18.9% |
| 2 | BI853520 | 25mg/kg | QD (21 days) | PO | 73.51% | +6.48% |
| 3 | Compound B | 25mg/kg | QD (21 days) | PO | 75.24% | +5.05% |
| 4 | Compound 19 | 25mg/kg | QD (21 days) | PO | 80.54% | +1.86% |
| 5 | Compound 89 | 25mg/kg | QD (21 days) | PO | 91.42% | + 1.26% |
| 6 | Compound 90 | 25mg/kg | QD (21 days) | PO | 83.72% | +3.67% |
| 7 | Compound 111 | 25mg/kg | QD (21 days) | PO | 82.32% | +6.08% |

As can be seen from the results of the *in vivo* experiments described above, the compounds of the present invention had significant inhibitory effects on the tumor growth in the human pancreatic cancer MiaPaCa-2 model and also had significant advantages compared to the control compounds. Moreover, all compounds showed good toxicity tolerance.

### Example 124. In Vivo Efficacy Test of Compounds of the Present Invention in PC-3 Model

Female BALB/c nude mice (aged 6 weeks, 18-22 g) were provided by Vital River Laboratory Animal Technology Co., Ltd. (China) and used one week after quarantine and acclimation. All animals were kept in a room at 23±2 °C with a relative humidity of 50±5%, artificial lighting was provided from 08:00 to 20:00 every day, and the air was exchanged 13-18 times per hour. They had free access to standard laboratory diets and water.

Human prostatic cancer PC3 cells were cultured conventionally in 1640 medium containing 10% fetal bovine serum in an incubator at 37 °C with 5% CO₂. After being subcultured, the cells were collected when they reached the desired amount. 1 × 10⁷ PC3 cells were injected into the left dorsal side of each nude mouse, and the animals were randomly grouped for administration after tumors grew to 100-200 mm³. The mice in the solvent control group were intragastrically administered 0.5% MC-Tween-80 twice a day; the mice in the compound groups were intragastrically administered 0.5% MC-Tween-80 suspension once a day. On Tuesday and Thursday each week, tumor volumes and body weight of the mice were measured, and the nude mice were sacrificed on day 21 after the administration.

The ability of the compounds to inhibit tumor growth was evaluated according to the tumor growth inhibition rate (TGI) = 1 - (tumor volume on day 28 in treatment group - tumor volume on day 1 in treatment group)/(administration volume on day 28 in control group - tumor volume on day 1 in control group). The toxicity of the compounds was evaluated according to the body weight of the mice.

The groups were as follows:
1) a solvent control group; 2) a control compound BI853520 group; 3) a compound 89 group; 4) a compound 90 group; 5) a compound 111 group.

The results are shown in Table 17 below.

**Table 17. In vivo efficacy of some of the compounds of the present invention in PC-3 model**

| **Group** | **Compound** | **Dose** | **Administration period** | **Route of administration** | **TGI (%)** | **Change in body weight** |
|---|---|---|---|---|---|---|
| 1 | Solvent control group | - | QD (21 days) | PO | -- | -0.2% |
| 2 | BI853520 | 25mg/kg | QD (21 days) | PO | 74.48% | -2.3% |
| 3 | Compound 89 | 25mg/kg | QD (21 days) | PO | 88.52% | -1.2% |
| 4 | Compound 90 | 25mg/kg | QD (21 days) | PO | 81.65% | -0.5% |
| 5 | Compound 111 | 25mg/kg | QD (21 days) | PO | 87.4% | -1.4% |

As can be seen from the results of the *in vivo* experiments described above, the compounds of the present invention had significant inhibitory effects on the tumor growth in the human prostatic cancer PC-3 model and also had significant advantages compared to the control compound. Moreover, all compounds showed good toxicity tolerance.

### Example 125. In Vivo Efficacy Experiment of Compounds of the Present Invention in MC38 Model

Female BALB/c nude mice (aged 6 weeks, 18-22 g) were provided by Vital River Laboratory Animal Technology Co., Ltd. (China) and used one week after quarantine and acclimation. All animals were kept in a room at 23±2 °C with a relative humidity of 50±5%, artificial lighting was provided from 08:00 to 20:00 every day, and the air was exchanged 13-18 times per hour. They had free access to standard laboratory diets and water.

Mouse colon cancer MC38 cells were cultured conventionally in 1640 medium containing 10% fetal bovine serum in an incubator at 37 °C with 5% CO₂. After being subcultured, the cells were collected when they reached the desired amount. The BALB/c mice were subcutaneously injected with 1 × 10⁶ MC38 cells on the right side to form tumors. After the tumors grew to about 100 mm³, the animals were randomly divided into a solvent control group, a test compound single drug group, a combination group of a test compound + PD-1 and a PD-1 single drug (purchased from Bio xCELL) group. The administration was then started. Tumor volumes were measured with a caliper on day 3, day 7, day 10, day 14, day 17 and day 21 after the administration.

The ability of the compounds to inhibit tumor growth was evaluated according to the tumor growth inhibition rate (TGI) = 1 - (tumor volume on day 28 in treatment group - tumor volume on day 1 in treatment group)/(administration volume on day 28 in control group - tumor volume on day 1 in control group). The toxicity of the compounds was evaluated according to the body weight and state of the mice.

The groups were as follows:
1) a solvent control group; 2) a control compound BI853520 group; 3) a PD-1 monoclonal antibody group; 4) a compound 89 group; 5) a compound 90 group; 6) a compound group 111; 7) a control compound BI853520 + PD-1 monoclonal antibody group; 8) a compound 89 + PD-1 monoclonal antibody group; 9) a compound 90 + PD-1 monoclonal antibody group; 10) a compound 111 + PD-1 monoclonal antibody group.

The results are shown in Table 18 below.

**Table 18. In vivo efficacy of some of the compounds of the present invention in MC38 model**

| **Group** | **Compound** | **Dose** | **Administration period** | **Route of administration** | **TGI (%)** | **Change in body weight** |
|---|---|---|---|---|---|---|
| 1 | Solvent control group | - | QD (21 days) | PO | -- | +30.81% |
| 2 | BI853520 | 50mg/kg | QD (21 days) | PO | 52.64% | +18.74% |
| 3 | PD-1 monoclonal antibody | 1.5mg/kg | QW (21 days) | IV | 65.49% | +20.53% |
| 4 | Compound 89 | 50mg/kg | QD (21 days) | PO | 42.58% | +18.47% |
| 5 | Compound 90 | 50mg/kg | QD (21 days) | PO | 59.02% | +13.54% |
| 6 | Compound 111 | 50mg/kg | QD (21 days) | PO | 47.67% | +21.07% |
| 7 | BI853520 + PD-1 monoclonal antibody | 50mg/kg +1.5mg/kg | QD (21 days) + QW (21 days) | PO +IV | 70.50% | +18.66% |
| 8 | Compound 89 + PD-1 monoclonal antibody | 50mg/kg +1.5mg/kg | QD (21 days) + QW (21 days) | PO +IV | 77.94% | +17.34% |
| 9 | Compound 90 + PD-1 monoclonal antibody | 50mg/kg+1.5mg/kg | QD (21 days) + QW (21 days) | PO +IV | 83.43% | +9.50% |
| 10 | Compound 111 + PD-1 monoclonal antibody | 50mg/kg +1.5mg/kg | QD (21 days) + QW (21 days) | PO +IV | 78.59% | +16.78% |

As can be seen from the results of the *in vivo* experiments described above, the combination of the compound of the present invention and the PD-1 monoclonal antibody had a good inhibitory effect on the tumor growth in the mouse colon cancer MC-38 model, while the combination effect of the control compound on the model was not significant. Moreover, all compounds showed good toxicity tolerance.

Although specific embodiments of the present invention have been described above, it will be appreciated by those skilled in the art that these embodiments are merely illustrative and that many changes or modifications can be made to these embodiments without departing from the principles and spirit of the present invention. The protection scope of the present invention is therefore defined by the appended claims.

## Claims

1. A compound of general formula (1) or an isomer, a crystalline form, a pharmaceutically acceptable salt, a hydrate or a solvate thereof: 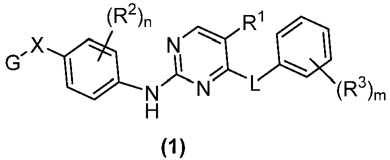 wherein in general formula (1):
L is selected from -CH₂-, -O- or -S-;
X is selected from a chemical bond, 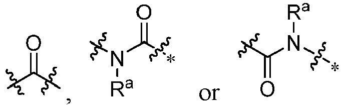 with * representing attachment to a phenyl ring;
G is selected from (9-18 membered) heterocycloalkyl, (C9-C18) cycloalkyl or (9-18 membered) heteroaryl, wherein the (9-18 membered) heterocycloalkyl, (C9-C18) cycloalkyl or (9-18 membered) heteroaryl may be each independently and optionally substituted with 1, 2, 3 or 4 R^{c};
R¹ is selected from -H, -D, halogen, hydroxy, amino, cyano, nitro, -OR^{a}, -NR^{a}R^{b}, -C(O)R^{a}, - CO₂R^{a}, -CONR^{a}R^{b}, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl or (5-14 membered) heteroaryl, wherein the (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl or (5-14 membered) heteroaryl may be each independently and optionally substituted with 1, 2, 3 or 4 R^{c};
R² is selected from -H, -D, halogen, hydroxy, amino, cyano, nitro, -OR^{a}, -NR^{a}R^{b}, (C1-C8) alkyl,
(C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl or (5-14 membered) heteroaryl, wherein the (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl or (5-14 membered) heteroaryl may be each independently and optionally substituted with 1, 2, 3 or 4 R^{c};
R³ is selected from -H, -D, halogen, hydroxy, amino, cyano, nitro, -OR^{3a}, -NR^{3a}R^{3b}, -C(O)R^{3a}, - CO₂R^{3a}, -S(O)ₚR^{3a}, -S(O)ₚNR^{3a}R^{3b}, -CONR^{3a}R^{3b}, -C(=NR^{3a})-NR^{3b}R^{3c}, -NR^{3a}COR^{3b}, - NR^{3a}CONR^{3b}R^{3c}, -NR^{3a}CO₂R^{3b}, -NR^{3a}S(O)ₚNR^{3b}R^{3c}, -NR^{3a}S(O)ₚR^{3b}, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C1-C8) alkoxy, -(C1-C8) alkylene-(C3-C14) cycloalkyl, -(C1-C8) alkylene-(3-14 membered) heterocycloalkyl, -(C1-C8) alkylene-(C6-C14) aryl and -(C1-C8) alkylene-(5-14 membered) heteroaryl; or two R³ attached to two adjacent carbon atoms, together with the two adjacent carbon atoms to which they are attached, form one partially unsaturated (C4-C7) cycloalkyl or
partially unsaturated (4-7 membered) heterocycloalkyl, wherein the partially unsaturated (C4-C7) cycloalkyl or partially unsaturated (4-7 membered) heterocycloalkyl can be each independently and optionally substituted with 1, 2, 3, 4 or 5 R^{c};
R^{a} and R^{b} are each independently -H, -D, halogen, hydroxy, amino, cyano, nitro, (C1-C8) alkyl,
(C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl or -(C1-C8) alkylene-(5-14 membered) heteroaryl, wherein the (C1-C8) alkyl,
(C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl or -(C1-C8) alkylene-(5-14 membered) heteroaryl may be each independently and optionally substituted with 1, 2, 3 or 4 R^{c};
R^{c} is -H, -D, halogen, hydroxy, amino, cyano, nitro, -OR^{xa}, -NR^{xa}R^{xb}, -(CH₂)ₘOR^{xa}, - (CH₂)ₘNR^{xa}R^{xb}, -C(O)R^{xa}, -CO₂R^{xa}, -S(O)ₚR^{xa}, -S(O)ₚNR^{xa}R^{xb}, -CONR^{xa}R^{xb}, -C(=NR^{xa})-NR^{xb}R^{xc}, -NR^{xa}COR^{xb} , -NR^{xa}CONR^{xb}R^{xc}, -NR^{xa}CO₂R^{xb}, -NR^{xa}S(O)ₚNR^{xb}R^{xc}, -NR^{xa}S(O)ₚR^{xb}, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, - (C1-C8) alkylene-(C1-C8) alkoxy, -(C1-C8) alkylene-(C3-C14) cycloalkyl, -(C1-C8) alkylene-(3-14 membered) heterocycloalkyl, -(C1-C8) alkylene-(C6-C14) aryl or -(C1-C8) alkylene-(5-14 membered) heteroaryl; or when two R^{c} are attached to the same atom, two R^{c} may form one oxo;
R^{xa}, R^{xb} and R^{xc} are each independently -H, (C1-C8) alkyl, (C1-C8) alkoxy, (C1-C8) haloalkyl, (C2-C8) alkenyl, (C2-C8) alkynyl, (C3-C14) cycloalkyl, (3-14 membered) heterocycloalkyl, (C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl or -(C1-C8) alkylene-(5-14 membered) heteroaryl;
R^{3a}, R^{3b} and R^{3c} are each independently -H, (C1-C8) alkyl, (C1-C8) alkoxy, (C1-C8) haloalkyl,
(C2-C8) alkenyl, (C2-C8) alkynyl, (C3-C14) cycloalkyl, (3-14 membered) heterocycloalkyl,
(C6-C14) aryl, (5-14 membered) heteroaryl, -(C1-C8) alkylene-(C3-C14) cycloalkyl, -(C1-C8) alkylene-(3-14 membered) heterocycloalkyl, -(C1-C8) alkylene-(C6-C14) aryl or -(C1-C8) alkylene-(5-14 membered) heteroaryl;
p is an integer of 0, 1 or 2;
m is an integer of 0, 1, 2 or 3;
n is an integer of 0, 1, 2 or 3.

2. The compound of general formula (1) or the isomer, the crystalline form, the pharmaceutically acceptable salt, the hydrate or the solvate thereof according to claim 1, wherein in general formula (1), L is -CH₂- or -O-; L is preferably -O-.

3. The compound of general formula (1) or the isomer, the crystalline form, the pharmaceutically acceptable salt, the hydrate or the solvate thereof according to claim 1 or 2, wherein in general formula (1), G is (9-15 membered) heterocycloalkyl, wherein the (9-15 membered) heterocycloalkyl can be optionally substituted with 1, 2, 3 or 4 of the following groups: -H, -D, -F, -Cl, -Br, -I, -OH, -NH₂, -CN, -NO₂, -OCH₃, -NHCH₃, -N(CH₃)₂, -C(O)NH₂, -OCH₂CH₃, - OCF₃, -CH₃, -CD₃, -CH₂CH₃, -CH₂F, -CHF₂, 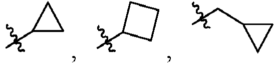 or -CF₃; or two substituents attached to the same atom can form one oxo.

4. The compound of general formula (1) or the isomer, the crystalline form, the pharmaceutically acceptable salt, the hydrate or the solvate thereof according to claim 3, wherein in general formula (1), G is (9-12 membered) heterocycloalkyl, wherein the (9-12 membered) heterocycloalkyl can be optionally substituted with 1, 2, 3 or 4 of the following groups: -H, -D, -CH₃, -CD₃, -CH₂CH₃, 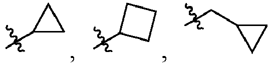 or -OCH₃, preferably -H or -CH₃; or two substituents attached to the same atom can form one oxo; preferably, G is (9-11 membered) heterocyclic spirocycloalkyl.

5. The compound of general formula (1) or the isomer, the crystalline form, the pharmaceutically acceptable salt, the hydrate or the solvate thereof according to claim 4, wherein in general formula (1), G is selected from 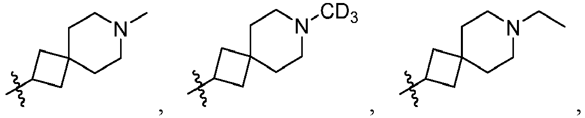 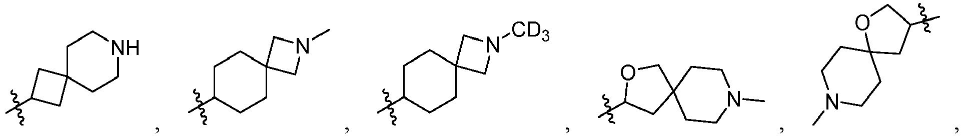 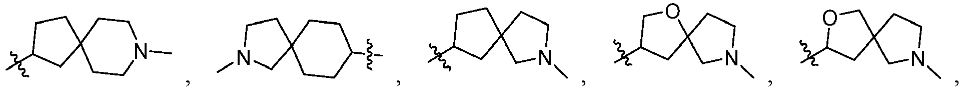 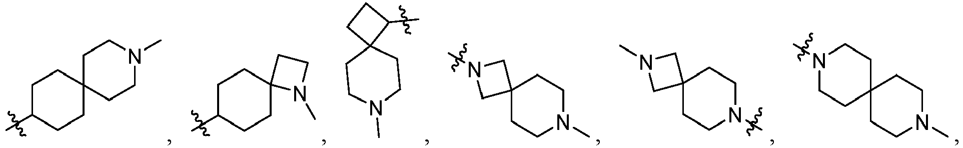 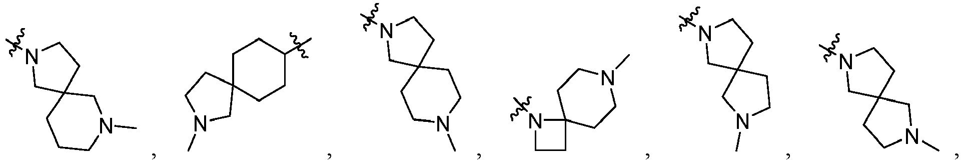 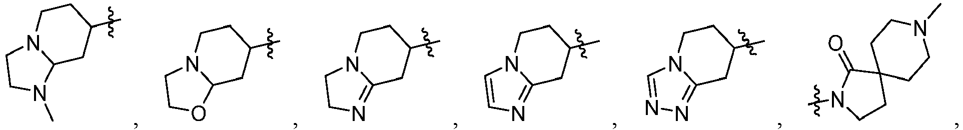 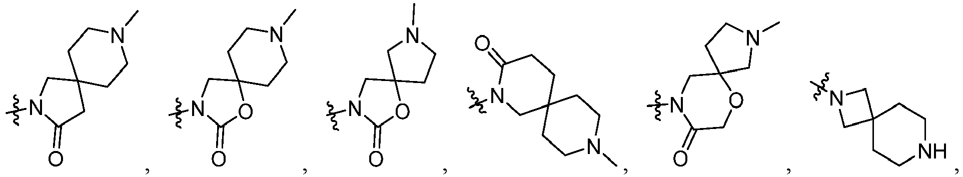 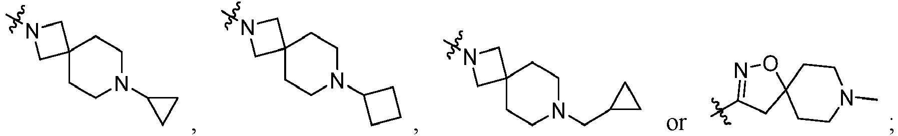 G is preferably 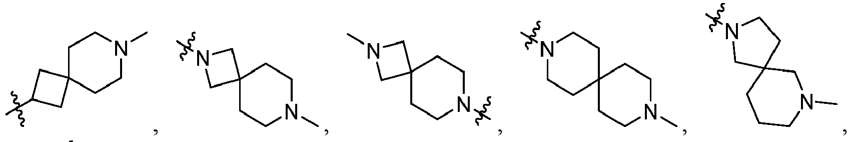 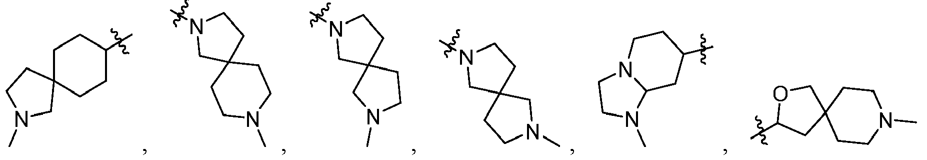 or

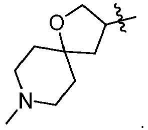

6. The compound of general formula (1) or the isomer, the crystalline form, the pharmaceutically acceptable salt, the hydrate or the solvate thereof according to any one of claims 1-5, wherein in general formula (1), X is selected from a chemical bond, 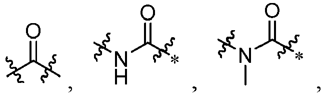 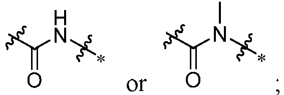 X is preferably selected from a chemical bond, 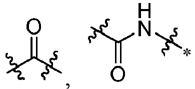 or 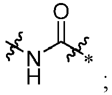 X is more preferably selected from a chemical bond, 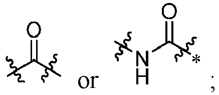 X is more preferably 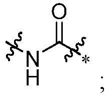 X is more preferably 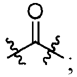 X is more preferably a chemical bond; wherein * represents attachment to a phenyl ring.

7. The compound of general formula (1) or the isomer, the crystalline form, the pharmaceutically acceptable salt, the hydrate or the solvate thereof according to any one of claims 1-6, wherein in general formula (1), R¹ is selected from -H, -D, -F, -Cl, -Br, -I, -OH, -NH₂, -CN, -NO₂, -OCH₃, -NHCH₃, -N(CH₃)₂, -C(O)NH₂, -OCH₂CH₃, -OCF₃, -CH₃, -CD₃, -CH₂CH₃, -CH₂F, -CHF₂ or - CF₃; R¹ is preferably -F, -Cl, -Br, -CN, -NO₂, -CF₃ or -C(O)NH₂; R¹ is more preferably -CF₃; R¹ is more preferably -Cl, -Br, -CN or -NO₂.

8. The compound of general formula (1) or the isomer, the crystalline form, the pharmaceutically acceptable salt, the hydrate or the solvate thereof according to any one of claims 1-7, wherein in general formula (1), R² is -H, -D, -F, -Cl, -Br, -I, -OH, -NH₂, -CN, -NO₂, -OCF₃, -NHCH₃, - N(CH₃)₂, -NHCH₂CH₃, -N(CH₂CH₃)₂, (C1-C3) alkyl, (C1-C3) alkoxy, (C1-C3) haloalkyl, (C2-C4) alkenyl, (C2-C4) alkynyl, (C3-C6) cycloalkyl, (3-6 membered) heterocycloalkyl, (C6-C10) aryl or (5-10 membered) heteroaryl, wherein the (C1-C3) alkyl, (C1-C3) alkoxy, (C1-C3) haloalkyl, (C2-C4) alkenyl, (C2-C4) alkynyl, (C3-C6) cycloalkyl, (3-6 membered) heterocycloalkyl, (C6-C10) aryl or (5-10 membered) heteroaryl can be each independently and optionally substituted with 1, 2, 3 or 4 of the following groups: -H, -D, -F, -Cl, -Br, -I, -OH, - NH₂, -CN, -NO₂, -OCH₃, -NHCH₃, -N(CH₃)₂, -C(O)NH₂, -OCH₂CH₃, -OCF₃, -CH₃, -CD₃, - CH₂CH₃, -CH₂F, -CHF₂ or -CF₃.

9. The compound of general formula (1) or the isomer, the crystalline form, the pharmaceutically acceptable salt, the hydrate or the solvate thereof according to claim 8, wherein in general formula (1), R² is selected from -H, -D, -F, -Cl, -Br, -I, -OH, -NH₂, -CN, -NO₂, -OCH₃, -NHCH₃, -N(CH₃)₂, -C(O)NH₂, -OCH₂CH₃, -OCF₃, -CH₃, -CD₃, -CH₂CH₃, -CH₂F, -CHF₂, -CF₃, 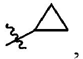 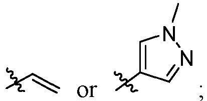 R² is preferably -D, -F, -Cl -OCH₃, -OCF₃, -CH₃, -CD₃, -CH₂CH₃, -CF₃, 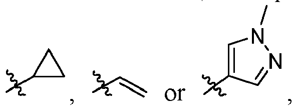 and n is 1 or 2; R² is more preferably -F, -Cl, -OCH₃, -OCF₃, - CH₂CH₃, 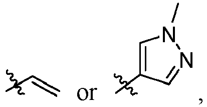 and n is 1 or 2; R² is more preferably -F, -Cl or -OCH₃, and n is 1 or 2; R² is more preferably -F or -OCH₃, and n is 1 or 2.

10. The compound of general formula (1) or the isomer, the crystalline form, the pharmaceutically acceptable salt, the hydrate or the solvate thereof according to any one of claims 1-9, wherein in general formula (1), R³ is selected from -H, -D, halogen, hydroxy, amino, cyano, nitro, -OR^{3a}, -NR^{3a}R^{3b}, -C(O)R^{3a}, -CO₂R^{3a}, -S(O)ₚR^{3a}, -S(O)ₚNR^{3a}R^{3b}, -CONR^{3a}R^{3b},-C(=NR^{3a})-NR^{3b}R^{3c}, -NR^{3a}COR^{3b}, -NR^{3a}CONR^{3b}R^{3c}, -NR^{3a}CO₂R^{3b}, -NR^{3a}S(O)ₚNR^{3b}R^{3c}, - NR^{3a}S(O)ₚR^{3b}, (C1-C6) alkyl, (C1-C6) alkoxy, (C1-C6) haloalkyl, (C2-C6) alkenyl, (C2-C6) alkynyl, (C3-C10) cycloalkyl, (3-10 membered) heterocycloalkyl, (C6-C10) aryl, (5-10 membered) heteroaryl, -(C1-C3) alkylene-(C1-C6) alkoxy, -(C1-C3) alkylene-(C3-C10) cycloalkyl, -(C1-C3) alkylene-(3-10 membered) heterocycloalkyl, -(C1-C3) alkylene-(C6-C10) aryl or -(C1-C3) alkylene-(5-10 membered) heteroaryl.

11. The compound of general formula (1) or the isomer, the crystalline form, the pharmaceutically acceptable salt, the hydrate or the solvate thereof according to claim 10, wherein in general formula (1), R³ is selected from -H, -D, -F, -Cl, -Br, -I, -OH, -NH₂, -CN, - NO₂, -OCH₃, -NHCH₃, -N(CH₃)₂, -C(O)NH₂, -OCH₂CH₃, -OCF₃, -CH₃, -CD₃, -CH₂CH₃, -CH₂F, -CHF₂, -CF₃, 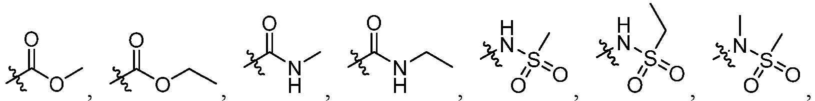 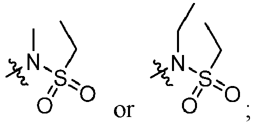 R³ is preferably selected from -H, -D, -F, -Cl, -CN, 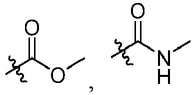 or 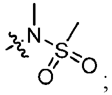 R³ is more preferably -H,

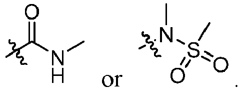

12. The compound of general formula (1) or the isomer, the crystalline form, the pharmaceutically acceptable salt, the hydrate or the solvate thereof according to claim 11 wherein in general formula (1), structural unit 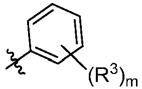 is selected from 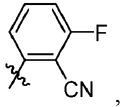 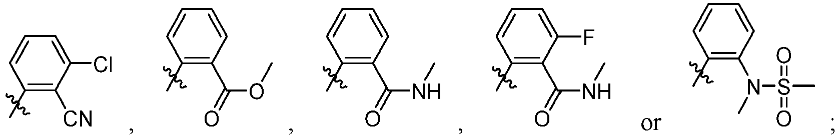 preferably

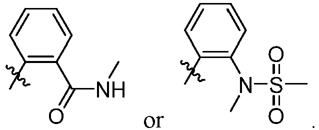

13. The compound of general formula (1) or the isomer, the crystalline form, the pharmaceutically acceptable salt, the hydrate or the solvate thereof according to any one of claims 1-9, wherein in general formula (1), two R³ attached to two adjacent carbon atoms, together with the two adjacent carbon atoms to which they are attached, form one partially unsaturated (C4-C7) cycloalkyl or partially unsaturated (4-7 membered) heterocycloalkyl, wherein the partially unsaturated (C4-C7) cycloalkyl or partially unsaturated (4-7 membered) heterocycloalkyl can be each independently and optionally substituted with 1, 2, 3, 4 or 5 substituents of -H, -D, -CH₃, -CD₃, -CH₂CH₃ or -OCH₃, preferably -H, -CH₃ or -OCH₃; or two substituents attached to the same atom can form one oxo; preferably, two R³ attached to two adjacent carbon atoms, together with the two adjacent carbon atoms to which they are attached, form one partially unsaturated (C5) cycloalkyl or partially unsaturated (5-membered) heterocycloalkyl, wherein the partially unsaturated (C5) cycloalkyl or partially unsaturated (5-membered) heterocycloalkyl can be each independently and optionally substituted with 1, 2 or 3 substituents of -D, -CH₃, -CD₃, -CH₂CH₃ or -OCH₃, and the substituent is preferably 1, 2 or 3 groups of -CH₃ or -OCH₃; more preferably, two R³ attached to two adjacent carbon atoms, together with the two adjacent carbon atoms to which they are attached, form one partially unsaturated (C5) cycloalkyl substituted with 1 oxo or partially unsaturated (5-membered) heterocycloalkyl substituted with 1 oxo, wherein the partially unsaturated (C5) cycloalkyl substituted with 1 oxo or partially unsaturated (5-membered) heterocycloalkyl substituted with 1 oxo can be each independently and optionally substituted with 1, 2 or 3 substituents of -CH₃ or -OCH₃.

14. The compound of general formula (1) or the isomer, the crystalline form, the pharmaceutically acceptable salt, the hydrate or the solvate thereof according to claim 13, wherein in general formula (1), structural unit 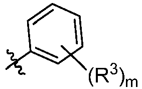 is selected from 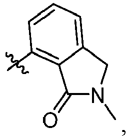 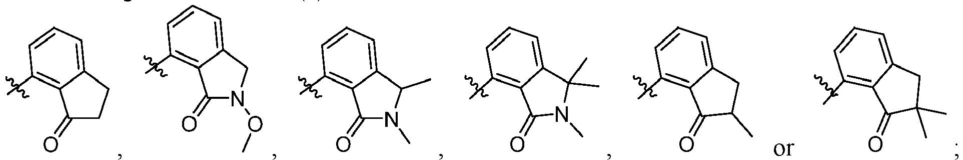 preferably 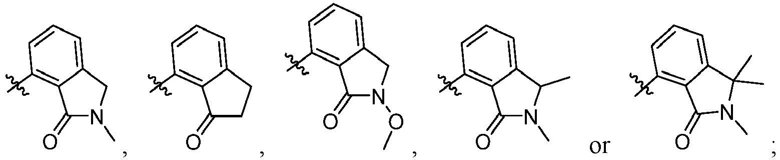 more preferably

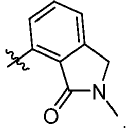

15. The compound of general formula (1) or the isomer, the crystalline form, the pharmaceutically acceptable salt, the hydrate or the solvate thereof according to any one of claims 1-14, wherein the compound has one of the following structures:

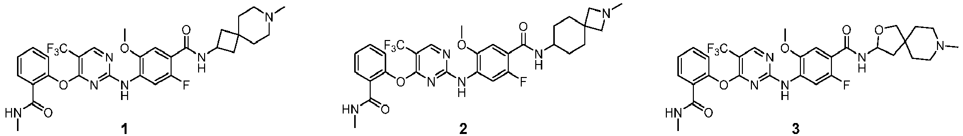

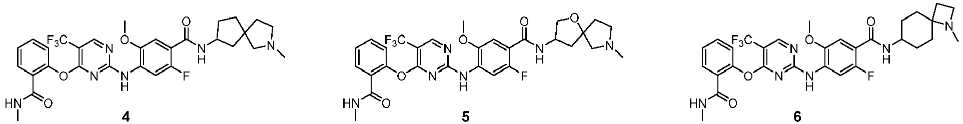

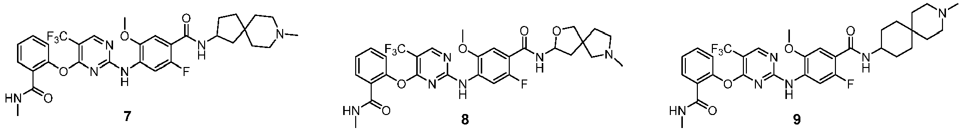

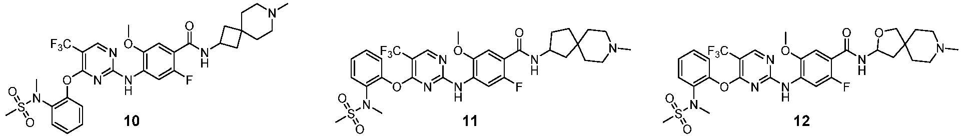

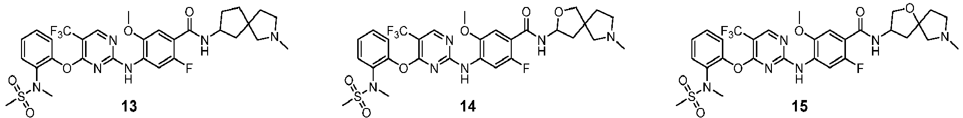

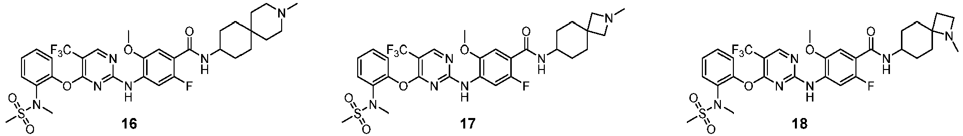

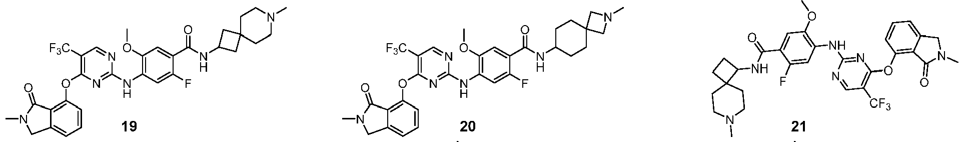

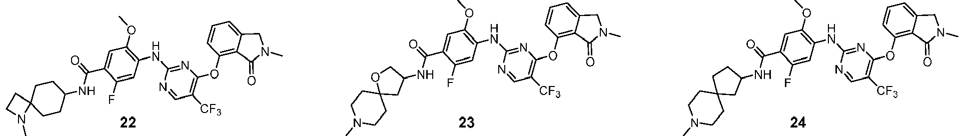

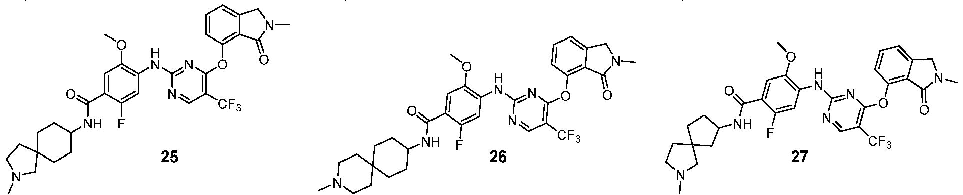

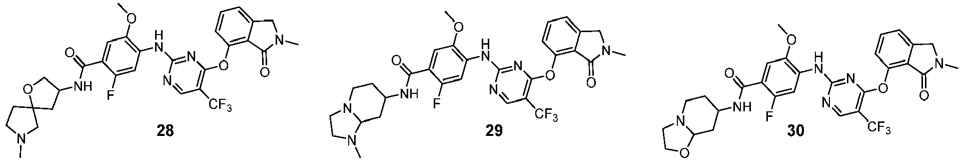

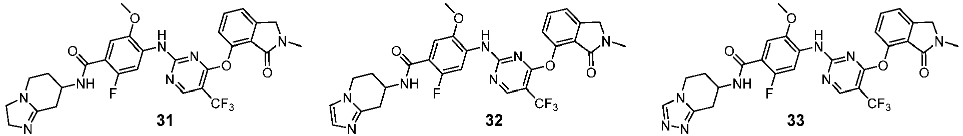

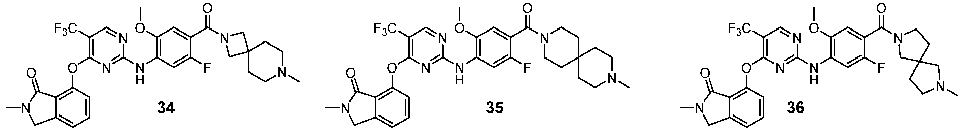

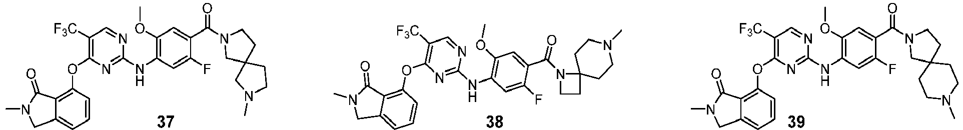

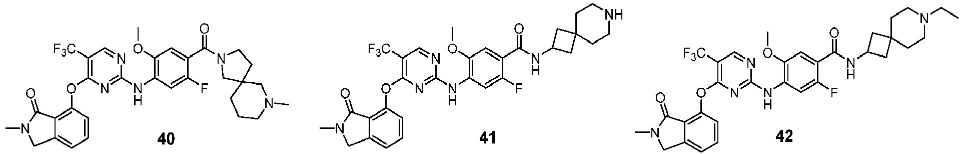

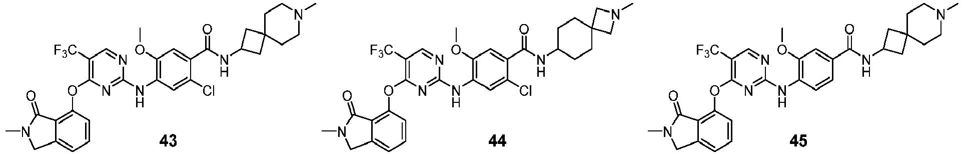

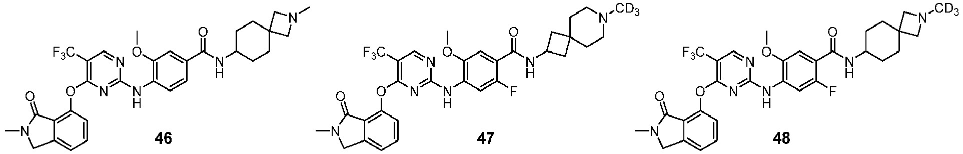

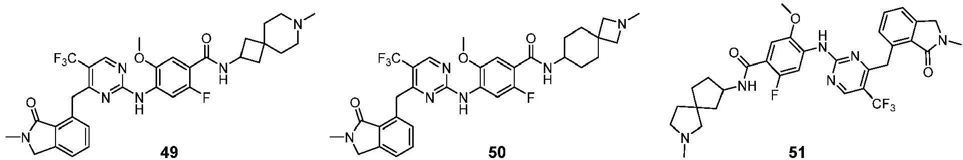

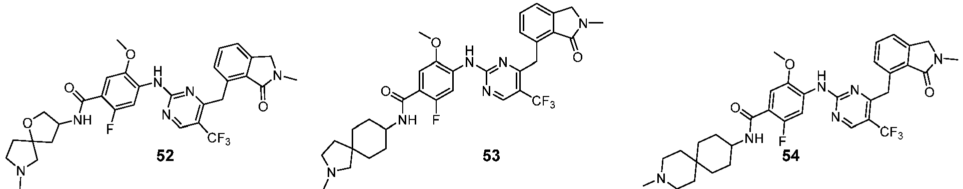

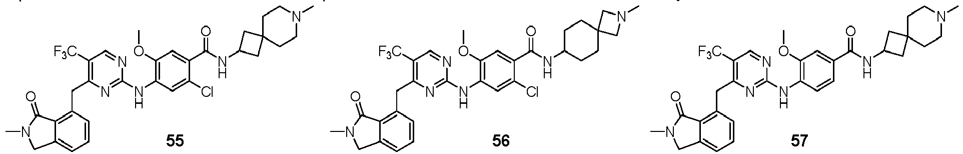

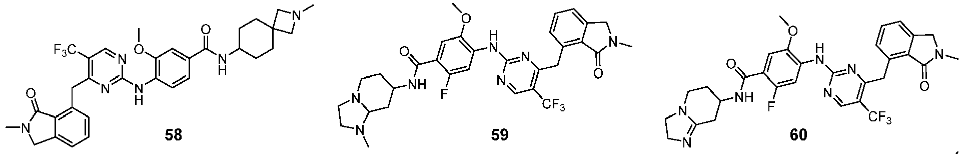

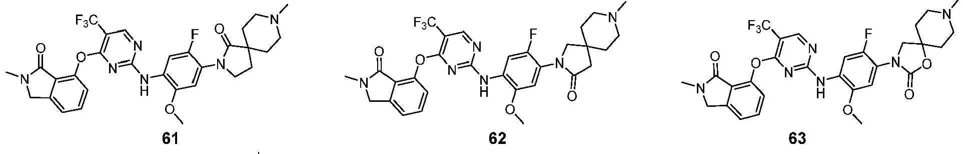

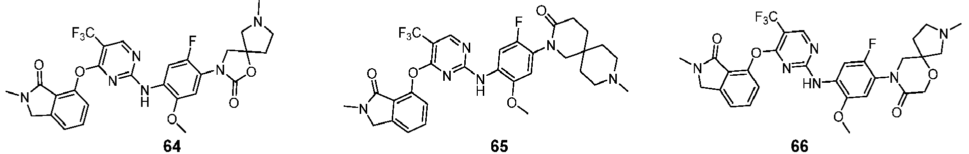

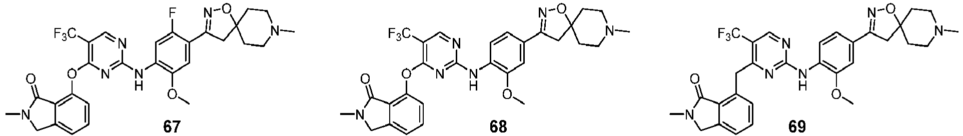

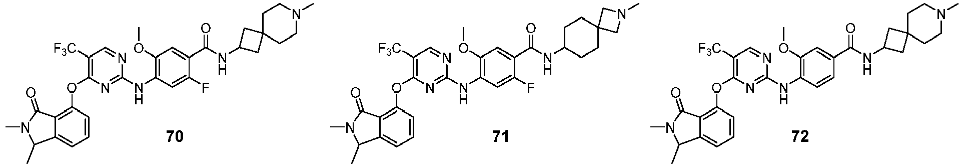

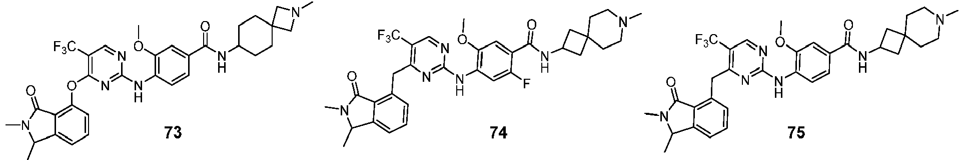

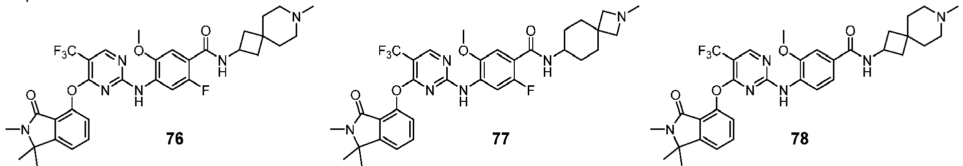

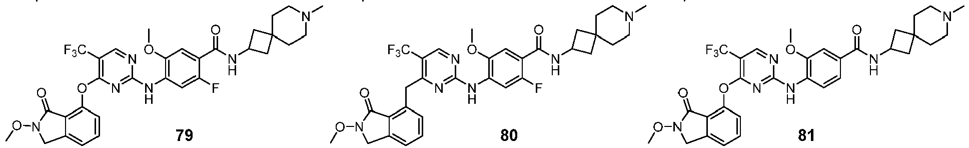

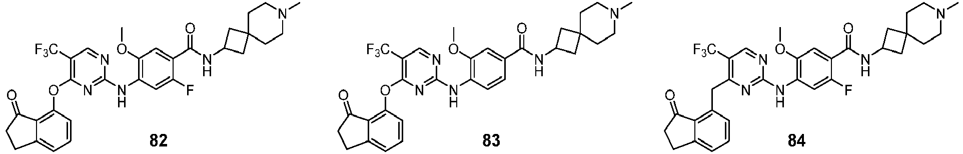

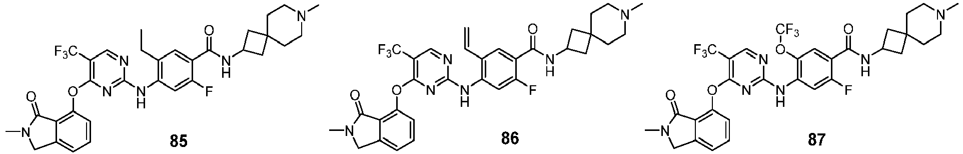

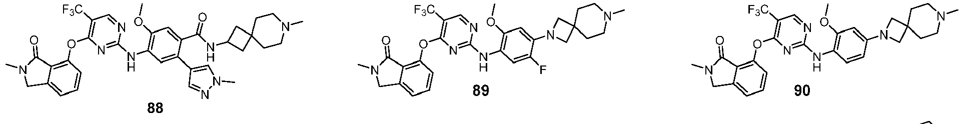

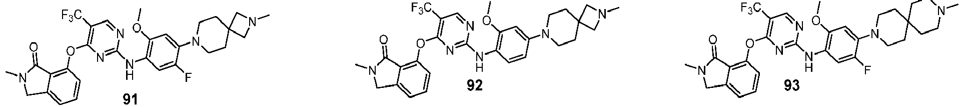

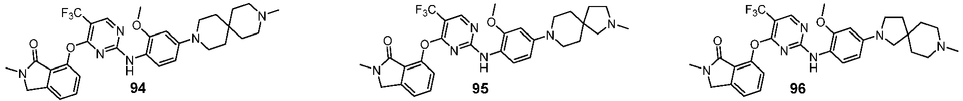

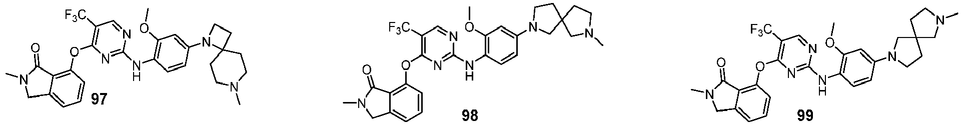

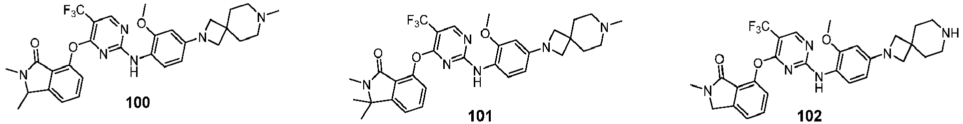

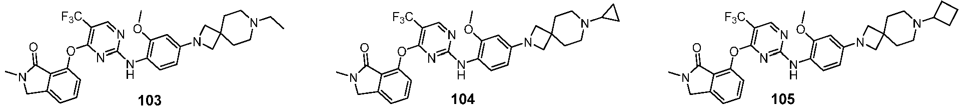

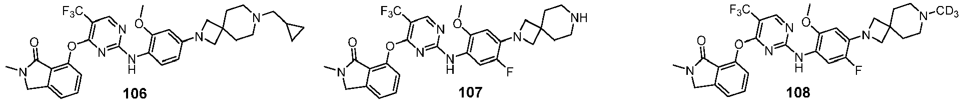

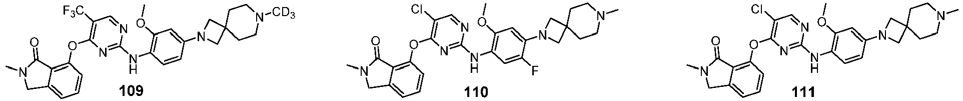

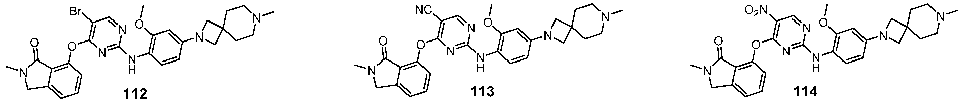

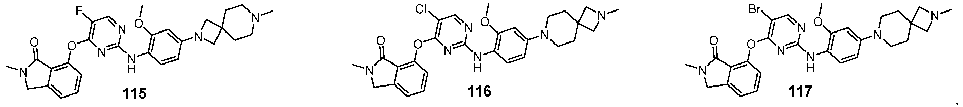

16. A pharmaceutical composition, comprising a pharmaceutically acceptable excipient or carrier, and the compound of general formula (1) or the isomer, the crystalline form, the pharmaceutically acceptable salt, the hydrate or the solvate thereof according to any one of claims 1-15 as an active ingredient.

17. Use of the compound of general formula (1) or the isomer, the crystalline form, the pharmaceutically acceptable salt, the hydrate or the solvate thereof according to any one of claims 1-15, or the pharmaceutical composition according to claim 16 in the preparation of a medicament for treating a disease related to FAK kinase.

18. The use according to claim 17, wherein the disease related to the FAK kinase is cancer, and the cancer is a hematologic cancer or a solid tumor.
